# EUROPEAN PATENT APPLICATION

(11) **EP 2 133 353 A1**
(43) Date of publication of application: **16.12.2009**
(21) Application number: 08738671.0
(22) Date of filing: 19.03.2008
(51) Int. Cl.: C07D 473/00, A61K 31/522, A61K 31/5377, A61K 31/551, A61P 1/04, A61P 3/10, A61P 9/00, A61P 9/12, A61P 11/00, A61P 11/02, A61P 11/06, A61P 11/14, A61P 13/02, A61P 13/08, A61P 13/12, A61P 15/00, A61P 15/10, A61P 17/00, A61P 17/06, A61P 17/14

(54) **NOVEL ADENINE COMPOUND**

(30) Priority: 20.03.2007 JP 2007071713
(71) Applicant: Dainippon Sumitomo Pharma Co., Ltd., Osaka 541-8524 (JP); AstraZeneca AB, 151 85 Södertälje (SE)
(72) Inventor: ISOBE, Yoshiaki, Osaka-shi Osaka 554-0022 (JP); NAKAMURA, Tomoaki, Osaka-shi Osaka 554-0022 (JP)
(74) Representative: Vossius & Partner
(86) International application number: PCT/JP2008/055078
(87) International publication number: WO 2008/114817

(57) **Abstract**

A novel adenine compound represented by the formula (1): 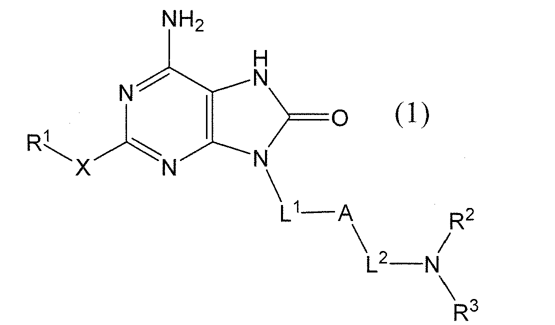 wherein A represents an (un)substituted aromatic carbocycle or (un)substituted aromatic heterocycle; L¹ and L² each independently represents straighted or branched alkylene, etc.; R¹ represents halogen, (un)substituted alkyl, (un)substituted alkenyl, (un)substituted alkynyl, (un)substituted cycloalkyl, (un)substituted aryl, or (un)substituted heteroaryl; R² and R³ each independently represents hydrogen, or (un)substituted alkyl, (un)substituted alkenyl, (un)substituted alkynyl, (un)substituted cycloalkyl, (un)substituted saturated heterocycle, (un)substituted aryl, or (un)substituted heteroaryl, or R² combines together with L² or R³ to form (un)substituted 4- to 8-membered nitrogen-containing saturated heterocycle; X is oxygen, sulfur, SO, SO₂, NR⁷, NR⁷CO wherein R⁷ is hydrogen or alkyl, or a single bond; provided that X is a single bond when R¹ is halogen, or a pharmaceutically acceptable salt thereof. The compound and salt are useful as a medicine.

## Description

### TECHNICAL FIELD

The present invention relates to a novel adenine compound having an aromatic ring, useful as a therapeutic and/or preventive agent for allergic disease, viral disease or cancer, etc.

### BACKGROUND ART

In case that foreign substances including bacteria, virus or parasite invade living organisms, immune systems exist in order to exclude said substances. In acquired immune systems, antigen processing by antigen presenting cells such as dendritic cells (DCs) is carried out when the foreign substances invade, and naive Th cells functionally differentiate via interactions of DCs/Th cells into Th1 or Th2 cells which play a central role of immune response in a body. It is reported that immune diseases are developed by one-way deflection of immuno-balance of Th1 or Th2 cells in this process.
Specifically, an excess amount of cytokine such as interleukin-4 (IL-4) and interleukin-5 (IL-5) secreted by Th2 cells is secreted in patients with allergic diseases, and the compound inhibiting immune response of Th2 cells may be expected to be a therapeutic agent for allergic disease. Also, the compound enhancing immune response of Th1 cells may be expected to be a therapeutic or preventive agent for viral disease or cancer.

In the meantime, it was believed until recently that natural immune system was caused by nonspecific phagocytosis, but it was proved that Toll-like receptor (TLR) exists and principle parts of natural immunity activation are carried out via TLR. Moreover, a ligand of TLR may be expected to have a function as a Th1/Th2 differentiation controlling agent and to be useful for treatment or prevention of immune diseases in that TLR recognizes a ligand to induce inflammatory cytokine such as IL-12, TNF, and IL-12 differentiates and induces naive T cell to Th1 cell. Actually, it is known that Th2 cell predominates in patients with asthma, atopic dermatitis, etc., and asthma-targeted clinical trials are carried out for DNA (CpGDNA) derived from microorganism, TLR9 agonist. Additionally, it is known that TLR7/8 agonist, imidazoquinoline derivative (see Patent Document 1) also shows an inhibitory activity toward the production of Th2 cytokine interleukin-4 (IL-4) and interleukin-5 (IL-5), and is actually useful for allergic diseases in experimental animal models.
Meanwhile, compounds described in, for example, Patent Documents 2 to 4 are known as compounds with adenine structures which are effective for immune diseases such as viral diseases and allergic diseases.
- Patent Document 1:: US Patent No. 4689338
- Patent Document 2:: WO98/01448
- Patent Document 3:: WO99/28321
- Patent Document 4:: WO04/029054

### DISCLOSURE OF INVENTION

### Problems to be Resolved by the Invention

Problems to be resolved by the invention are directed to provide a TLR activator, more particularly a novel adenine compound which activates as a TLR7 activator, and an immune-response modifier comprising the same as an active ingredient, for example, a therapeutic or preventive agent for allergic disease such as asthma, COPD, allergic rhinitis, allergic conjunctivitis or atopic dermatitis, viral disease such as hepatitis B, hepatitis C, HIV or HPV, bacterial infectious disease, cancer or dermatitis, etc.

### Means of Solving the Problems

The present inventors found the novel adenine compounds of the present invention according to their intensive study in order to obtain a therapeutic or preventive agent for immune diseases such as allergic disease, viral disease or cancer with excellent TLR activating action. In other words, the compounds of the present invention are effective as a therapeutic or preventive agent for allergic disease, viral disease, or cancer, etc.
The present invention has been achieved on the basis of the above knowledge. Specifically, the present invention relates to the following inventions.
[1] An adenine compound of the formula (1): 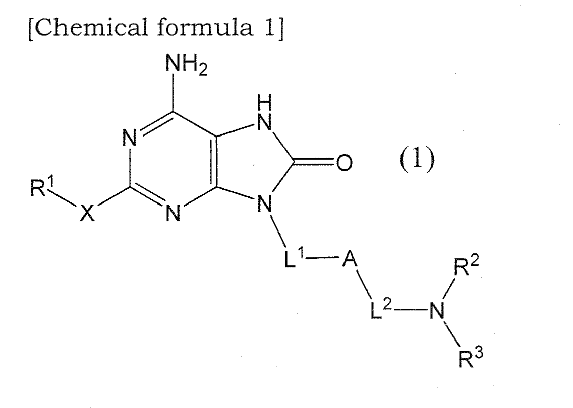 wherein
   A is substituted or unsubstituted aromatic carbocycle, or substituted or unsubsituted aromatic heterocycle;
   L¹ is a single bond, or straight chain or branched chain alkylene;
   L² is a single bond, or straight chain or branched chain alkylene optionally substituted with hydroxy, amino, alkylamino or dialkylamino;
   in the case that L² is a single bond, -NR²R³ is not unsubstituted amino, unsubstituted alkylamino, unsubstituted dialkylamino, unsubstituted pyrrolidinyl, unsubstituted piperidinno or unsubstituted morpholino;
   any one to three of methylene group(s) in the alkylene in L² may be replaced by oxygen, sulfur, SO, SO₂ carbonyl, NR⁴CO, CONR⁴, NR⁴SO₂, SO₂NR⁴, NR⁴CO₂, OCONR⁴, NR⁵CONR⁴, NR⁶C(=NR⁴)NR⁵, C(=NR⁴)NR⁵ wherein R⁴, R⁵ and R⁶ are independently hydrogen or alkyl;
   and one to three of methylene group(s) in the alkylene in L¹ may be replaced by oxygen;
   R¹ is halogen, substituted or unsubstituted alkyl, substituted or unsubstituted alkenyl, substituted or unsubstituted alkynyl, substituted or unsubstituted cycloalkyl, substituted or unsubstituted aryl, or substituted or unsubstituted heteroaryl;
   R² and R³ are independently hydrogen, substituted or unsubstituted alkyl, substituted or unsubstituted alkenyl, substituted or unsubstituted alkynyl, substituted or unsubstituted cycloalkyl, substituted or unsubstituted saturated heterocycle, substituted or unsubstituted aryl, or substituted or unsubstituted heteroaryl, or R² may be combined together with L² or R³ to form a substituted or unsubstituted 4- to 8-membered nitrogen-containing saturated heterocycle; and
   X is oxygen, sulfur, SO, SO₂ NR⁷, NR⁷CO wherein R⁷ is hydrogen or alkyl, or a single bond; provided that when R¹ is halogen, then X is a single bond,
   or a pharmaceutically acceptable salt thereof.
[2] The adenine compound according to [1] or a pharmaceutically acceptable salt thereof, wherein L² in formula (1) is a single bond, or straight chain or branched chain alkylene, in which any one to three of methylene group(s) in said alkylene may be replaced by oxygen, sulfur, SO, SO₂ carbonyl, NR⁴CO, CONR⁴, NR⁴SO₂, SO₂NR⁴, NR⁴CO₂, OCONR⁴, NR⁵CONR⁴, NR⁶C(=NR⁴)NR⁵, C(=NR⁴)NR⁵ wherein R⁴, R⁵ and R⁶ are independently hydrogen or alkyl.
[3] The adenine compound according to [1] or [2] wherein A in the formula (1) is substituted or unsubstituted benzene ring, or substituted or unsubstituted 5- to 6-membered monocyclic aromatic heterocycle;
   in case that A is substituted, it is substituted with one or more group(s) independently selected from the group consisting of: halogen, hydroxy, nitro, alkyl with 1 to 6 carbon atom(s), haloalkyl with 1 to 6 carbon atom(s), alkoxy with 1 to 6 carbon atom(s), alkylthio with 1 to 6 carbon atom(s), haloalkoxy with 1 to 6 carbon atom(s), alkylcarbonyl with 2 to 6 arbon atom(s), alkylsulfonyl with 1 to 6 carbon atom(s), alkylsulfinyl with 1 to 6 carbon atom(s), and amino optionally substituted with the same or different one or two alkyl(s) with 1 to 6 carbon atom(s),
   or a pharmaceutically acceptable salt thereof.
[4] The adenine compound according to [3], wherein the 5- to 6-membered monocyclic aromatic heterocycle is pyridine, furan or thiophene, or a pharmaceutically acceptable salt thereof.
[5] The adenine compound according to any one of [1] to [4] or a pharmaceutically acceptable salt thereof, wherein in case that alkyl, alkenyl or alkynyl in R¹ is substituted, each group may be substituted with one or more substituent(s) selected from the following (a) to (c):
   (a) halogen, hydroxy, carboxy, mercapto, haloalkyl with 1 to 6 carbon atom(s) and haloalkoxy with 1 to 6 carbon atom(s);
   (b) alkoxy with 1 to 6 carbon atom(s), alkylcarbonyl with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), alkylcarbonyloxy with 2 to 6 carbon atoms, and alkylthio with 1 to 6 carbon atom(s), wherein each group may further be substituted with one or more group(s) selected independently from halogen, hydroxy, carboxy, alkoxy with 1 to 6 carbon atom(s), alkoxycarbonyl with 2 to 6 carbon atoms, amino optionally substituted with the same or different one or two alkyl(s) with 1 to 6 carbon atom(s), carbamoyl optionally substituted with the same or different one or two alkyl(s) with 1 to 6 carbon atom(s), sulfamoyl optionally substituted with the same or different one or two alkyl(s) with 1 to 6 carbon atom(s), or alkylsulfonyl with 1 to 6 carbon atom(s);
   (c) substituted or unsubstituted 3- to 8-membered cycloalkyl and substituted or unsubstituted 4- to 8-membered saturated heterocycle, wherein each group may further be substituted with one or more group(s) selected from the following (d), (e) and (f); substituted or unsubstituted 6- to 10-membered aryl, substituted or unsubstituted 5-to 10-membered heteroaryl, substituted or unsubstituted 6- to 10-membered aryloxy and substituted or unsubstituted 5- to 10-membered heteroaryloxy, wherein each group may further be substituted with one or more group(s) selected from the following (g), (h), (i) and (j); and substituted or unsubstituted amino, substituted or unsubstituted carbamoyl and substituted or unsubstituted sulfamoyl, wherein each group may further be substituted with one or two group(s) selected from the following (k), (1) and (m);
      in case that cycloalkyl in R¹ is substituted, each group may be substituted with one or more group(s) selected from the following (d), (e) and (f):
   (d) halogen, hydroxy, carboxy, mercapto, oxo, cyano, nitro, haloalkyl with 1 to 6 carbon atom(s), and haloalkoxy with 1 to 6 carbon atom(s);
   (e) alkyl with 1 to 6 carbon atom(s), alkoxy with 1 to 6 carbon atom(s), alkenyl with 2 to 6 carbon atoms, alkynyl with 2 to 6 carbon atoms, alkoxycarbonyl with 2 to 6 carbon atoms, and alkylthio with 1 to 6 carbon atom(s), wherein each group may further be substituted with one or more group(s) selected independently from halogen, hydroxy, carboxy, alkoxy with 1 to 6 carbon atom(s), alkoxycarbonyl with 2 to 6 carbon atoms, amino optionally substituted with the same or different one or two alkyl(s) with 1 to 6 carbon atom(s), carbamoyl optionally substituted with the same or different one or two alkyl(s) with 1 to 6 carbon atom(s), sulfamoyl optionally substituted with the same or different one or two alkyl(s) with 1 to 6 carbon atom(s), and alkylsulfonyl with 1 to 6 carbon atom(s);
   (f) substituted or unsubstituted 6- to 10-membered aryl and substituted or unsubstituted 5- to 10-membered heteroaryl, wherein each group may further be substituted with one or more group(s) selected from the following (g), (h), (i) and (j); substituted or unsubstituted amino, substituted or unsubstituted carbamoyl and substituted or unsubstituted sulfamoyl, wherein each group may further be substituted with one or two group(s) selected from the following (k), (1) and (m);
      in case that aryl and heteroaryl in R¹ is substituted, each group may be substituted with one or more group(s) selected from the following (g), (h), (i) and (j):
   (g) halogen, hydroxy, mercapto, cyano, nitro, haloalkyl with 1 to 6 carbon atom(s), and haloalkoxy with 1 to 6 carbon atom(s);
   (h) alkyl with 1 to 6 carbon atom(s), alkoxy with 1 to 6 carbon atom(s), alkenyl with 2 to 6 carbon atoms, alkynyl with 2 to 6 carbon atoms and alkylthio with 1 to 6 carbon atom(s), wherein each group may further be substituted with one or more group(s) selected independently from halogen, hydroxy, carboxy, alkoxy with 1 to 6 carbon atom(s), alkoxycarbonyl with 2 to 6 carbon atoms, amino optionally substituted with the same or different one or two alkyl(s) with 1 to 6 carbon atom(s), carbamoyl optionally substituted with the same or different one or two alkyl(s) with 1 to 6 carbon atom(s), sulfamoyl optionally substituted with the same or different one or two alkyl(s) with 1 to 6 carbon atom(s), and alkylsulfonyl with 1 to 6 carbon atom(s);
   (i) 3- to 8-membered cycloalkyl and 4- to 8-membered saturated heterocycle, wherein each group may further be substituted with one or more group(s) selected independently from halogen, hydroxy, carboxy, alkyl with 1 to 6 carbon atom(s) and alkoxy with 1 to 6 carbon atom(s);
   (j) substituted or unsubstituted amino, substituted or unsubstituted carbamoyl and substituted or unsubstituted sulfamoyl, wherein each group may further be substituted with one or two group(s) selected from the following (k), (1) and (m);
      in case that amino, carbamoyl and sulfamoyl in (c), (f) and (j) is substituted, each group may be substituted with one or two group(s) selected independently from the following (k), (1) and (m):
   (k) alkyl with 1 to 6 carbon atom(s), alkenyl with 2 to 6 carbon atoms, alkynyl with 2 to 6 carbon atoms, alkylcarbonyl with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), 3- to 8-membered cycloalkyl, 3- to 8-membered cycloalkylcarbonyl, and 3- to 8-membered cycloalkoxycarbonyl, 3- to 8-membered cycloalkylsulfonyl, and 3- to 8-membered cycloalkylsulfinyl, wherein each group may further be substituted with one or more group(s) selected independently from halogen, hydroxy, carboxy, alkyl with 1 to 6 carbon atom(s), alkoxy with 1 to 6 carbon atom(s) and alkoxycarbonyl with 2 to 6 carbon atoms;
   (l) 6- to 10-membered aryl, 6- to 10-membered arylcarbonyl, 6- to 10-membered aryloxycarbonyl, 6- to 10-membered arylsulfonyl, 6- to 10-membered arylsulfinyl, 5- to 10-membered heteroaryl, 5- to 10-membered heteroarylcarbonyl, 5- to 10-membered heteroaryloxycarbonyl, 5- to 10-membered heteroarylsulfonyl, and 5- to 10-membered heteroarylsulfinyl, wherein each group may further be substituted with halogen, hydroxy, mercapto, carboxy, cyano, nitro, alkyl with 1 to 6 carbon atom(s), alkoxy with 1 to 6 carbon atom(s), alkoxycarbonyl with 2 to 6 carbon atoms or alkylthio with 1 to 6 carbon atom(s);
   (m) two substituents are combined together with nitrogen atom to form 4- to 8-membered nitrogen-containing saturated heterocycle with 1 to 4 heteroatom(s) selected from 1 to 3 nitrogen(s), 0 to 1 oxygen and 0 to 1 sulfur, wherein the nitrogen-containing saturated heterocycle may be substituted on any carbon or nitrogen atom by halogen, hydroxy, carboxy, alkyl with 1 to 6 carbon atom(s), alkoxy with 1 to 6 carbon atom(s), alkoxycarbonyl with 2 to 6 carbon atoms or alkylcarbonyl with 2 to 6 carbon atoms, where the substituent may be kept in chemically stable state,
      in case that alkyl, alkenyl and alkynyl in R² and R³ is substituted, each group may be substituted with one or more group(s) selected independently from the following (a') to (c'):
      (a') halogen, hydroxy, mercapto, haloalkyl with 1 to 4 carbon atom(s) and haloalkoxy with 1 to 6 carbon atom(s), cyano;
      (b') alkoxy with 1 to 6 carbon atom(s), alkylsulfonyl with 1 to 6 carbon atom(s), alkylsulfinyl with 1 to 6 carbon atom(s), alkylcarbonyloxy with 2 to 6 carbon atoms, alkylthio with 1 to 6 carbon atom(s), substituted or unsubstituted 3- to 8-membered cycloalkyl, and substituted or unsubstituted 3- to 8-membered cycloalkyloxy, wherein each group may further be substituted with the same or different one or more group(s) slected from halogen, hydroxy, alkyl with 1 to 6 carbon atom(s) and alkoxy with 1 to 6 carbon atom(s);
      (c') substituted or unsubstituted 6- to 10-membered aryl, substituted or unsubstituted 6- to 10-membered aryloxy, substituted or unsubstituted 5- to 10-membered heteroaryl and substituted or unsubstituted 5- to 10-membered heteroaryloxy, wherein each group may further be substituted with the same or different one or more group(s) selected from the following (g') to (j'); substituted or unsubstituted amino, substituted or unsubstituted carbamoyl and substituted or unsubstituted sulfamoyl, wherein each group may further be substituted with the same or different one or more group(s) selected from the following (k') to (m');
         in case that aryl, aryloxy, heteroaryl and heteroaryloxy in the above (c') is substituted, each group may be substituted with one or more group(s) selected from the following (g') to (j'):
      (g') halogen, hydroxy, mercapto, cyano, nitro, haloalkyl with 1 to 6 carbon atom(s), and haloalkoxy with 1 to 6 carbon atom(s);
      (h') alkyl with 1 to 6 carbon atom(s), alkoxy with 1 to 6 carbon atom(s), alkenyl with 2 to 6 carbon atoms, alkynyl with 2 to 6 carbon atoms and alkylthio with 1 to 6 carbon atom(s), wherein each group may further be substituted with one or more group(s) independently selected from halogen, hydroxy, alkoxy with 1 to 6 carbon atom(s), amino optionally substituted with the same or different one or two alkyl(s) with 1 to 6 carbon atom(s), carbamoyl optionally substituted with the same or different one or two alkyl(s) with 1 to 6 carbon atom(s), sulfamoyl optionally substituted with the same or different one or two alkyl(s) with 1 to 6 carbon atom(s), and alkylsulfonyl with 1 to 6 carbon atom(s);
      (i') 3- to 8-membered cycloalkyl and 4- to 8-membered saturated heterocycle, wherein each group may further be substituted with one or more group(s) independently selected from halogen, hydroxy, oxo, alkyl with 1 to 6 carbon atom(s), alkoxy with 1 to 6 carbon atom(s), and alkylcarbonyl with 2 to 6 carbon atoms;
      (j') amino, carbamoyl, sulfamoyl, wherein each group may further be substituted with one or two group(s) selected from the following (k') to (m');
         in case that amino, carbamoyl and sulfamoyl in the above (c') and
      (j') is substituted, each group may be substituted with one or two group(s) selected from the following (k'), (1') and (m'):
      (k') alkyl with 1 to 6 carbon atom(s), alkenyl with 2 to 6 carbon atoms, alkynyl with 2 to 6 carbon atoms, alkylcarbonyl with 2 to 6 carbon atoms, alkylsulfonyl with 1 to 6 carbon atom(s), alkylsulfinyl with 1 to 6 carbon atom(s), 3- to 8-membered cycloalkyl, 3- to 8-membered cycloalkylcarbonyl, 3- to 8-membered cycloalkoxycarbonyl, 3- to 8-membered cycloalkylsulfonyl, 3- to 8-membered cycloalkylsulfinyl, wherein each group may further be substituted with one or more group(s) selected independently from halogen, hydroxy, alkyl with 1 to 6 carbon atom(s), and alkoxy with 1 to 6 carbon atom(s);
      (1') 6- to 10-membered aryl, 6- to 10-membered arylalkyl, 6- to 10-membered aryloxyalkyl, 6- to 10-membered arylcarbonyl, 6- to 10-membered arylsulfonyl, 6- to 10-membered arylsulfinyl, 5- to 10-membered heteroaryl, 5- to 10-membered heteroarylalkyl, 5- to 10-membered heteroaryloxyalkyl, 5- to 10-membered heteroarylcarbonyl, 5- to 10-membered heteroarylsulfonyl, and 5- to 10-membered heteroarylsulfinyl, wherein each group may further be substituted with one or more group(s) selected from halogen, hydroxy, mercapto, cyano, nitro, alkyl with 1 to 6 carbon atom(s), alkoxy with 1 to 6 carbon atom(s) and alkylthio with 1 to 6 carbon atom(s);
      (m') two substituents are combined together with nitrogen atom to form 4- to 8-membered nitrogen-containing saturated heterocycle with 1 to 4 heteroatom(s) selected from 1 to 3 nitrogen(s), 0 to 1 oxygen and 0 to 1 sulfur, wherein the nitrogen-containing saturated heterocycle may be substituted on any carbon or nitrogen atom by halogen, hydroxy, alkyl with 1 to 6 carbon atom(s), alkoxy with 1 to 6 carbon atom(s) and alkylcarbonyl with 2 to 6 carbon atoms, where the substituent may be kept in chemically stable state,
         in case that cycloalkyl, saturated heterocycle in R², the nitrogen-containing saturated heterocycle formed by combining R² with R³, and the nitrogen-containing saturated heterocycle formed by combining R² with L² is substituted, each group may be substituted with one or more group(s) selected independently from the group consisting of: halogen; hydroxy; oxo; substituted or unsubstituted alkyl with 1 to 6 carbon atom(s), substituted or unsubstituted alkoxy with 1 to 6 carbon atom(s) and substituted or unsubstituted alkylcarbonyl with 2 to 6 carbon atoms, wherein the alkyl, alkoxy or alkylcarbonyl may be substituted with one or more group(s) selected independently from the above (a') to (c'); substituted or unsubstituted aryl, substituted or unsubstituted aryloxy, substituted or unsubstituted a heteroaryl and substituted or unsubstituted heteroaryloxy, wherein the aryl, aryloxy, heteroaryl or heteroaryloxy may be substituted with one or more group(s) selected independently from the above (g') to (j'); substituted or unsubstituted amino, substituted or unsubstituted carbamoyl and substituted or unsubstituted sulfamoyl, wherein the amino, carbamoyl or sulfamoyl may be substituted with one or two group(s) selected independently from the above (k') to (m'); and
         in case that aryl and heteroaryl in R² is substituted, each group may be substituted with one or more group(s) selected independently from the above (g') to (j').
[6] The adenine compound according to [5] or a pharmaceutically acceptable salt thereof,
   wherein R² and R³ are independently hydrogen, substituted or unsubstituted alkyl with 1 to 6 carbon atom(s), substituted or unsubstituted 4- to 8-membered saturated heterocycle with 1 to 4 heteroatom(s) selected from 0 to 3 nitrogen(s), 0 to 1 oxygen and 0 to 1 sulfur, substituted or unsubstituted 3- to 8-membered cycloalkyl, substituted or unsubstituted 6- to 10-membered aryl, or substituted or unsubstituted 5- to 10-membered heteroaryl; or R² and R³ are combined together to form 4- to 8-membered nitrogen-containing saturated heterocycle with 1 to 4 heteroatom(s) selected from 1 to 3 nitrogen(s), 0 to 1 oxygen and 0 to 1 sulfur;
   said alkyl is optionally substituted with one or more group(s) selected from halogen, hydroxy, alkoxy with 1 to 6 carbon atom(s), substituted or unsubstituted 6- to 10-membered aryl, substituted or unsubstituted 6- to 10-membered aryloxy, substituted or unsubstituted amino, and carbamoyl optionally substituted with the same or different one or two alkyl(s) with 1 to 6 carbon atom(s);
   said saturated heterocycle, cycloalkyl and nitrogen-containing saturated heterocycle formed by combining R² with R³ are optionally substituted with one or more group(s) selected from halogen, hydroxy, oxo, alkyl with 1 to 6 carbon atom(s), alkoxy with 1 to 6 carbon atom(s), substituted or unsubstituted 6- to 10-membered aryl, substituted or unsubstituted 6- to 10-membered aryloxy, substituted or unsubstituted 6- to 10-membered arylalkyl, substituted or unsubstituted 6- to 10-membered aryloxyalkyl, substituted or unsubstituted 5- to 10-membered heteroaryl, substituted or unsubstituted 5- to 10-membered heteroaryloxy, substituted or unsubstituted 5- to 10-membered heteroarylalkyl, substituted or unsubstituted 5- to 10-membered heteroaryloxyalkyl, substituted or unsubstituted amino, and carbamoyl optionally substituted with the same or different one or two alkyl(s) with 1 to 6 carbon atom(s);
   in case that said aryl, aryloxy, arylalkyl, aryloxyalkyl, heteroaryl, heteroaryloxy, heteroarylalkyl and heteroaryloxyalkyl are substituted, each group may be substituted with one or more group(s) selected from halogen, hydroxy, alkyl with 1 to 6 carbon atom(s), alkoxy with 1 to 6 carbon atom(s), substituted or unsubstituted amino; and in case that said amino is substituted, it may be substituted with the same or different one or two group(s) selected from alkyl with 1 to 6 carbon atom(s), alkylcarbonyl with 2 to 6 carbon atoms and alkylsulfonyl with 1 to 6 carbon atom(s), or two substituents on said substituted amino are combined together to form 4- to 8-membered saturated heterocycle with 1 to 4 heteroatom(s) selected from 1 to 3 nitrogen(s), 0 to 1 oxygen and 0 to 1 sulfur wherein said saturated heterocycle is optionally substituted with one or more group(s) selected from halogen, hydroxy, alkyl with 1 to 6 carbon atom(s), alkoxy with 1 to 6 carbon atom(s), alkylcarbonyl with 2 to 6 carbon atoms, and amino optionally substituted with the same or different one or two alkyl(s) with 1 to 6 carbon atom(s).
[7] The adenine compound according to any one of [1] to [5] or a pharmaceutically acceptable salt thereof,
   wherein R² and R³ are independently hydrogen; alkyl with 1 to 6 carbon atom(s); or alkyl with 1 to 6 carbon atom(s) substituted with 1 to 3 substituent(s) selected from halogen, cyano, hydroxy, alkoxy with 1 to 6 carbon atom(s), substituted or unsubstituted aryl, substituted or unsubstituted aryloxy and substituted or unsubstituted amino;
   in case that aryl and aryloxy are substituted, each group may be substituted with one or more group(s) selected from halogen, hydroxy, alkyl with 1 to 6 carbon atom(s), alkoxy with 1 to 6 carbon atom(s) and substituted or unsubstituted amino;
   in case that amino is substituted, it may be substituted with the same or different one or two group(s) selected from alkyl with 1 to 6 carbon atom(s), alkylcarbonyl with 2 to 6 carbon atoms and alkylsulfonyl with 1 to 6 carbon atom(s), or two substituents on said substituted amino are combined together to form 4- to 8-membered saturated heterocycle with 1 to 4 heteroatom(s) selected from 1 to 3 nitrogen(s), 0 to 1 oxygen and 0 to 1 sulfur wherein said saturated heterocycle is optionally substituted with one or more group(s) selected from halogen, hydroxy, alkyl with 1 to 6 carbon atom(s), alkoxy with 1 to 6 carbon atom(s), alkylcarbonyl with 2 to 6 carbon atoms, and amino optionally substituted with the same or different one or two alkyl(s) with 1 to 6 carbon atom(s).
[8] The adenine compound according to [7] or a pharmaceutically acceptable salt thereof wherein R² and R³ are independently hydrogen or alkyl with 1 to 6 carbon atom(s) optionally substituted with hydroxy, C₁₋₆ alkoxy, or an amino group optionally substituted with the same or different one or two C₁₋₆ alkyl(s).
[9] The adenine compound according to any one of [1] to [5] or a pharmaceutically acceptable salt thereof,
   wherein R² is substituted or unsubstituted 4- to 8-membered saturated heterocycle with 1 to 4 heteroatom(s) selected from 0 to 3 nitrogen(s), 0 to 1 oxygen and 0 to 1 sulfur, substituted or unsubstituted 3- to 8-membered cycloalkyl, substituted or unsubstituted 6- to 10-membered aryl, or substituted or unsubstituted 5- to 10-membered heteroaryl;
   R³ is hydrogen or alkyl with 1 to 6 carbon atom(s);
   in case that saturated heterocycle, cycloalkyl, aryl and heteroaryl are substituted, each group may be substituted with one or more group(s) selected from halogen, hydroxy, alkyl with 1 to 6 carbon atom(s), alkoxy with 1 to 6 carbon atom(s) and substituted or unsubstituted amino
   in case that amino is substituted, it may be substituted with the same or different one or two group(s) selected from alkyl with 1 to 6 carbon atom(s), alkylcarbonyl with 2 to 6 carbon atoms and alkylsulfonyl with 1 to 6 carbon atom(s), or two substituents on said amino are combined together to form 4- to 8-membered saturated heterocycle with 1 to 4 heteroatom(s) selected from 1 to 3 nitrogen(s), 0 to 1 oxygen and 0 to 1 sulfur wherein said saturated heterocycle is optionally substituted with one or more group(s) selected from halogen, hydroxy, alkyl with 1 to 6 carbon atom(s), alkoxy with 1 to 6 carbon atom(s), alkylcarbonyl with 2 to 6 carbon atoms, and amino optionally substituted with the same or different one or two alkyl(s) with 1 to 6 carbon atom(s).
[10] The adenine compound according to any one of [1] to [5] or a pharmaceutically acceptable salt thereof, wherein R² and R³ are combined together to form 4- to 8-membered nitrogen-containing saturated heterocycle with 1 to 4 heteroatom(s) selected from 1 to 3 nitrogen(s), 0 to 1 oxygen and 0 to 1 sulfur;
   wherein said nitrogen-containing saturated heterocycle is optionally substituted with one or more group(s) selected from halogen; hydroxy; oxo; alkyl with 1 to 6 carbon atom(s), alkoxy with 1 to 6 carbon atom(s) and alkylcarbonyl with 2 to 6 carbon atoms wherein said alkyl, alkoxy and alkylcarbonyl is optionally substituted with 1 to 3 substituent(s) selected from halogen, cyano, hydroxy, alkoxy with 1 to 6 carbon atom(s), substituted or unsubstituted 6- to 10-membered aryl, substituted or unsubstituted 6- to 10-membered aryloxy, substituted or unsubstituted amino, and carbamoyl optionally substituted with the same or different one or two alkyl(s) with 1 to 6 carbon atom(s); substituted or unsubstituted 3- to 8-membered cycloalkyl; substituted or unsubstituted 6- to 10-membered aryl; substituted or unsubstituted 6- to 10-membered aryloxy; substituted or unsubstituted 5- to 10-membered heteroaryl; substituted or unsubstituted 5- to 10-membered heteroaryloxy; substituted or unsubstituted amino, and carbamoyl optionally substituted with the same or different one or two alkyl(s) with 1 to 6 carbon atom(s);
   in case that aryl, aryloxy, heteroaryl and heteroaryloxy are substituted, each group may be substituted with one or more group(s) selected from halogen, hydroxy, alkyl with 1 to 6 carbon atom(s), alkoxy with 1 to 6 carbon atom(s) and substituted or unsubstituted amino;
   in case that amino is substituted, it may be substituted with one or more group(s) selected from the same or different one or two group(s) selected from alkyl with 1 to 6 carbon atom(s), alkylcarbonyl with 2 to 6 carbon atoms and alkylsulfonyl with 1 to 6 carbon atom(s), or two substituents on said substituted amino are combined together to form 4- to 8-membered saturated heterocycle with 1 to 4 heteroatom(s) selected from 1 to 3 nitrogen(s), 0 to 1 oxygen and 0 to 1 sulfur wherein said saturated heterocycle is optionally substituted with one or more group(s) selected from halogen, hydroxy, alkyl with 1 to 6 carbon atom(s), alkoxy with 1 to 6 carbon atom(s), alkylcarbonyl with 2 to 6 carbon atoms, and amino optionally substituted with the same or different one or two alkyl(s) with 1 to 6 carbon atom(s).
[11] The adenine compound according to [5] or [10], or a pharmaceutically acceptable salt thereof, wherein the nitrogen-containing saturated heterocycle formed by combining R² with R³ is substituted or unsubstituted azetidine, substituted or unsubstituted morpholine, substituted or unsubstituted piperidine, substituted or unsubstituted piperazine, substituted or unsubstituted pyrrolidine or substituted or unsubstituted 1,4-perhydrodiazepine.
[12] The adenine compound according to any one of [1] to [5], or a pharmaceutically acceptable salt thereof, wherein R³ is hydrogen or alkyl with 1 to 6 carbon atom(s); any carbon atom on R² and L² are combined together to form optionally substituted 4- to 8-membered nitrogen-containing saturated heterocycle containing 1 to 4 heteroatom(s) selected from 1 to 3 nitrogen(s), 0 to 1 oxygen and 0 to 1 sulfur.
[13] The adenine compound according to [12], or a pharmaceutically acceptable salt thereof, wherein -L²-NR²R³ in the formula (1) is represented by the formula: 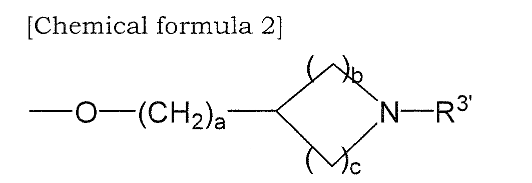 in which a is an integer of 0 to 2, b is an integer of 0 to 2, c is an integer of 1 to 4, with the proviso that the sum of b and c is 2 to 4, and R^{3'} is hydrogen or alkyl with 1 to 6 carbon atom(s).
[14] The adenine compound according to any one of [1] to [11], or a pharmaceutically acceptable salt thereof, wherein -L²- in the formula (1) is a single bond or divalent group of the formula: -(O)ₚ-(CH₂)ₙ- wherein p is 0 or 1, n is an integer of 0 to 6 when p is 0 or an integer of 2 to 6 when p is 1.
[15] The adenine compound according to any one of [1] to [14], or a pharmaceutically acceptable salt thereof, wherein L¹ in the formula (1) is alkylene with 1 to 6 carbon atom(s) or divalent group of the formula: - (CH₂)_{n'}-(O)_{p'}- wherein p' is 0 or 1; n' is an integer of 1 to 6 when p' is 0 or an integer of 2 to 6 when p' is 1.
[16] The adenine compound according to [15], or a pharmaceutically acceptable salt thereof, wherein L¹ is alkylene with 1 to 3 carbons; L² is methylene or divalent group of the formula: -O-(CH₂)ₙ- wherein n is an integer of 2 to 4.
[17] The adenine compound according to any one of [1] to [16], or a pharmaceutically acceptable salt thereof, wherein X in the formula (1) is a single bond, NH, oxygen or sulphur; R¹ is alkyl with 1 to 6 carbon atom(s), or alkyl with 1 to 6 carbon atom(s) substituted with a substituent selected from haloalkyl with 1 to 4 carbon atom(s), alkoxy with 1 to 4 carbons, 3- to 6-membered cycloalkyl, 6- to 10-membered aryl and 5- to 10-membered heteroaryl wherein said cycloalkyl, aryl and heteroaryl is optionally substitued with one to four group(s) selected from halogen, hydroxy, alkyl with 1 to 6 carbon atom(s), and alkoxy with 1 to 6 carbon atom(s).
[18] The adenine compound according to [17] or a pharmaceutically acceptable salt thereof, wherein X in the formula (1) is NH or oxygen.
[19] The adenine compound according to any one of [1] to [5] or [17] to [18], or a pharmaceutically acceptable salt thereof, wherein A is pyridine ring; L¹ is alkylene with 1 to 3 carbons; L² is single bond; R² is hydrogen, alkyl with 1 to 6 carbon atom(s), or alkyl with 1 to 6 carbon atom(s) substituted with amino, alkylamino or dialkylamino; R³ is alkyl with 1 to 6 carbon atom(s) substituted with amino, alkylamino or dialkylamino; or R² and R³ are combined together to form piperazine ring optionally substituted with alkyl with 1 to 6 carbon atom(s), 1,4-perhydrodiazepine ring optionally substituted with alkyl with 1 to 6 carbon atom(s), or saturated nitrogen-containing heterocycle selected from pyrrolidine ring, piperidine ring, morpholine ring, thiomorpholine ring and azetidine ring, wherein said saturated nitrogen-containing heterocycle is substituted with amino, alkylamino, dialkylamino, or alkyl with 1 to 6 carbon atom(s) substituted with amino, alkylamino or dialkylamino.
[20] The adenine compound according to [1], which is selected from the following compounds:
   6-amino-2-butoxy-9-(4-morpholin-4-ylmethylbenzyl)-7,9-dihydropurin-8-one;
   6-amino-2-butoxy-9-(4-piperidin-1-ylmethylbenzyl)-7,9-dihydropurin-8-one;
   6-amino-2-butoxy-9-[4-(4-methylpiperazin-1-ylmethyl)benzyl]-7,9-dihydropurin-8-one;
   6-amino-2-butoxy-9-[4-(4-dimethylamminiopiperidin-1-ylmethyl)benzyl]-7,9-dihydropurin-8-one;
   6-amino-2-butoxy-9-[4-(3-dimethylamminiopyrrolidin-1-ylmethyl)benzyl]-7,9-dihydropurin-8-one;
   6-amino-2-butoxy-9-(4-{[methyl(1-methylpyrrolidin-3-yl)amino]methyl}benzyl)-7,9-dihydropurin-8-one;
   N-{1-[4-(6-amino-2-butoxy-8-oxo-7,8-dihydropurin-9-ylmethyl)benzyl]piperidin-4-yl}acetamide;
   1-[4-(6-amino-2-butoxy-8-oxo-7,8-dihydropurin-9-ylmethyl)benzyl]piperidin-4-carboxylic acid amide;
   6-amino-2-butoxy-9-[3-(4-methylpiperazin-1-ylmethyl)benzyl]-7,9-dihydropurin-8-one;
   6-amino-2-(2-methoxyethoxy)-9-(4-piperidin-1-ylmethylbenzyl)-7,9-dihydropurin-8-one;
   6-amino-2-(2-methoxyethoxy)-9-[4-(4-methylpiperazin-1-ylmethyl)benzyl]-7,9-dihydropurin-8-one;
   6-amino-2-(2-methoxyethoxy)-9-[4-(4-phenylpiperazin-1-ylmethyl)benzyl]-7,9-dihydropurin-8-one;
   6-amino-2-(2-methoxyethoxy)-9-[4-(4-phenoxypiperidin-1-ylmethyl)benzyl]-7,9-dihydropurin-8-one;
   6-amino-9-(4-dimethylamminiomethylbenzyl)-2-(2-methoxyethoxy)-7,9-dihydropurin-8-one;
   6-amino-9-{4-[(diisopropylamino)methyl]benzyl}-2-(2-methoxyethoxy)-7,9-dihydropurin-8-one;
   6-amino-2-(2-methoxyethoxy)-9-(4-{[(2-methoxyethyl)methylamino]methyl}benzyl)-7,9-dihydropurin-8-one;
   6-amino-9-(4-[(cyclohexylmethylamino)methyl]benzyl)-2-(2-methoxyethoxy)-7,9-dihydropurin-8-one;
   6-amino-9-(4-cyclohexylaminomethylbenzyl)-2-(2-methoxyethoxy)-7,9-dihydropurin-8-one;
   6-amino-2-(2-methoxyethoxy)-9-{4-[(methylphenylamino)methyl]benzyl}-7,9-dihydropurin-8-one;
   6-amino-9-{4-[(benzylmethylamino)methyl]benzyl}-2-(2-methoxyethoxy)-7,9-dihydropurin-8-one;
   6-amino-9-(4-morpholin-4-ylmethylbenzyl)-2-propoxy-7,9-dihydropurin-8-one;
   6-amino-2-cyclopropylmethoxy-9-(4-morpholin-4-ylmethylbenzyl)-7,9-dihydropurin-8-one;
   6-amino-9-(4-morpholin-4-ylmethylbenzyl)-2-(4,4,4-trifluorobutoxy)-7,9-dihydropurin-8-one;
   6-amino-9-[4-(4-methylpiperazin-1-ylmethyl)benzyl]-2-(4,4,4-trifluorobutoxy)-7,9-dihydropurin-8-one;
   6-amino-9-(4-{[(2-methoxyethyl)methylamino]methyl}benzyl)-2-(4,4,4-trifluorobutoxy)-7,9-dihydropurin-8-one;
   6-amino-9-[4-(4-methoxypiperidin-1-ylmethyl)benzyl]-2-(2,2,2-trifluoroethoxy)-7,9-dihydropurin-8-one;
   6-amino-9-[4-(4-oxopiperidin-1-ylmethyl)benzyl]-2-(2,2,2-trifluoroethoxy)-7,9-dihydropurin-8-one;
   6-amino-2-butylamino-9-(4-dimethylamminiomethylbenzyl)-7,9-dihydropurin-8-one;
   6-amino-2-butylamino-9-(4-piperidin-1-ylmethylbenzyl)-7,9-dihydropurin-8-one;
   6-amino-2-butylamino-9-(4-morpholin-4-ylmethylbenzyl)-7,9-dihydropurin-8-one;
   6-amino-2-butylamino-9-[4-(4-dimethylamminiopiperidin-1-ylmethyl)benzyl]-7,9-dihydropurin-8-one;
   6-amino-2-butylamino-9-[4-(4-methylpiperazin-1-ylmethyl)benzyl]-7,9-dihydropurin-8-one;
   6-amino-2-butylamino-9-(3-piperidin-1-ylmethylbenzyl)-7,9-dihydropurin-8-one;
   6-amino-2-butoxy-9-(3-morpholin-4-ylmethylbenzyl)-7,9-dihydropurin-8-one;
   6-amino-9-[4-(4-aminopiperidin-1-ylmethyl)benzyl]-2-butoxy-7,9-dihydropurin-8-one;
   6-amino-2-butoxy-9-[4-(2-dimethylaminoethoxy)benzyl]-7,9-dihydropurin-8-one;
   6-amino-2-butoxy-9-[4-(3-dimethylamminiopropoxy)benzyl]-7,9-dihydropurin-8-one;
   6-amino-2-(2-methoxyethoxy)-9-[4-(3-piperidin-1-ylpropoxy)benzyl]-7,9-dihydropurin-8-one;
   6-amino-2-butylamino-9-[4-(3-morpholin-4-ylpropoxy)benzyl]-7,9-dihydropurin-8-one;
   6-amino-2-butoxy-9-[6-(4-methylpiperazin-1-yl)pyridin-3-ylmethyl]-7,9-dihydropurin-8-one;
   6-amino-2-butoxy-9-[6-(4-methyl-[1,4]diazepan-1-yl)pyridin-3-ylmethyl]-7,9-dihydropurin-8-one;
   6-amino-2-(2-methoxyethoxy)-9-[6-(4-methylpiperazin-1-yl)pyridin-3-ylmethyl]-7,9-dihydropurin-8-one;
   6-amino-9-[6-(4-methylpiperazin-1-yl)pyridin-3-ylmethyl]-2-(4,4,4-trifluorobutoxy)-7,9-dihydropurin-8-one;
   6-amino-2-ethoxy-9-[6-(4-methylpiperazin-1-yl)pyridin-3-ylmethyl]-7,9-dihydropurin-8-one;
   6-amino-9-[6-(4-methylpiperazin-1-yl)pyridin-3-ylmethyl]-2-(2,2,2-trifluoroethoxy)-7,9-dihydropurin-8-one;
   6-amino-2-butylamino-9-[6-(4-methylpiperazin-1-yl)pyridin-3-ylmethyl]-7,9-dihydropurin-8-one;
   6-amino-2-butylamino-9-[6-(4-methyl-[1,4]diazepan-1-yl)pyridin-3-ylmethyl]-7,9-dihydropurin-8-one;
   6-amino-2-butylamino-9-(6-piperazin-1-ylpyridin-3-ylmethyl)-7;9-dihydropurin-8-one;
   6-amino-2-butylamino-9-[6-(4-dimethylamminiopiperidin-1-yl)pyridin-3-ylmethyl]-7,9-dihydropurin-8-one;
   6-amino-2-butylamino-9-{6-[(3-dimethylaminopropyl)methylamino]pyridin-3-ylmethyl}-7,9-dihydropurin-8-one;
   6-amino-2-butylamino-9-[6-(3-dimethylamminiopyrrolidin-1-yl)pyridin-3-ylmethyl}-7,9-dihydropurin-8-one;
   6-amino-2-butoxy-9-[6-(2-morpholin-4-ylethoxy)pyridin-3-ylmethyl}-7,9-dihydropurin-8-one;
   6-amino-2-butylamino-9-[6-(2-morpholin-4-ylethoxy)pyridin-3-ylmethyl}-7,9-dihydropurin-8-one;
   6-amino-2-butylamino-9-[6-(2-dimethylaminoethoxy)pyridin-3-ylmethyl}-7,9-dihydropurin-8-one;
   6-amino-2-butylamino-9-[6-(4-dimethylamminiobutoxy)pyridin-3-ylmethyl}-7,9-dihydropurin-8-one;
   6-amino-2-butylamino-9-[5-chloro-6-(4-methylpiperazin-1-yl)pyridin-3-ylmethyl]-7,9-dihydropurin-8-one;
   6-amino-9-[5-chloro-6-(4-methylpiperazin-1-yl)pyridin-3-ylmethyl]-2-ethoxy-7,9-dihydropurin-8-one;
   6-amino-2-butylamino-9-[5-chloro-6-(2-dimethylaminoethoxy)pyridin-3-ylmethyl]-7,9-dihydropurin-8-one;
   6-amino-2-butylamino-9-[5-chloro-6-(2-morpholin-4-ylethoxy)pyridin-3-ylmethyl]-7,9-dihydropurin-8-one;
   6-amino-2-butylamino-9-[4-(4-methylpiperazin-1-yl)-3-nitrobenzyl]-7,9-dihydropurin-8-one;
   6-amino-9-[3-amino-4-(4-methylpiperazin-1-yl)benzyl]-2-butylamino-7,9-dihydropurin-8-one;
   6-amino-2-ethoxy-9-(3-methoxy-4-morpholin-4-ylmethylbenzyl)-7,9-dihydropurin-8-one;
   6-amino-9-(4-dimethylamminiomethylbenzyl)-2-ethoxy-7,9-dihydropurin-8-one;
   6-amino-9-(4-diethylaminomethylbenzyl)-2-ethoxy-7,9-dihydropurin-8-one;
   6-amino-9-(4-diisopropylaminomethylbenzyl)-2-ethoxy-7,9-dihydropurin-8-one;
   6-amino-2-ethoxy-9-(4-piperidin-1-ylmethylbenzyl)-7,9-dihydropurin-8-one;
   6-amino-2-ethoxy-9-[4-(4-methoxypiperidin-1-ylmethyl)benzyl]-7,9-dihydropurin-8-one;
   6-amino-2-ethoxy-9-(4-morpholin-4-ylmethylbenzyl)-7,9-dihydropurin-8-one;
   6-amino-2-ethoxy-9-(4-thiomorpholine-4-ylmethylbenzyl)-7,9-dihydropurin-8-one;
   6-amino-2-ethoxy-9-[4-(4-methylpiperazin-1-ylmethylbenzyl)]-7,9-dihydropurin-8-one;
   6-amino-2-butyl-9-(4-dimethylamminiomethylbenzyl)-7,9-dihydropurin-8-one;
   6-amino-2-butyl-9-(4-morpholin-4-ylmethylbenzyl)-7,9-dihydropurin-8-one;
   6-amino-2-butyl-9-[4-(4-methoxypiperidin-1-ylmethyl)benzyl]-7,9-dihydropurin-8-one;
   6-amino-2-butoxy-9-[3-(4-dimethylamminiomethylphenoxy)propyl]-7,9-dihydropurin-8-one;
   6-amino-2-butoxy-9-(5-dimethylamminiomethylfuran-2-ylmethyl)-7,9-dihydropurin-8-one;
   6-amino-9-(4-dimethylamminiomethylbenzyl)-2-[(pyridin-4-ylmethyl)amino]-7,9-dihydropurin-8-one;
   6-amino-2-(2-methoxyethoxy)-9-[4-(4-pyridin-4-ylpiperazin-1-ylmethyl)benzyl]-7,9-dihydropurin-8-one;
   6-amino-9-(4-{[bis(2-methoxyethyl)amino]methyl}benzyl)-2-(2-methoxyethoxy)-7,9-dihydropurin-8-one;
   6-amino-9-(4-{[bis(2-hydroxyethyl)amino]methyl}benzyl)-2-butoxy-7,9-dihydropurin-8-one;
   6-amino-2-butoxy-9-(4-{[(2,3-dihydroxypropyl)methylamino]methyl}benzyl)-7,9-dihydropurin-8-one;
   6-amino-2-butoxy-9-(4-{[(2-dimethylamminioethyl)methylamino]methyl)benzyl)-7,9-dihydropurin-8-one;
   6-amino-9-[6-(2-dimethylaminoethoxy)pyridin-3-ylmethyl]-2-(2-methoxyethoxy)-7,9-dihydropurin-8-one;
   6-amino-2-butoxy-9-(4-dimethylamminiomethylbenzyl)-7,9-dihydropurin-8-one;
   6-amino-2-butoxy-9-[4-(3-hydroxyazetidine-1-ylmethyl)benzyl]-7,9-dihydropurin-8-one;
   6-amino-9-(4-{[bis(2-diethylamminioethyl)amino]methyl}benzyl)-2-butoxy-7,9-dihydropurin-8-one;
   6-amino-2-butoxy-9-{4-[4-(2-dimethylaminoacetyl)piperazin-1-ylmethyl]benzyl}-7,9-dihydropurin-8-one;
   2-{4-[4-(6-amino-2-butoxy-8-oxo-7,8-dihydropurin-9-ylmethyl)benzyl]piperazin-1-yl}-N,N-dimethylacetamide;
   6-amino-2-(2-methoxyethoxy)-9-[4-(4-methoxypiperidin-1-ylmethyl)benzyl]-7,9-dihydropurin-8-one;
   6-amino-9-{4-[(butylmethylamino)methyl]benzyl}-2-(2-methoxyethoxy)-7,9-dihydropurin-8-one;
   4-({4-[6-amino-2-(2-methoxyethoxy)-8-oxo-7,8-dihydropurin-9-ylmethyl]benzyl}methylamino)butyronitrile;
   N-(1-{4-[6-amino-2-(2-methoxyethoxy)-8-oxo-7,8-dihydropurin-9-ylmethyl]benzyl}pyrrolidin-3-yl)-N-methylacetamide;
   6-amino-9-(4-{[ethyl(tetrahydropyran-4-yl)amino]methyl}benzyl)-2-(2-methoxyethoxy)-7,9-dihydropurin-8-one;
   6-amino-9-[4-(4,4-difluoropiperidin-1-ylmethyl)benzyl]-2-(2-methoxyethoxy)-7,9-dihydropurin-8-one;
   6-amino-9-[4-(4-cyclopentylpiperazin-1-ylmethyl)benzyl]-2-(2-methoxyethoxy)-7,9-dihydropurin-8-one;
   6-amino-9-(4-{[isopropyl(2-methoxyethyl)amino]methyl}benzyl)-2-(2-methoxyethoxy)-7,9-dihydropurin-8-one;
   6-amino-2-butoxy-9-{6-[(2-dimethylamminioethyl)methylamino]pyridin-3-ylmethyl}-7,9-dihydropurin-8-one;
   6-amino-9-[5-chloro-6-(4-methylpiperazin-1-yl)pyridin-3-ylmethyl]-2-(2-methoxyethoxy)-7,9-dihydropurin-8-one;
   6-amino-9-[5-chloro-6-(4-methyl-[1,4]diazepan-1-yl)pyridin-3-ylmethyl]-2-(2-methoxyethoxy)-7,9-dihydropurin-8 -one;
   6-amino-2-butoxy-9-(6-{2-[(2-hydroxyethyl)methylamino]ethoxy}pyridin-3-ylmethyl)-7,9-dihydropurin-8-one;
   6-amino-2-butoxy-9-[6-(2-dimethylamminio-1-dimethylamminiomethylethoxy)pyridin-3-ylmethyl]-7,9-dihydropurin-8-one;
   6-amino-2-(2-methoxyethoxy)-9-[6-(2-piperidin-1-ylethoxy)pyridin-3-ylmethyl]-7,9-dihydropurin-8-one;
   6-amino-9-[6-(3-dimethylamminio-2,2-dimethylpropoxy)pyridin-3-ylmethyl]-2-(2-methoxyethoxy)-7,9-dihydropurin-8-one;
   6-amino-2-(2-methoxyethoxy)-9-[6-(1-methylpiperidine-3-ylmethoxy)pyridin-3-ylmethyl]-7,9-dihydropurin-8-one;
   6-amino-2-(2-methoxyethoxy)-9-[6-(1-methylpiperidine-4-yloxy)pyridin-3-ylmethyl]-7,9-dihydropurin-8-one;
   6-amino-9-[6-(2-dimethylaminoethoxy)pyridin-3-ylmethyl}-2-ethoxy-7,9-dihydropurin-8-one;
   6-amino-2-ethoxy-9-{6-[2-(4-methylpiperazin-1-yl)ethoxy]pyridin-3-ylmethyl}-7,9-dihydropurin-8-one;
   6-amino-2-ethoxy-9-{6-[3-(4-methylpiperazin-1-yl)propoxy]pyridin-3-ylmethyl}-7,9-dihydropurin-8-one;
   6-amino-2-butylamino-9-[6-(3-dimethylamminiopropoxy)pyridin-3-ylmethyl}-7,9-dihydropurin-8-one;
   6-amino-2-(2-methoxyethoxy)-9-[6-(1-methylpiperidine-4-ylmethoxy)pyridin-3-ylmethyl]-7,9-dihydropurin-8-one;
   6-amino-9-[5-chloro-6-(2-dimethylaminoethoxy)pyridin-3-ylmethyl]-2-(2-methoxyethoxy)-7,9-dihydropurin-8-one;
   6-amino-9-[5-chloro-6-(3-dimethylamminiopropoxy)pyridin-3-ylmethyl]-2-(2-methoxyethoxy)-7,9-dihydropurin-8-one;
   6-amino-9-[5-chloro-6-(3-dimethylamminio-2,2-dimethylpropoxy)pyridin-3-ylmethyl]-2-(2-methoxyethoxy)-7,9-dihydropurin-8-one;
   6-amino-9-[5-chloro-6-(2-pyrrolidin-1-ylethoxy)pyridin-3-ylmethyl]-2-(2-methoxyethoxy)-7,9-dihydropurin-8-one; 6-amino-9-{5-chloro-6-[3-(4-methylpiperazin-1-yl)propoxy]pyridin-3-ylmethyl}-2-(2-methoxythoxy)-7,9-dihydropurin-8-one;
   6-amino-9-[5-chloro-6-(1-methylpiperidine-4-yloxy)pyridin-3-ylmethyl]-2-(2-methoxyethoxy)-7,9-dihydropurin-8-one;
   6-amino-2-(2-methoxyethoxy)-9-[6-(3-morpholin-4-yl-propyl)pyridin-3-ylmethyl]-7,9-dihydropurin-8-one;
   6-amino-2-(2-methoxyethoxy)-9-[6-(3-dimethylaminopropyl)pyridin-3-ylmethyl]-7,9-dihydropurin-8-one;
   6-amino-2-(2-methoxyethoxy)-9-[6-(1-methylpiperidine-2-ylmethoxy)pyridin-3-ylmethyl]-7,9-dihydropurin-8-one;
   6-amino-2-(2-methoxyethoxy)-9-[6-(1-methylpyrrolidin-2-ylmethoxy)pyridin-3-ylmethyl]-7,9-dihydropurin-8-one;
   6-amino-9-[6-(1-ethylpiperidine-3-yloxy)pyridin-3-ylmethyl]-2-(2-methoxyethoxy)-7,9-dihydropurin-8-one;
   6-amino-9-[6-(1-isopropylpyrrolidin-3-yloxy)pyridin-3-ylmethyl]-2-(2-methoxyethoxy)-7,9-dihydropurin-8-one;
   6-amino-2-butoxy-9-{6-[2-(4-methylpiperazin-1-yl)ethoxy]pyridin-3-ylmethyl}-7,9-dihydropurin-8-one;
   6-amino-2-butoxy-9-{6-[3-(4-methylpiperazin-1-yl)propoxy]pyridin-3-ylmethyl}-7,9-dihydropurin-8-one;
   6-amino-2-(2-methoxyethoxy)-9-[6-(1-propylpiperidin-4-yloxy)pyridin-3-ylmethyl]-7,9-dihydropurin-8-one;
   6-amino-9-[6-(1-isopropylpiperidin-4-yloxy)pyridin-3-ylmethyl]-2-(2-methoxyethoxy)-7,9-dihydropurin-8-one,
   or a pharmaceutically acceptable salt thereof.
[21] A pharmaceutical composition comprising as an active ingredient the adenine compound according to any one of [1] to [20], or a pharmaceutically acceptable salt thereof.
[22] TLR7 activating agent comprising as an active ingredient the adenine compound according to any one of [1] to [20], or a pharmaceutically acceptable salt thereof.
[23] An immune-response modifier comprising as an active ingredient the adenine compound according to any one of [1] to [20], or a pharmaceutically acceptable salt thereof.
[24] A therapeutic or prophylactic agent for allergic diseases, viral diseases or cancer, which comprises as an active ingredient the adenine compound according to any one of [1] to [20], or a pharmaceutically acceptable salt thereof.
[25] A therapeutic or prophylactic agent for asthma, COPD, allergic rhinitis, allergic conjunctivitis, atopic dermatitis, cancer, hepatitis B, hepatitis C, HIV, HPV, bacterial infectious disease or dermatitis, which comprises as an active ingredient the adenine compound according to any one of [1] to [20], or a pharmaceutically acceptable salt thereof.

The present invention enables to provide useful and novel adenine compounds as a therapeutic or preventive agent for allergic disease, viral disease or cancer, etc.

### BEST MODE FOR CARRYING OUT THE INVENTION

The embodiments of the present invention are explained in detail below.
The term "halogen" as used herein includes fluorine, chlorine, bromine or iodine, preferably fluorine or chlorine.
The term "alkyl" includes straight chain or branched chain alkyl with 1 to 12 carbon atom(s), particularly, methyl, ethyl, propyl, 1-methylethyl, butyl, 2-methylpropyl, 1-methylpropyl, 1, 1-dimethylethyl, pentyl, 3-methylbutyl, 2-methylbutyl, 2,2-dimethylpropyl, 1-ethylpropyl, 1, 1-dimethylpropyl, hexyl, 4-methylpentyl, 3-methylpentyl, 2-methylpentyl, 1-methylpentyl, 3,3-dimethylbutyl, 2,2-dimethylbutyl, 1, 1-dimethylbutyl, 1 2-dimethylbutyl, heptyl, 1-methylhexyl, 1-ethylpentyl, octyl, 1-methylheptyl, 2-ethylhexyl, nonyl, decyl, etc. Preferable one is alkyl with 1 to 6 carbon atom(s), more preferably, alkyl with 1 to 4 carbon atom(s).
The term "alkenyl" includes straight chain or branched chain alkenyl with 2 to 10 carbon atoms, particularly, ethenyl, propenyl, 1-methylethenyl, butenyl, 2-methylpropenyl, 1-methylpropenyl, pentenyl, 3-methylbutenyl, 2-methylbutenyl, 1-ethylpropenyl, hexenyl, 4-methylpentenyl, 3-methylpentenyl, 2-methylpentenyl, 1-methylpentenyl, 3,3-dimethylbutenyl, 1 2-dimethylbutenyl, heptenyl, 1-methylhexenyl, 1-ethylpentenyl, octenyl, 1-methylheptenyl, 2-ethylhexenyl, nonenyl, decenyl, etc. Preferable one is alkenyl with 2 to 6 carbon atoms, more preferably, alkenyl with 2 to 4 carbon atoms.
The term "alkynyl" includes straight chain or branched chain alkynyl with 1 to 10 carbon atoms, particularly, ethynyl, propynyl, butynyl, pentynyl, 3-methylbutynyl, hexynyl, 4-methylpentynyl, 3-methylpentynyl, 3,3-dimethylbutynyl, heptynyl, octynyl, 3-methylheptynyl, 3-ethylhexynyl, nonyl, or decynyl, etc. Preferable one is alkynyl with 2 to 6 carbon atoms, more preferably, alkynyl with 2 to 4 carbon atoms.

The term "cycloalkyl" includes 3- to 8-membered monocyclic cycloalkyl, particularly, cyclopropyl, cyclobutyl, cyclopentyl, cyclohexyl, cycloheptyl or cyclooctyl. Preferable one is 4- to 6-membered cycloalkyl.
The term "cycloalkoxy" includes 3- to 8-membered monocyclic cycloalkoxy, particularly, cyclopropyloxy, cyclobutyloxy, cyclopentyloxy, cyclohexyloxy, cycloheptyloxy or cyclooctyloxy.

The term "aryl" includes 6- to 10-membered aryl, particularly, phenyl, 1-naphthyl or 2-naphthyl. Preferable one is phenyl.
The term "heteroaryl" includes 5- to 10-membered mono- or bi-cyclic heteroaryl containing 1 to 4 heteroatom(s) selected from 0 to 2 nitrogen(s), 0 to 1 oxygen and 0 to 1 sulfur, particularly, furyl, thienyl, pyrrolyl, pyridyl, indolyl, isoindolyl, quinolyl, isoquinolyl, pyrazolyl, imidazolyl, pyrimidinyl, pyrazinyl, pyridazinyl, thiazolyl, oxazolyl, etc. Substituents may bind on any carbon or nitrogen atom where it may be kept in chemically stable state without any limitation for binding positions. Preferable one is 5- or 6-membered heteroaryl.
The term "saturated heterocycle" includes 4- to 10-membered mono- or bi-cyclic saturated heterocycle containing 1 to 4 heteroatom(s) selected from 0 to 3 nitrogen(s), 0 to 1 oxygen and 0 to 1 sulfur, particularly, azetidinyl, pyrrolidinyl, piperidinyl, piperazinyl, perhydroazepinyl, perhydrodiazepinyl (homopiperazinyl), morpholinyl, thiomorpholinyl, 1-oxothiomorpholinyl, 1, 1-dioxothiomorpholinyl, tetrahydrofuranyl, etc. Substituents may bind on any carbon or nitrogen atom where it may be kept in chemically stable state without any limitation for binding positions. Preferable one is 4- to 8-membered monocyclic saturated heterocycle, more preferably, 4- to 6-membered saturated heterocycle.

The term "alkylene" includes straight chain or branched chain alkylene with 1 to 12 carbon atom(s), particularly, methylene, ethylene, trimethylene, tetramethylene, pentamethylene, hexamethylene, heptamethylene, octamethylene, nonamethylene, decamethylene, 1-methylmethylene, 1-ethylmethylene, 1-propylmethylene, 1-methylethylene, 2-methylethylene, 1-methyltrimethylene, 2-methyltrimethylene, 2-methyltetramethylene, or 3-methylpentamethylene, etc. Preferable one is straight chain or branched chain alkylene with 1 to 10 carbon atom(s), more preferably, with 1 to 8 carbon atom(s), more preferably, with 1 to 6 carbon atom(s).
The substituents of the substituted alkylene include hydroxy, amino, alkylamino, dialkylamino.

The term "haloalkyl" includes alkyl substituted with 1 to 5 of the same or different halogen(s), particularly, trifluoromethyl, 2,2,2-trifluoroethyl, 2,2-difluoroethyl, 2-fluoroethyl, 4,4,4-trifluorobutoxy, pentafluoroethyl, etc.
The term "alkoxy" includes straight chain or branched chain alkoxy with 1 to 10 carbon atom(s), particularly, methoxy, ethoxy, propoxy, 1-methylethoxy, butoxy, 2-methylpropoxy, 1-methylpropoxy, 1, 1-dimethylethoxy, pentoxy, 3-methylbutoxy, 2-methylbutoxy, 2,2-dimethylpropoxy, 1-ethylpropoxy, 1, 1-dimethylpropoxy, hexyloxy, 4-methylpentyloxy, 3-methylpentyloxy, 2-methylpentyloxy, 1-methylpentyloxy, 3,3-dimethylbutoxy, 2,2-dimethylbutoxy, 1, 1-dimethylbutoxy, 1 2-dimethylbutoxy, heptyloxy, 1-methylhexyloxy, 1-ethylpentyloxy, octyloxy, 1-methylheptyloxy, 2-ethylhexyloxy, nonyloxy, decyloxy, etc. Preferable one is alkoxy with 1 to 6 carbon atom(s), more preferably, alkoxy with 1 to 4 carbon atom(s).
The term "haloalkoxy" includes alkoxy substituted with 1 to 5 of the same or different halogen(s), particularly, trifluoromethoxy, 2,2,2-trifluoroethoxy, 2,2-difluoroethoxy, 2-fluoroethoxy, 4, 4, 4-trifluorobutoxy, pentafluoroethoxy, etc.

The term "alkyl" in "alkylthio", "alkylcarbonyl", "alkylcarbonyloxy", "alkylsulfonyl", "alkylsulfinyl", "alkylamino", "dialkylamino", "alkylcarbamoyl", "dialkylcarbamoyl", "alkylsulfamoyl" and "dialkylsulfamoyl" includes the same as the alkyl group as defined above.
The term "alkylthio" includes straight chain or branched chain alkylthio with 1 to 10 carbon atom(s), particularly, alkylthio with 1 to 6 carbon atom(s), more preferably, alkylthio with 1 to 4 carbon atom(s).
The term "alkylcarbonyl" particularly includes straight chain or branched chain alkylcarbonyl with 2 to 11 carbon atom(s), preferably, with 2 to 6 carbon atom(s), more preferably, with 2 to 5 carbon atom(s).
The term "alkylcarbonyloxy" includes straight chain or branched chain alkylcarbonyloxy with 2 to 11 carbon atoms, more preferably, with 2 to 6 carbon atoms, more preferably, with 2 to 5 carbon atoms.
The term "alkylsulfonyl" includes straight chain or branched chain alkylsulfonyl with 1 to 10 carbon atom(s), more preferably, with 1 to 6 carbon atom(s), more preferably, with 1 to 4 carbon atom(s).
The term "alkylsulfinyl" includes straight chain or branched chain alkylsulfinyl with 1 to 10 carbon atom(s), more preferably, with 1 to 6 carbon atom(s), more preferably, with 1 to 4 carbon atom(s).
The term "alkylamino" includes amino substituted with an alkyl group having 1 to 10 carbon atom(s), preferably, 1 to 6 carbon atom(s), more preferably, 1 to 4 carbon atom(s). The term "dialkylamino" includes amino substituted with the same or different two alkyl group(s) having 1 to 10 carbon atom(s), preferably, 1 to 6 carbon atom(s), more preferably, 1 to 4 carbon atom(s).
The term "alkylcarbamoyl" includes carbamoyl substituted with an alkyl group having 1 to 10 carbon atom(s), preferably, 1 to 6 carbon atom(s), more preferably, 1 to 4 carbon atom(s). The term "dialkylcarbamoyl" includes carbamoyl substituted with the same or different two alkyl group(s) having 1 to 10 carbon atom(s), preferably, 1 to 6 carbon atom(s), more preferably, 1 to 4 carbon atom(s).
The term "alkylsulfamoyl" includes sulfamoyl substituted with an alkyl group having 1 to 10 carbon atom(s), preferably, 1 to 6 carbon atom(s), more preferably, 1 to 4 carbon atom(s). The term "dialkylsulfamoyl" includes sulfamoyl substituted with the same or different two alkyl group(s) having 1 to 10 carbon atom(s), preferably, 1 to 6 carbon atom(s), more preferably, 1 to 4 carbon atom(s).

The term "alkoxy" in "alkoxycarbonyl" includes the same as the alkoxy group as defined above. Specifically, "alkoxycarbonyl" includes straight chain or branched chain alkoxycarbonyl with 2 to 11 carbon atoms, preferably, with 2 to 6 carbon atoms, more preferably, with 2 to 5 carbon atoms.

The term "cycloalkyl" in "cycloalkylcarbonyl", "cycloalkylsulfonyl" and "cycloalkylsulfinyl" includes the same as the cycloalkyl group as defined above.
The term "cycloalkylcarbonyl" particularly includes 3- to 8-membered monocyclic cycloalkylcarbonyl, preferably, 4- to 6-membered monocyclic cycloalkylcarbonyl.
The term "cycloalkylsulfonyl" particularly includes 3- to 8-membered monocyclic cycloalkylsulfonyl, preferably, 4- to 6-membered monocyclic cycloalkylsulfonyl.
The term "cycloalkylsulfinyl" particularly includes 3- to 8-membered monocyclic cycloalkylsulfinyl, preferably, 4- to 6-membered monocyclic cycloalkylsulfinyl.
The term "cycloalkoxy" in "cycloalkoxycarbonyl" includes the same as the cycloalkoxy group as defined above. Particularly, "cycloalkoxycarbonyl" includes 3- to 8-membered monocyclic cycloalkoxycarbonyl, preferably, 4- to 6-membered cycloalkoxycarbonyl.

The term "aryl" in "aryloxy", "arylcarbonyl", "aryloxycarbonyl", "arylsulfonyl", "arylsulfinyl", "arylalkyl" and "aryloxyalkyl" includes the same as the aryl group as defined above. Particularly, "aryloxy" includes phenoxy, 1-naphthoxy or 2-naphthoxy. Particularly, "arylcarbonyl" includes benzoyl, 1-naphthaloyl or 2-naphthaloyl. Particularly, "aryloxycarbonyl" includes phenoxycarbonyl, 1-naphthoxycarbonyl or 2-naphthoxycarbonyl. Particularly, "arylsulfonyl" includes phenylsulfonyl, 1-naphthylsulfonyl, 2-naphthylsulfonyl. Particularly, "arylsulfinyl" includes phenylsulfinyl, 1-naphthylsulfinyl, 2-naphthylsulfinyl. The term "arylalkyl" includes straight chain or branched chain alkyl with 1 to 10 carbon atoms, particularly, with 1 to 6 carbon atoms, more particularly, with 1 to 4 carbon atoms, which is substituted with aryl. The "aryl" includes phenyl. The "aryloxyalkyl" includes straight chain or branched chain alkyl with 1 to 10 carbon atoms, particularly, with 1 to 6 carbon atoms, more particularly, with 1 to 4 carbon atoms, which is substituted with the "aryloxy" as defined above. The "aryloxy" includes phenoxy.
The term "heteroaryl" in "heteroaryloxy", "heteroarylcarbonyl", "heteroaryloxycarbonyl", "heteroarylsulfonyl", "heteroarylsulfinyl", "heteroarylalkyl", and "heteroaryloxyalkyl" includes the same as the heteroaryl as defined above. Particularly, "heteroaryloxy" includes pyrrolyloxy, pyridyloxy, pyrazinyloxy, pyrimidinyloxy, pyridazinyloxy, furyloxy, thienyloxy. Particularly, "heteroarylcarbonyl" includes pyrrolylcarbonyl, pyridylcarbonyl, pyrazinylcarbonyl, pyrimidinylcarbonyl, pyridazinylcarbonyl, furylcarbonyl, thienylcarbonyl, etc. Particularly, "heteroaryloxycarbonyl" includes pyrrolyloxycarbonyl, pyridyloxycarbonyl, pyrazinyloxycarbonyl, pyrimidinyloxycarbonyl, pyridazinyloxycarbonyl, furyloxycarbonyl, thienyloxycarbonyl. Particularly, "heteroarylsulfonyl" includes pyrrolylsulphonyl, pyridylsulphonyl, pyrazinylsulphonyl, pyrimidinylsulphonyl, pyridazinylsulphonyl, furylsulphonyl, thienylsulphonyl. Particularly, "heteroarylsulfinyl" includes pyrrolylsulfinyl, pyridylsulfinyl, pyrazinylsulfinyl, pyrimidinylsulfinyl, pyridazinylsulfinyl, furylsulfinyl, thienylsulfinyl. The "heteroarylalkyl" includes straight chain or branched chain alkyl with 1 to 10 carbon atoms, particularly, with 1 to 6 carbon atoms, more particularly, with 1 to 4 carbon atoms, which is substituted with the heteroaryl group as defined above. The "heteroaryloxyalkyl" includes straight chain or branched chain alkyl with 1 to 10 carbon atoms, particularly, with 1 to 6 carbon atoms, more particularly, with 1 to 4 carbon atoms, which is substituted with the heteroaryloxy group as defined above. The "heteroaryl" includes pyrrolyl, pyridyl, pyrazinyl, pyrimidinyl, pyridazinyl, furyl, thienyl.

The term "nitrogen-containing saturated heterocycle" used herein includes, preferably, 4- to 8-membered nitrogen-containing saturated heterocycle containing 1 to 4 heteroatom(s) selected from 1 to 3 nitrogen(s), 0 to 1 oxygen and 0 to 1 sulfur.
The "nitrogen-containing saturated heterocycle" formed by combining R² with L² may include pyrrolidine, piperidine, piperazine, morpholine or the like.
The "NR²R³", wherein R² is combined together with R³ to form a nitrogen-containing saturated heterocycle, includes preferably the nitrogen-containing saturated heterocycle of the formulae (2) to (8):

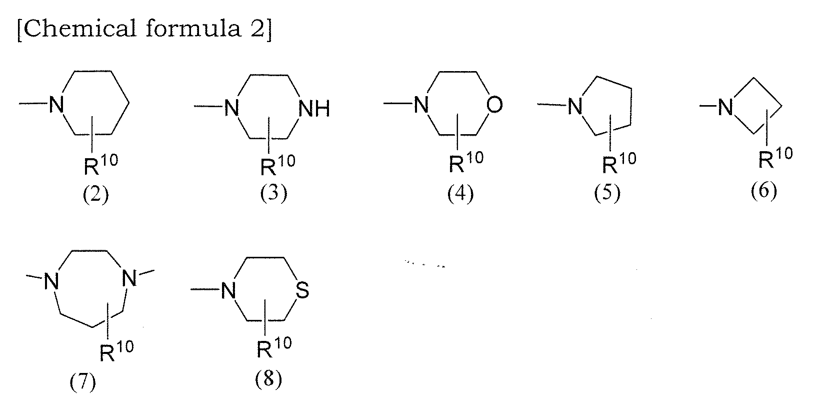

wherein R¹⁰ is the substituent of the nitrogen-containing saturated heterocycle as defined above, which may bind to any carbon atoms and imino.
Preferably, R¹⁰ may include halogen; hydroxy; oxo; alkyl with 1 to 6 carbon atom(s), alkoxy with 1 to 6 carbon atom(s), alkylcarbonyl with 2 to 6 carbon atoms, wherein said alkyl, alkoxy and alkylcarbonyl is optionally substituted with 1 to 3 substituent(s) selected from halogen, cyano, hydroxy, alkoxy with 1 to 6 carbon atom(s), substituted or unsubstituted phenyl, substituted or unsubstituted phenoxy, amino optionally substituted with the same or different one or two alkyl(s) with 1 to 6 carbon atom(s), and carbamoyl optionally substituted with the same or different one or two alkyl(s) with 1 to 6 carbon atom(s); 3- to 6-membered cycloalkyl; amino optionally substituted with the same or different one or two alkyl(s); alkylcarbonylamino with 2 to 6 carbon atoms; carbamoyl optionally substituted with the same or different one or two alkyl(s); substituted or unsubstituted phenyl; substituted or unsubstituted phenoxy, substituted or unsubstituted phenyl-alkyl with 1 to 6 carbon atom(s); substituted or unsubstituted phenoxy-alkyl with 1 to 6 carbon atom(s); substituted or unsubstituted 5- to 6-membered heteroaryl; substituted or unsubstituted 5- to 6-membered heteroaryloxy; substituted or unsubstituted 5- to 6-membered heteroaryl-alkyl with 1 to 6 carbon atom(s); and substituted or unsubstituted 5- to 6-membered heteroaryloxy-alkyl with 1 to 6 carbon atom(s). Said substituted phenyl, substituted phenoxy, substituted heteroaryl and substituted heteroaryloxy may be those substituted with one or more group(s) selected from halogen, hydroxy, alkyl with 1 to 6 carbon atom(s), alkoxy with 1 to 6 carbon atom(s), amino optionally substituted with the same or different one or two alkyl(s).
In formula (1), "NR²R³" includes preferably the groups of the formulae (3) and (7) as represented above, when the group L² is a single bond.

The aromatic carbocycle in A includes benzene ring or naphthalene ring without any limitation for binding position. Since said ring A is divalent in the formula (1), any two hydrogens on the ring should involve in the linkages.
The aromatic heterocycle in A includes 5- to 10-membered mono- or bi-cyclic aromatic heterocycle containing 1 to 4 heteroatom(s) selected from 0 to 3 nitrogen(s), 0 to 1 oxygen and 0 to 1 sulfur without any limitation for binding position, where it may be kept in chemically stable state. The aromatic heterocycle particularly includes furan, thiophen, pyrrole, pyridine, indole, isoindole, quinoline, isoquinoline, pyrazole, imidazole, pyrimidine, pyrazine, pyridazine, thiazole or oxazole, etc. 5- to 6- membered monocyclic aromatic heterocycle is preferable. Since ring A is divalent in the formula (1), any two hydrogens on the ring should involve in the linkages.
The aromatic carbocycle and aromatic heterocycle in A may be substituted with the same or different 1 to 3 substituent(s), wherein the substituent includes halogen, hydroxy, nitro, alkyl, haloalkyl, alkoxy, haloalkoxy, alkylcarbonyl, alkylsulfonyl alkylsulfinyl, and amino optionally substituted with one or two alkyl(s).
In case that L² is alkylene and any one to three of methylene group(s) in said alkylene is replaced by oxygen, sulfur, SO, SO₂ carbonyl, NR⁴CO, CONR⁴, NR⁴SO₂, SO₂NR⁴, NR⁴CO₂, OCONR⁴, NR⁵CONR⁴, NR⁶C(=NR⁴)NR⁵, C(=NR⁴)NR⁵ wherein R⁴, R⁵ and R⁶ are independently hydrogen or alkyl, it is not limited which methylene group should be replaced, so long as the methylene is not bind to NR²R³ and kept in chemically stable state. Said methylene group may be replaced preferably with oxygen, sulfur, SO, SO₂ or carbonyl, more preferably with oxygen.
In a preferable embodiment, the compounds of the formula (1) may be that wherein L¹ and L² are both methylene, or L¹ is methylene and L² is the group of the formula: -O- (CH₂)ₙ wherein n is an integer of 2 to 4.
In another preferable embodiment, the compounds of the formula (1) may be that wherein A is pyridine; L¹ is methylene; L² is the group of the formula: -O- (CH₂)ₙ wherein n is an integer of 2 to 4, NR²R³ is amino, alkylamino, dialkylamino, or the group of any one of the formulae (2) to (8) as represented above.
In another preferable embodiment, the compounds of the formula (1) may be that wherein A is pyridine; L¹ is methylene; L² is a single bond, NR²R³ is the group of the formula (3) as represented above.

As used herein, "4- to 8-membered saturated heterocycle", to which two substituents of substituted amino are combined together with the mitrogen atom, includes 4- to 8-membered saturated heterocycle with 1 to 4 heteroatom(s) selected from 1 to 3 nitrogen(s), 0 to 1 oxygen and 0 to 1 sulfur, and it may bind on any positions without any limitation where it may be kept in chemically stable state. The sulfur atom may be substituted with 1 or 2 oxygen atom(s). Suitable examples are azetidine, pyrrolidine, piperidine, piperazine, morpholine, thiomorpholine, thiomorpholin-1-oxide, thiomorpholine-1,1-dioxide, 1,4-perhydrodiazepine, perhydroazepine, imidazolidine, oxazolidine, etc.

In formula (1), X is preferably oxygen or a single bond. In case that X is NR⁷, R⁷ is preferably hydrogen or alkyl with 1 to 3 carbon atom(s), more preferably, hydrogen or methyl.
In formula (1), R¹ is preferably substituted or unsubstituted straight chain or branched chain alkyl with 1 to 6 carbon atom(s), more preferably, substituted or unsubstituted straight chain alkyl with 1 to 4 carbon atom(s), and particularly incluses substituted or unsubstituted methyl, ethyl, propyl, butyl, pentyl, 1-methylethyl, 1-methylpropyl, 2-methylbutyl, etc.
In case that R¹ is substituted alkyl, the substituent of the alkyl preferably includes halogen, hydroxy, straight chain or branched chain alkoxy with 1 to 4 carbon atom(s), straight chain or branched chain alkylthio with 1 to 4 carbon atom(s), 3- to 6-membered cycloalkyl, phenyl, 5- to 6-membered heteroaryl, wherein said cycloalkyl, phenyl and heteroaryl are optionally substituted with halogen, hydroxy, alkyl with 1 to 6 carbon atom(s) or alkoxy with 1 to 6 carbon atom(s). More preferably, the substituent includes fluorine, hydroxy, cyclopropyl or straight chain or branched chain alkoxy with 1 to 3 carbon atom(s), which may be substituted with the same or different one or more, preferably one to five, more preferably one to three, substituent(s). The R¹ particularly includes methyl, ethyl, propyl, butyl, pentyl, 1-methylethyl, 1-methylpropyl, 2-methylbutyl, 2-methoxyethyl, cyclopropylmethyl, 2,2,2-trifluoroethyl, 4, 4, 4-trifluorobutyl, 4-pyridylmethyl, etc.
The adenine compounds of the present invention are intended to include all tautomers, geometric isomers or stereoisomers, and optionally, a mixture thereof depending on the kinds of substituents.
In other words, in case that one or more asymmetric carbon atom(s) may exist in the compound of the formula (1), diastereomers and enantiomers may also exist, and the present invention includes the diastereomers, the enantiomers, and mixtures and isolated forms thereof.

Additionally, the adenine compound of the formula (1) and a tautomer thereof are chemically equivalent, and the adenine compound of the present invention also includes the tautomer thereof. Particularly, the tautomer is in the form of hydroxy of the formula (1'):

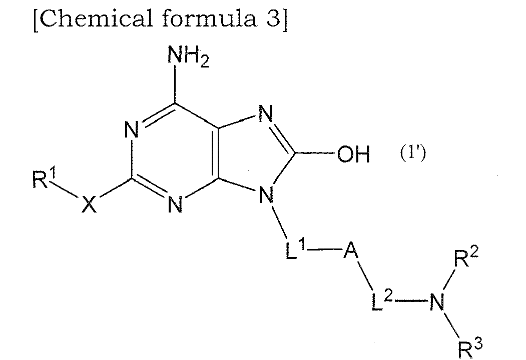

wherein R¹ R², R³, A, X, L¹ and L² are as defined above.
A pharmaceutically acceptable salt includes acid addition salt and base addition salt. For example, the acid addition salt includes an inorganic acid salt such as hydrochloride, hydrobromide, sulfate, hydroiodide, nitrate, phosphate, etc., and an organic acid salt such as citrate, oxalate, acetate, formate, propionate, benzoate, trifluoroacetate, fumarate, maleate, succinate, tartrate, lactate, pyruvate, methanesulfonate, benzenesulfonate, para-toluenesulfonate, etc., and the base addition salt includes an inorganic base salt such as sodium salt, potassium salt, calcium salt, magnesium salt, ammonium salt, etc., and an organic base salt such as triethyl ammonium salt, triethanol ammonium salt, pyridinium salt, diisopropyl ammonium salt, etc., and additionally, amino acid salt such as basic or acidic amino acid including arginine, aspartic acid and glutamic acid. The compound of the formula (1) may be a hydrate, or a solvate such as ethanolate.

The compound of the general formula (1) may be prepared by the following methods. Starting compounds which are not described below may be prepared according to the following methods or known methods or those similar thereto.

### Preparation Method 1

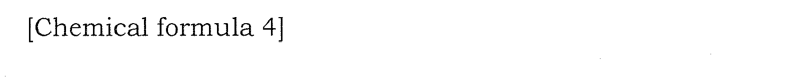

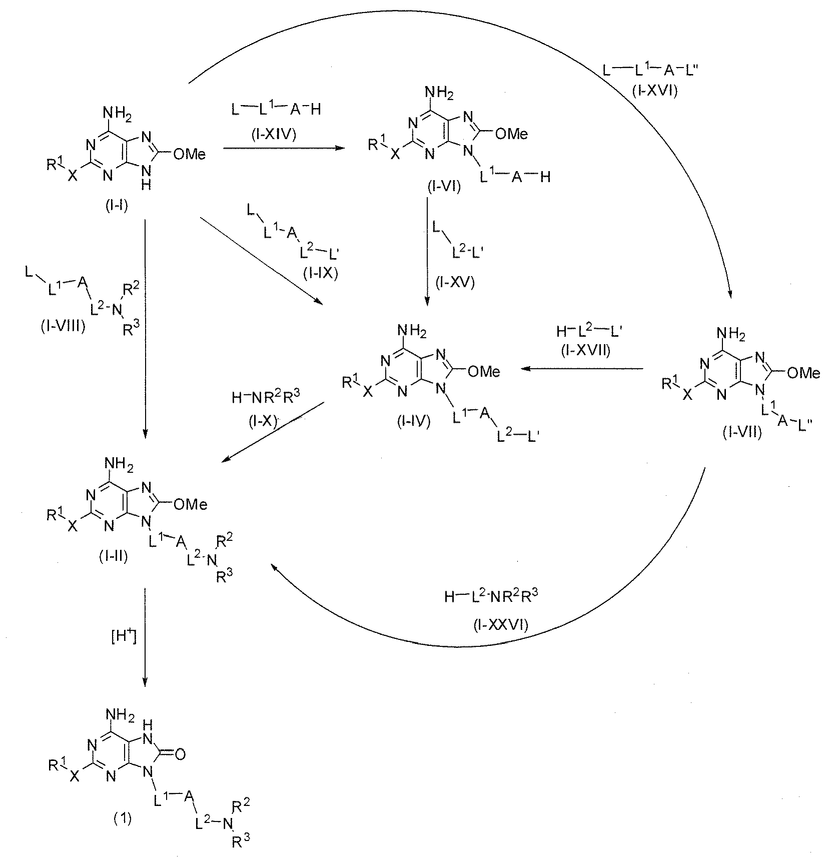

In the above Scheme, L, L' and L" are leaving groups which may be the same or different each other; A, R¹, R², R³, X, L¹ and L² are as defined above.
In case that the compound or the intermediate thereof of the present invention has a functional group such as amino, carboxy, hydroxy or oxo, a protection or deprotection technique may be applied, if necessary. A preferable protective group, a method for protection and deprotection are particularly described in "Protective Groups in Organic Synthesis 2nd Edition (John Wiley & Sons, Inc.; 1990)", etc.
Compound (I-I) may be reacted with compound (I-VIII) in the presence of a base to give compound (I-II). For example, the base which may be used therein includes alkali metal carbonate such as sodium carbonate or potassium carbonate, alkaline earth metal carbonate such as calcium carbonate, metal hydroxide such as sodium hydroxide or potassium hydroxide, metal hydride such as sodium hydride, or metal alkoxide such as potassium t-butoxide, etc. For example, the solvent which may be used therein includes aprotic solvent such as N,N-dimethylformamide, dimethylsulfoxide or acetonitrile, halogenated hydrocarbon solvent such as carbon tetrachloride, chloroform or methylene chloride, ether solvent such as diethyl ether, tetrahydrofuran or 1,4-dioxane, etc. For example, the reaction temperature is selected from the range of about 0°C to around a boiling point of the solvent.

The compound of formula (1) may be obtained by treating the compound (I-II) under an acidic condition. For example, the acid used in the acid-treatment includes an inorganic acid such as hydrochloric acid, hydrobromic acid or sulfuric acid, or an organic acid such as trifluoroacetic acid, etc. For example, the solvent which may be used therein includes water, or a mixture of water and an organic solvent. The organic solvent includes ether solvent such as diethyl ether or tetrahydrofuran, aprotic solvent such as N,N-dimethylformamide or acetonitrile, or alcoholic solvent such as methanol or ethanol, etc. The reaction temperature is, for example, selected from the range of room temperature to around a boiling point of the solvent. The conversion of methoxy on 8-position of the adenine ring into oxo by acid treatment may be carried out not in the final step but in any steps.

Compound (I-VIII) may be prepared by the following methods.

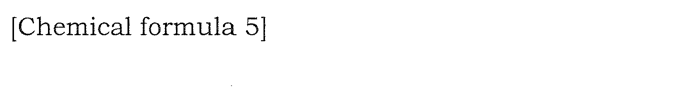

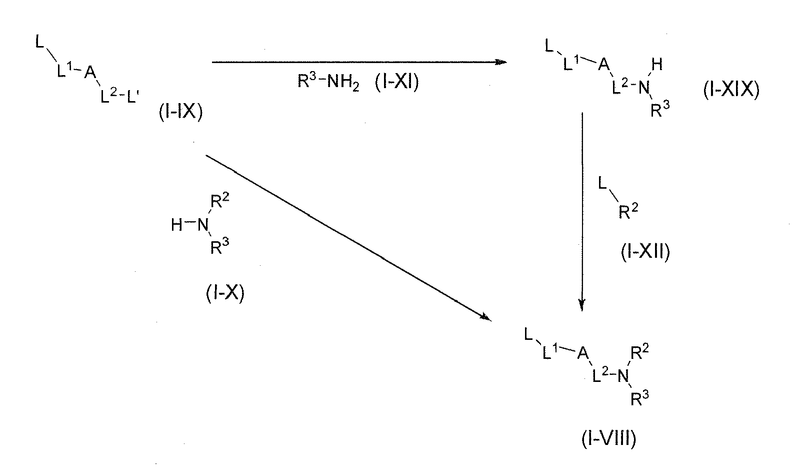

In the above Scheme, L, L', A, R², R³, L¹ and L² are as defined above.
Compound (I-IX) may be reacted with compound (I-X) in the similar manner to the above to give compound (I-VIII). Alternatively, compound (I-IX) may be reacted with compound (I-XI) in the similar manner to the above to give compound (I-XIX), followed by reacting with compound (I-XII) in the similar manner to the above to give compound (I-VIII). Compound (I-IX) may be known in the art or prepared from any known compound in a manner known to those skilled in the art.
In the preparation step from compound (I-I) to compound (I-II), compound (I-I) may be also reacted with compound (I-IX) in the similar manner to the above to give compound (I-IV), followed by reacting with compound (I-X) in the similar manner to the above to give compound (I-II).
In the preparation step from compound (I-I) to compound (I-IV), compound (I-I) may be also reacted with compound (I-XIV) in the similar manner to the above to give compound (I-VI), followed by reacting with compound (I-XV) in the similar manner to the above to give compound (I-IV).
In the preparation step from compound (I-I) to compound (I-II), compound (I-I) may be also reacted with compound (I-XVI) in the similar manner to the above to give compound (I-VII), followed by reacting with compound (I-XXVI) in the similar manner to the above to give compound (I-II).

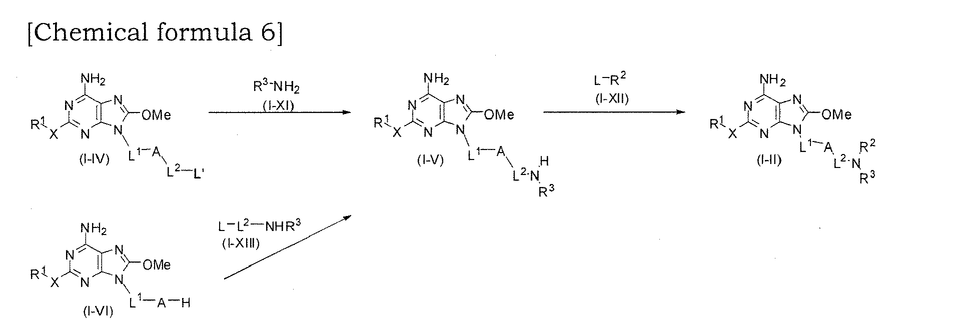

In the above Scheme, L, L', A, R¹, R², R³, X, L¹ and L² are as defined above. Compound (I-VI) can be further reacted with compound (I-XIII) to give compound (I-V). Alternatively, compound (I-V) may be obtained by the reaction of compound (I-IV) with compound (I-XI). Compound (I-V) may be reacted with compound (I-XII) to gene compound (I-II).

Compound (I-I) may be prepared according to the following methods.

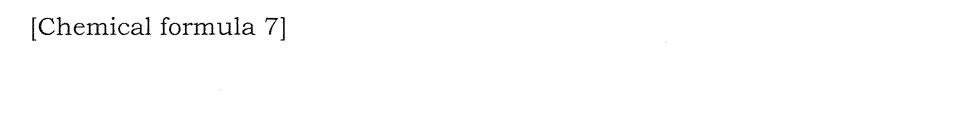

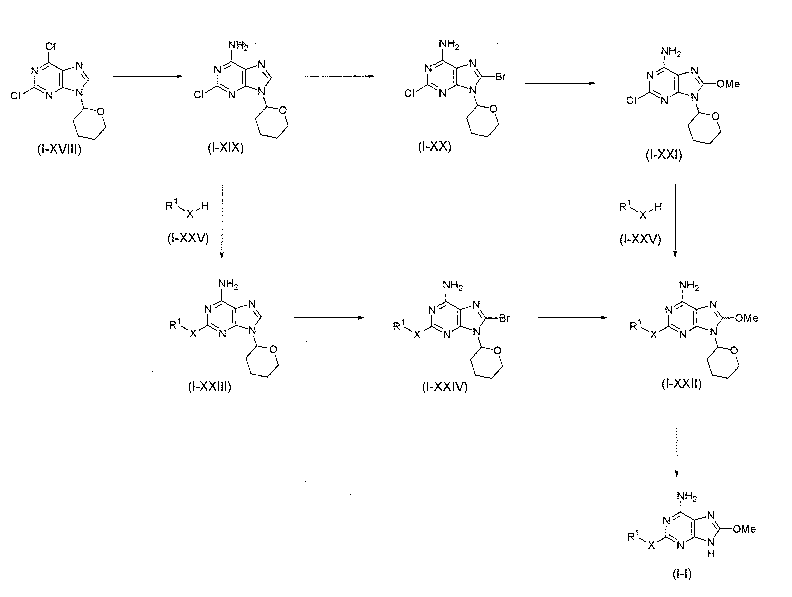

In the above Scheme, R¹ and X are as defined above.
Compound (I-XVIII) may be reacted with ammonia in aqueous solution, organic solvent or a mixture of water and organic solvent to give compound (I-XIX).
For example, the organic solvent includes alcoholic solvent such as methanol, ethanol, propanol or butanol, ether solvent such as tetrahydrofuran, 1,4-dioxane or diglyme, aprotic solvent such as acetonitrile, etc. For example, the reaction temperature is selected from the range of room temperature to 200°C. A reaction container such as autoclave may be used in the reaction.

Compound (I-XIX) may be brominated to give compound (I-XX). For example, a brominating agent which may be used therein includes bromine, hydrobromic acid perbromide or N-bromosuccinimide, etc., and for example, a reaction auxiliary such as sodium acetate may be added to the reaction. For example, the solvent which may be used therein includes halogenated hydrocarbon solvent such as carbon tetrachloride, methylene chloride or dichloroethane, ether solvent such as diethyl ether, acetic acid, or carbon disulfide, etc. For example, the reaction temperature is selected from the range of about 0°C to around a boiling point of the solvent.
Compound (I-XX) may be reacted with sodium methoxide to give compound (I-XXI).
For example, the organic solvent which may be used therein includes ether solvent such as diethyl ether, tetrahydrofuran or 1,4-dioxane, aprotic solvent such as N,N-dimethylformamide, or alcoholic solvent such as methanol, etc. For example, the reaction temperature is selected from the range of room temperature to around a boiling point of the solvent.
Compound (I-XX) may be also treated in an aqueous alkaline solution containing methanol to give compound (I-XXI).
The aqueous alkaline solution which may be used therein includes an aqueous solution of alkali metal hydroxide such as sodium hydroxide or potassium hydroxide. For example, the reaction temperature is selected from the range of room temperature to around a boiling point of the solvent.

Compound (I-XXI) may be reacted with compound (I-XXV) to give compound (I-XXII).
The reaction is carried out in the presence or absence of a base in case that X is NR⁷ wherein R⁷ is hydrogen or alkyl. For example, the base which may be used therein includes alkali metal carbonate such as sodium carbonate or potassium carbonate, alkaline earth metal carbonate such as calcium carbonate, metal hydroxide such as sodium hydroxide or potassium hydroxide, or an organic base such as triethylamine, diisopropylethylamine or 4-dimethylaminopyridine, etc. For example, the solvent which may be used therein includes ether solvent such as tetrahydrofuran, 1,4-dioxane or diglyme, alcoholic solvent such as propanol or butanol, or aprotic solvent such as N,N-dimethylformamide, or the reaction may be carried out in the absence of solvent. For example, the reaction temperature is selected from the range of about 50°C to 200°C.
The reaction is carried out in the presence of a base in case that X is oxygen or sulfur. For example, the base which may be used therein includes alkali metal such as sodium or potassium, or alkali metal hydride such as sodium hydride. For example, the solvent which may be used therein includes ether solvent such as tetrahydrofuran, 1,4-dioxane or diglyme, or aprotic solvent such as N,N-dimethylformamide or dimethylsulfoxide, or the reaction may be carried out in the absence of solvent. For example, the reaction temperature is selected from the range of about 50°C to 200°C.
The reaction may be carried out by oxidizing the corresponding intermediate for preparation wherein X is sulfur with oxone™ or m-chloroperbenzoic acid (mCPBA) in case that X is SO₂
Alternatively, in the preparation step from compound (I-XIX) to compound (I-XXII), compound (I-XXIII) may be synthesized in the similar manner to the above to give compound (I-XXIV), followed by obtaining compound (I-XXII).
Compound (I-XXII) may be treated with an acid in an organic solvent such as methanol to give compound (I-I).
For example, the acid which may be used therein includes an inorganic acid such as hydrochloric acid, hydrobromic acid or sulfuric acid, or an organic acid such as trifluoroacetic acid. For example, the solvent which may be used therein includes water, or a mixture of water and an organic solvent. The organic solvent includes ether solvent such as diethyl ether or tetrahydrofuran, aprotic solvent such as N,N-dimethylformamide or acetonitrile, or alcoholic solvent such as methanol or ethanol. For example, the reaction temperature is selected from the range of room temperature to around a boiling point of the solvent.

Compound (I-I) may be prepared according to the following methods in case that X is NR⁷CO wherein R⁷ is as defined above.

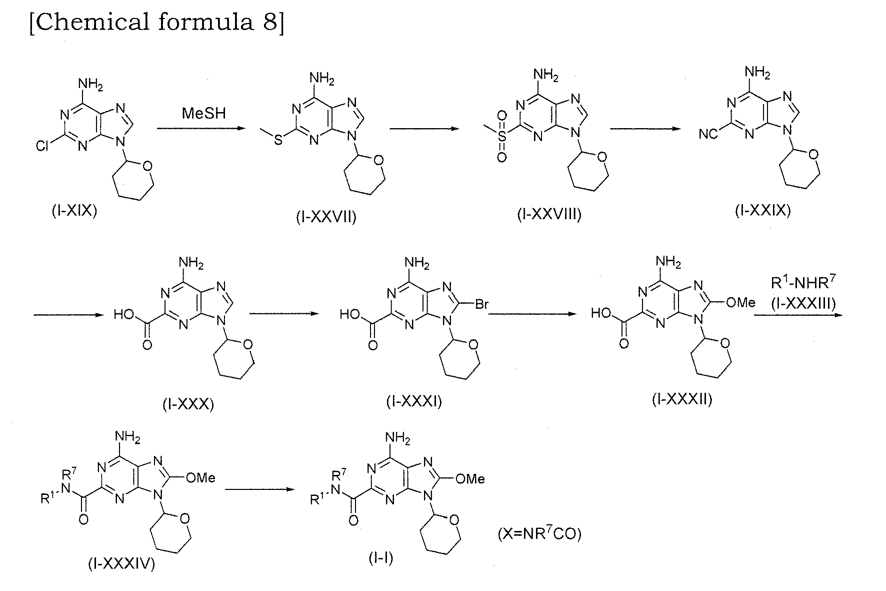

In the above Scheme, R¹ and R⁷ is as defined above.
Compound (I-XIX) is reacted with methanethiol in the presence of a base to give Compound (I-XXVII). Suitable base includes, for example, alkali metals such as sodium and potassium, or alkali metal hydrides such as sodium hydride.
Compound (I-XXVIII) may be prepared by oxidating compound (I-XXVII) with oxone or m-chloroperhydrobenzoate (mCPBA).
Compound (I-XXVIII) may be treated with sodium cyanide or potassium cyanide to give compound (I-XXIX).
Compound (I-XXIX) may be hydrolyzed with alkaline aqueous solution to give Compound (I-XXX).
Compound (I-XXX) may be brominated at 8-posion of the adenine in a similar manner to the above to give compound (I-XXXI), which is methoxylated to afford compound (I-XXXII).
Compound (I-XXXII) may be converted to an amide compound (I-XXXIV), which has been substituted with various substituents. For example, compound (I-XXXII) can be condensed with amine (compound (I-XXXIII)) in the presence of a condensing agent such as dicyclohexylcarbodiimide (DCC) can afford the corresponding amido compound (compound I-XXXIV).
Compound (I-XXXIV) may be treated with trifluoroacetate in an organic solvent such as methanol to geve Compound (I-I).

The compound of the general formula (1) may be also prepared by the following methods using compound (II-I) as a starting compound. The starting compound (II-I) is disclosed in WO 2002/085905 and WO 2004/029054 in detail. Starting compounds which are not described below may be prepared according to the following methods or known methods or those similar thereto.

### Preparation Method 2

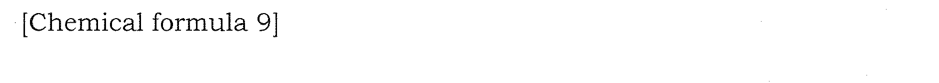

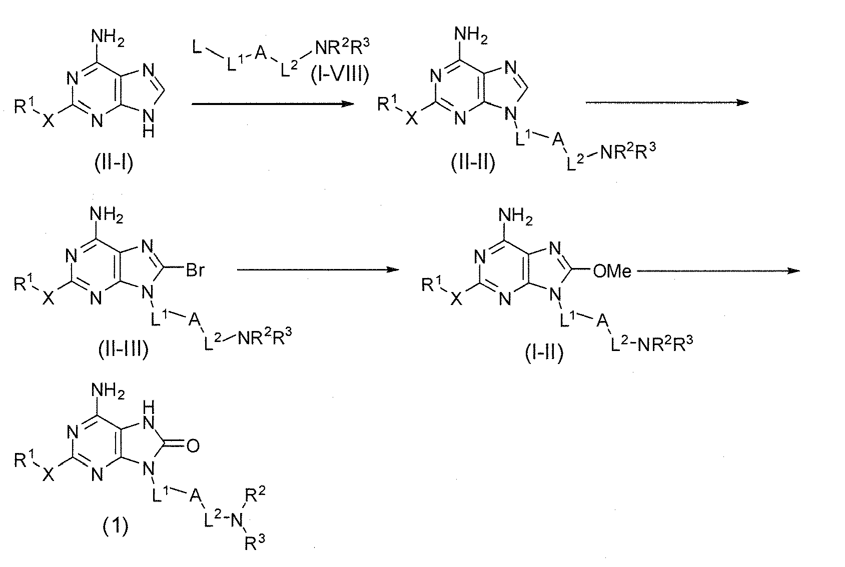

In the above Scheme, L, A, R¹, R², R³, X, L¹ and L² are as defined above.
Compound (II-I) may be reacted with compound (I-VIII) in the presence of a base to give compound (II-II). For example, the base which may be used therein includes alkali metal carbonate such as sodium carbonate or potassium carbonate, alkaline earth metal carbonate such as calcium carbonate, metal hydroxide such as sodium hydroxide or potassium hydroxide, metal hydride such as sodium hydride, or metal alkoxide such as potassium t-butoxide. For example, the solvent which may be used therein includes aprotic solvent such as N,N-dimethylformamide, dimethylsulfoxide or acetonitrile, halogenated hydrocarbon solvent such as carbon tetrachloride, chloroform or methylene chloride, ether solvent such as diethyl ether, tetrahydrofuran or 1,4-dioxane. For example, the reaction temperature is selected from the range of about 0°C to around a boiling point of the solvent.
Compound (1) may be obtained in a similar manner to Preparation Method 1 from compound (II-II).
Alternatively, in the preparation step from compound (II-I) to compound (II-II), compound (II-II) may be obtained via synthesis of compound (II-IV), compound (II-V) or compound (II-VI), in a similar manner to Preparation Method 1.

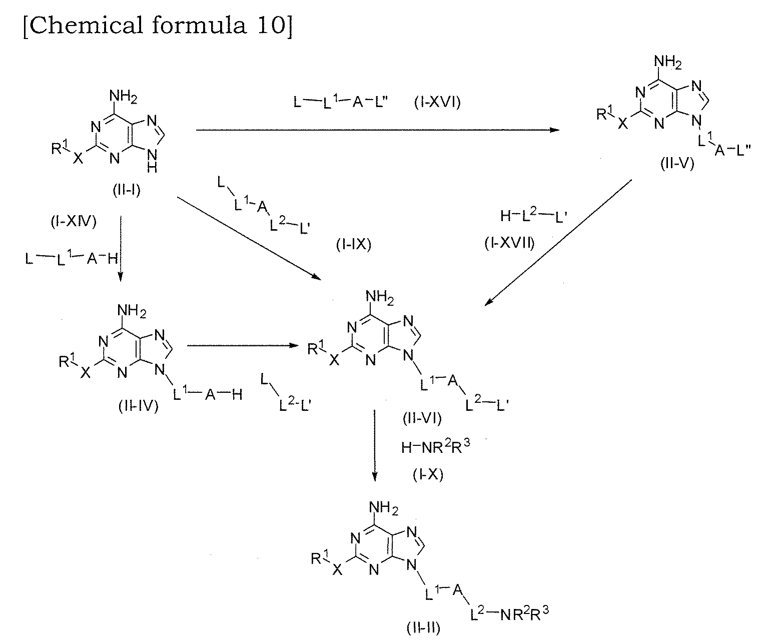

In the above Scheme, L, L', L", A, R¹, R², R³, X, L¹ and L² are as defined above.
Compound (II-II) may be brominated to give compound (II-III). For example, a brominating agent which may be used therein includes bromine, hydrobromic acid perbromide or N-bromosuccinimide, etc., and for example, a reaction auxiliary such as sodium acetate may be added to the reaction. For example, the solvent which may be used therein includes halogenated hydrocarbon solvent such as carbon tetrachloride, methylene chloride or dichloroethane, ether solvent such as diethyl ether, acetic acid, or carbon disulfide, etc. For example, the reaction temperature is selected from the range of about 0°C to around a boiling point of the solvent.
Compound (II-III) may be reacted with metal alkoxide such as sodium methoxide to give compound (I-II).
For example, the solvent which may be used in the reaction with metal alkoxide includes ether solvent such as diethyl ether, tetrahydrofuran or 1,4-dioxane, aprotic solvent such as N,N-dimethylformamide, or alcoholic solvent corresponding to metal alkoxide used therein such as methanol, etc. For example, the reaction temperature is selected from the range of room temperature to around a boiling point of the solvent.
Alternatively, compound (I-II) may be obtained using compound compound (II-I), compound (II-II), compound (II-IV), compound (II-V) and compound (II-VI), via bromination at 8-position and the steps in the similar manner to the above sinthesis from compound (II-I) to compound (II-II).

The adenine compounds, intermediates or starting compounds thereof with any functional groups in the present invention may be optionally subjected to homologation reaction, substituent introduction reaction or functional group transformation reaction, etc. in an appropriate step, or more specifically, in any halfway step of each preparation method described in the above Preparation Method 1 or 2 according to a conventional method known to those skilled in the art. For these reactions, a method described in "Jikken-Kagaku-Koza (edited by the Chemical Society of Japan, Maruzen)", or "Comprehensive Organic Transformation, Author: R. C. Larock, (VCH Publishers, Inc, 1989)", etc. may be used. The homologation reaction includes, for example, a method wherein ester is converted into hydroxymethyl using a reducing agent such as lithium aluminum hydride, followed by introducing a leaving group to introduce cyano, etc. The functional group transformation reaction includes, for exmaple, acylation or sulfonylation reaction using acid halide, sulfonyl halide, etc., a reaction using alkylating agent such as halogenated alkyl, carbon-carbon bond formation reaction such as hydrolysis reaction, Friedel-Crafts reaction or Wittig reaction, oxidation or reduction reaction, etc.
When the compound of the present invention or an intermediate thereof contains a functional group such as amino, carboxy, hydroxy or oxo in the present invention, a protection or deprotection technique may optionally be applied. A preferable protective group, a method for protection and deprotection are specifically described in "Protective Groups in Organic Synthesis 2nd Edition (John Wiley & Sons, Inc.; 1990)", etc.
The compound of the formula (1) or an intermediate for preparing the same of the present invention may be purified by a method known to those skilled in the art. For example, it may be purified by column chromatography (e.g., silica gel column chromatography, or ionexchange column chromatography), or recrystallization, etc. The solvent which may be used in the recrystallization includes, for example, alcoholic solvents such as methanol, ethanol or 2-propanol, ether solvents such as diethyl ether, ester solvents such as ethyl acetate, aromatic hydrocarbon solvents such as benzene or toluene, ketone solvents such as acetone, hydrocarbon solvents such as hexane, aprotic solvents such as N,N-dimethylformamide or acetonitrile, water, or a mixture thereof, etc. Other purification methods include a method described in Jikken-Kagaku-Koza (edited by the Chemical Society of Japan, Maruzen), vol. 1, etc.

The compound of the formula (1) with one or more asymmetric center(s) of the present invention may be prepared by using a starting material with asymmetric centers or introducing asymmetric centers in any half way steps according to a conventional method. For example, enantiomers may be obtained by using optically active starting materials or carrying out optical resolution in an appropriate step of the preparation method. For example, the optical resolution may be carried out by a diastereomeric method wherein the compound of the formula (1) or an intermediate thereof is reacted with an optically active acid (e.g., monocarboxylic acid such as mandelic acid, N-benzyloxyalanine or lactic acid, dicarboxylic acid such as tartaric acid, o-diisopropylidene tartaric acid or malic acid, or sulfonic acid such as camphorsulfonic acid or bromocamphorsulfonic acid) to form a salt thereof in an inactive solvent (e.g., alcoholic solvent such as methanol, ethanol or 2-propanol, ether solvent such as diethyl ether, ester solvent such as ethyl acetate, hydrocarbon solvent such as toluene, or aprotic solvent such as acetonitrile, and a mixture thereof).
The optical resolution may be also carried out by reacting the compound of the formula (1) or an intermediate thereof having an acidic functional group such as carboxy with an optically active amine (e.g., organic amine such as α-phenethylamine, kinin, quinidine, cinchonidine, cinchonine, strychnine) to form a salt thereof.

A temperature for forming the salt is selected from the range of room temperature to a boiling point of the solvent. In order to improve an optical purity, it is desirable to raise the temperature up to around a boiling point of the solvent. The precipitated salt may be cooled in filtration to improve its yield as necessary. The usage of an optically active acid or amine is properly in the range of about 0.5 to about 2.0 equivalents, preferably around 1 equivalent, to the substrate. The crystal may be also, as necessary, recrystallized in an inactive solvent (e.g., alcoholic solvent such as methanol, ethanol, 2-propanol, ether solvent such as diethyl ether, ester solvent such as ethyl acetate, hydrocarbon solvent such as toluene, aprotic solvent such as acetonitrile, and a mixture thereof) to give an optically active salt in high purity. The optically resolved salt may be also, as necessary, treated with acid or base in a conventional manner to give in a free form.

The adenine compound, or a pharmaceutically acceptable salt thereof of the present invention activates toll-like receptor (TLR), specifically TLR7, and is useful as an immune-response modifier and a therapeutic or preventive agent for diseases such as diseases associated with abnormality of immune response (e.g., autoimmune diseases and allergic diseases), various infectious diseases wherein an immune response is desired to be activated or cancer. For example, the adenine compound or a pharmaceutically acceptable salt thereof of the present invention is useful as a therapeutic or preventive agent for diseases including the following (1) to (8).
(1) Respiratory affections, including intermittent or persistent asthma of every severity (e.g., bronchial asthma, allergic asthma, intrinsic asthma, extrinsic asthma, exercise-induced asthma, asthma induced by drug (e.g., NSAID such as aspirin and indometacin), dust-induced asthma, and airway hyper-responsiveness diseases caused by other factors); chronic obstructive pulmonary disease (COPD); bronchitis (e.g., infectious bronchitis, eosinophilic bronchitis); emphysema; bronchiectasis; cystic fibrosis; sarcoidosis; farmer's lung and related diseases thereof; hypersensitivity pneumonitis; lung fibrosis (e.g., cryptogenic fibrosing alveolitis, idiopathic interstitial pneumonitis, and complicating anti-neoplastic therapy chronic infectious diseases including tuberculosis bacterial, aspergillus or other fungal infectious diseases, etc.); complication by lung transplantation; vascular and thrombotic pulmonary disease and pulmonary hypertension; antitussive activity including treatment of chronic cough associated with inflammation or secretion of airway and iatrogenic cough; acute or chronic rhinitis including rhinitis medicamentosa or vasomotor rhinitis; perennial or seasonal allergic rhinitis including rhinitis nervosa (hay fever); nasal polyposis; acute virus infection including common cold disease and respiratory infectious diseases by syncytium virus, influenza, coronavirus (including SARS) and adenovirus.
(2) Skin diseases, including psoriasis, atopic dermatitis, contact dermatitis and other eczematous dermatitis, and delayed hypersensitivity reaction; phytodermatitis and photodermatitis; seborrheic dermatitis, dermatitis herpetiformis; lichen planus, lichen sclerosis, lichen sclerosus et atrophicus, pyoderma gangrenosum, skin sarcoidosis, discoid lupus erythematosus, pemphigus, pemphigoid, epidermolysis bullosa, urticaria, angioedema, vasculitides, toxic erythemas, cutaneous eosinophilias, alopecia areata, malepattern boldness, Sweet syndrome, Weber-Christian syndrome, erythema multiforme; infectious or noninfectious cellulitis; panniculitis; cutaneous lymphoma, nonmelanoma skin cancer or other dysplastic lesions; drug-induced disease including fixed drug eruption.

(3) Eye diseases, including blepharitis; conjunctivitis including perennial and vernal allergic conjunctivitis; iritis; anterior and posterior uveitis; choroiditis; retinal disease associated with autoimmune, denaturation or inflammation; ophthalmitis including sympathetic ophthalmia; sarcoidosis; viral, fungal or bacterial infectious diseases.
(4) Genitourinary diseases, including nephritis including interstitial and glomerulonephritis; nephrotic syndrome; cystitis including acute or chronic (interstitial) cystitis and Hunner's ulcer; acute or chronic urethritis, prostatitis, epididymitis, oophoritis and salpingitis; vulvovaginitis; Peyronie's disease; erectile dysfunction (male and female).
(5) Allograft rejections, including posttransfusion acute and chronic rejection after transplantation of kidney, heart, liver, lung, marrow, skin or cornea, etc.; or chronic graft-versus-host disease.
(6) Autoimmune diseases, including chronic rheumatoid arthritis, inflammatory bowel disease such as ulcerative colitis, systemic lupus erythematosus, multiple sclerosis, Hashimoto's thyroiditis, Grave's disease, Addison's disease, diabetes, idiopathic thrombocytopenic purpura, eosinophilic fasciitis, high IgE syndrome, or other autoimmune diseases and allergic diseases such as autoimmune disease syndrome including antiphospholipid antibody syndrome.
(7) Cancer diseases, including prostate cancer, breast cancer, lung cancer, uterus cancer, ovarian cancer, pancreas cancer, liver cancer, colon cancer, stomach cancer, skin cancer or cerebral tumor, and malignant bone marrow neoplasm (e.g., leukemia) and lymphoproliferative tumor such as Hodgkin's lymphoma or non-Hodgkin's lymphoma. It is useful for usual treatment of these cancer diseases and also for prevention or treatment of metastasis, tumor recurrence and paraneoplastic syndrome.

(8) Infectious diseases, including viral infectious diseases such as genital wart, common wart, plantar wart, hepatitis B, hepatitis C, herpes simplex viral disease, molluscum contagiosum, variola, acquired immune deficiency syndrome (HIV), and infectious diseases caused by human papillomavirus (HPV), cytomegalovirus (CMV), varicella-zoster virus (VZV), rhinovirus, adenovirus, coronavirus, influenza virus or parainfluenza virus; bacterial diseases such as tuberculosis, mycobacterium avium complex, Hansen's disease; other infectious diseases such as infectious diseases caused by various fungi, candida, chlamydia or aspergillus, cryptococcus meningitis, carinii pneumonia, cryptosporidiosis, histoplasmosis, toxoplasmosis, trypanosome infectious diseases, or leishmaniasis.
The adenine compound or a pharmaceutically acceptable salt thereof of the present invention is also useful as a vaccine adjuvant.

The adenine compound or a pharmaceutically acceptable salt thereof of the present invention has a TLR activating effect, more specifically a TLR7 activating effect. The adenine compound or a pharmaceutically acceptable salt thereof of the present invention also shows interferon-α- and interferon-y-inducing activity, and IL-4/IL-5 producing inhibition activity, and acts as an agent with helper T cell type 1 (Th1 cell)/helper T cell type 2 (Th2 cell) selective immunomodulating activity. In other words, it is preferably useful as a therapeutic or preventive agent for allergic diseases caused by Th2 cell such as asthma, COPD, allergic rhinitis, allergic conjunctivitis or atopic dermatitis due to its Th2 cell selective immunosuppressive action. On the other hand, owing to its immunostimulatory action, they are also useful as a therapeutic or preventive agent for various diseases, such as cancer, viral infectious diseases (e.g., hepatitis B, hepatitis C, acquired immune deficiency syndrome (HIV), human papillomavirus disease (HPV)), bacterial infectious diseases, skin diseases (e.g., psoriasis), etc.

The adenine compound or a pharmaceutically acceptable salt thereof of the present invention is useful for treatment of airway obstruction diseases/conditions such as asthma or COPD, or for reducing the risk of these diseases.

The "preventive agent" is adminstered to a patient, who has not been affected with certain disease or who has no problem in a health condition at the time of administration of the agent, in order to prevent the disease or prevent the symptoms of the disease from worsening. The "prevention" (or "preventive ") is expected to be suitable particularly for a person who has previous history of certain disease or be at increased risk for such disease. Generally, a person at risk for certain disease or deplopment of symptoms has a family history of such disease or can be identified by genetic diagnosis for such diseases.

The adenine compound or a pharmaceutically acceptable salt thereof of the present invention may be orally or parenterally administered without any limitation to the dosage forms. For example, an oral preparation may include capsules, powders, tablets, granules, subtle granules, syrups, liquids, suspensions, etc., and a parenteral preparation may include injections, drips, eye-drops, preparations for intrarectal administration, inhalations, air sprays (e.g., aerosols, dry powders, or liquid/suspensions for sprays, aerosols, or cartridge sprays for inhalators or insufflators, etc.), lotions, gels, ointments, creams, transdermal absorbents, transmucosal absorbents, nasal preprations, eardrops, tapes, transdermal patches, cataplasms, external powders, etc. These preparations may be prepared according to a conventional technique, and may contain conventional carriers, fillers, binders, lubricants, stabilizers, disintegrants, buffers, solubilizing agents, isotonic agents, surfactants, preservative agents, perfumes, and further optionally contains 2 or more kinds of additives for preparations.
The adenine compound or a pharmaceutically acceptable salt thereof of the present invention may be incorporated with a pharmaceutically acceptable carrier in a manner known to those skilled in the art to prepare a pharmaceutical composition suitable for each dosage form. For example, the adenine compound or a pharmaceutically acceptable salt thereof may be formed into a pharmaceutical composition comprising as an active ingredient 0.05 to 99% by weight, preferably 0.05 to 80% by weight, more preferably 0.1 to 70% by weight, more preferably 0.1 to 50% by weight of the compound.

Among the oral preparations, liquid preparations such as emulsions and syrups may be prepared by optionally using additives for preparations including water; sugars such as sucrose, sorbit, fructose; ethanol; glycols such as polyethyleneglycol, propyleneglycol, glycerol; oils such as sesame oil, olive oil, soy bean oil; preservative such as p-hydroxybenzoic esters; sweetener such as saccharin; thickener such as carboxymethylcellulose; flavors or colorants such as strawberry flavor, peppermint flavor, etc.
Solid preparations such as capsules, tablets, powders, granules, etc. may be prepared by optionally compounding the following carriers. Specifically, they may be prepared by using excipient such as lactose, glucose, sucrose, sorbitol, mannitol (mannite), cellulose derivatives; disintegrant such as starch (e.g., potato starch, cornstarch, amylopectin), sodium alginate; lubricant such as magnesium stearate, calcium stearate, polyethyleneglycol, wax, paraffin, talc; a binder such as polyvinyl alcohol, polyvinylpyrrolidone, hydroxypropylcellulose, gelatine; surfactant such as fatty ester; plasticizer such as glycerin, etc. In case of preparation of sugar coated tablets, it may be coated by concentrated carbohydrate solutions, optionally containing gum arabic, gelatine, talc, titanium dioxide, etc., on tablet cores prepared by using the above fillers. Alternatively, tablets may be film-coated by appropriate polymers dissolved in organic solvents which may be easily distilled away.
Soft gelatine capsules may be prepared by, for example, compounding the present compound with vegetable oil or polyethyleneglycol. Hard gelatine capsules may be prepared by using granules of the present compound which may be prepared by optionally compounding any one of the above carriers.

Among the parenteral preparations, liquid preparations in the form of injections, drips, eye-drops, eardrops, etc. may be preferably prepared as sterilized isotonic liquid preparations. For example, the injections may be prepared by using aqueous media comprising saline solution, glucose solution, or a mixture of saline solution and glucose solution. The preparations for intrarectal administration may be prepared by using carriers such as cacao butter, and usually prepared in the form of suppositories.
The ointments, creams and gels usually contain 0.01 to 10% by weight of the present compound, and to aqueous or oily base may be optionally added preferable thickener and/or gelatinizing agent and/or solvent. For example, the base includes water and/or oil such as liquid paraffin, vegetable oil such as peanut oil or castor oil, or solvent such as polyethyleneglycol. The thickener and gelatinizing agent include soft paraffin, aluminum stearate, cetostearyl alcohol, polyethyleneglycol, lanolin, bee wax, carboxypolymethylene and cellulose derivative and/or glyceryl monostearate and/or nonionic emulsifier.
The lotions usually contain 0.01 to 10% by weight of the present compound, and may be formulated by aqueous or oily base and may typically comprise emulsifier, stabilizer, dispersing agent, precipitation inhibitor or thickener.

The external powders usually contain 0.01 to 10% by weight of the present compound, and may be formed by preferable powder base such as talc, lactose or starch.
The drips may be formulated by aqueous or nonaqueous base and may contain dispersing agent, solubilizer, precipitation inhibitor or preservative.
The spray (e.g., spray, aerosol, dry powder preparation, etc.) may be optionally formulated as aqueous solution or suspension, or aerosol delivered from pressurized pack such as quantitative dose inhaler by using, for example, a preferable liquefied propellant. Dry powder preparation may be also used.
The aerosol appropriate for inhalation may be either suspension or solution, and typically contains the present compound and any appropriate propellants such as fluorocarbon or hydrogen-containing chlorofluorocarbon or a mixture thereof. Specifically, it contains hydrofluoroalkane, particularly 1,1,1,2-tetrafluoroethane, heptafluoroalkane (HFA) such as 1,1,1,2,3,3,3-heptafluoro-n-propane, or a mixture thereof. The aerosol may optionally contain additional preparation excipient well-known to those skilled in the art such as surfactant (e.g., oleic acid or lecithin) and cosolvent (e.g., ethanol), etc. Specifically, it may include inhaler known as "Turbuhaler™".

For example, capsule or cartridge of gelatine used in inhaler or ventilator may be formulated containing a powder mixture and preferable powder base such as lactose or starch for inhalation of the compound used in the present invention. Each capsule or cartridge usually contains 20 µg to 10 mg of the present compound. Alternatively, the compound used in the present invention may be provided without an excipient such as lactose.
In case of oral or nasal inhalation as pressurized HFA aerosol and dry powder preparation, etc., the adenine compound or a pharmaceutically acceptable salt thereof of the present invention may be finely ground into 10 µm or less to suspend in fatty acid with 8 to 20 carbon atoms or a salt thereof (e.g., oleic acid), bile acid salt, phospholipid, alkyl saccharide, fully-fluorinated or polyethoxylated surfactant, or other pharmaceutically acceptable dispersing agent.

The adenine compound of the invention can be administered as a preparation for local administration. Specifically, the preferable preparation includes ointment, lotion (solution or suspension), cream, gel, tape, transdermal patch, poultice, spray, aerosol, dry powder preparation, water/suspensions for cartridge sprays for inhaler or ventilator, eye-drops, eardrops, nose drops, transdermal patches, lung absorbents, airway absorbents or external powders, etc.
In the preparation for local administration in the present invention, the ratio of the active compound used in the present invention is generally 0.001 to 10% by weight, preferably 0.005 to 1% by weight depending on the forms of preparations. The ratio used in powders for inhalation or ventilation is in the range of 0.1 to 5% by weight.
Each quantitative dose or "whiff amount" in the aerosol preferably contains 20 µg to 2000 µg, preferably about 20 µg to 500 µg of the compound used in the present invention. The administration may be once or several times a day, for example 2, 3, 4 or 8 times a day, for example 1, 2 or 3 doses each.
The pharmacological activity may be measured in any assessments well-known to those skilled in the art, preferably in vitro assessments. Specific measuring method includes the one discribed in Examples in the present specification.

The present invention also encompasses a combination therapy for treating diseases described in the present specification wherein the compound of the formula (1) or a pharmaceutically acceptable salt thereof, or a pharmaceutical composition comprising the compound of the formula (1) or a pharmaceutically acceptable salt thereof is sequentially or simultaneously administered in combination with one or more of other following medicaments.
Particularly, the medicaments for treating inflammatory disease, COPD, asthma and allergic rhinitis include TNF-α inhibitor such as anti TNF monoclonal antibody (e.g., Remicade, CDP-870 and adalimumab) or TNF receptor immunoglobulin molecule (e.g., enbrel); locally- or systemically-administered nonselective cyclooxygenase: COX-1/COX-2 inhibitor (e.g., piroxicam, diclofenac, propionic acids such as naproxen, flurbiprofen, fenoprofen, ketoprofen and ibuprofen, fenamate such as mefenamic acid, indomethacin, sulindac, azapropazone, pyrazolones such as phenylbutazone, salicylate salt such as aspirin), COX-2 inhibitor (e.g., meloxicam, celecoxib, rofecoxib, valdecoxib, lumarocoxib, parecoxib and etoricoxib); glucocorticoid which is administered locally, orally, intramuscularly, intravenously or intraarticularly; methotrexate, leflunomide; hydroxychloroquine, d-penicillamine, auranofin, or other parenteral or oral gold preparation, etc.

The present invention also encompasses a combination of the present compounds with leukotriene biosynthetic inhibitor, 5-lipoxygenase (5-LO) inhibitor or 5-lipoxygenase activated protein (FLAP) antagonist, for example zileutone; ABT-761; fenleutone; tepoxalin; Abbott-79175; Abbott-85761; N-(5-substituted)-thiophen-2-alkylsulfonamide; 2,6-di-tert-butylphenolhydrazone; methoxytetrahydropyrane such as Zeneca ZD-2138; SB-210661; pyridinyl-substituted-2-cyanonaphthalene compound such as L-739010; 2-cyanoquinoline compound such as L-746530; MK-591, MK-886 and BAY-X-1005, etc.
The present invention also encompasses a combination therapy of the present compound with leukotriene (LT) B4, LTC4, LTD4, LTE4 receptor antagonist selected from the following group: phenothiazine compound such as L-651392; amidino compound such as CGS-25019c; benzoxalamine such as ontazolast; benzenecarboximidamide such as BIIL284/260; and compounds such as zafirlukast, ablukast, montelukast, pranlukast, Verlukast (MK-679), RG-12525, Ro-245913, iralukast (CGP45715A) and BAY-X-7195, etc.

The present invention also encompasses a combination therapy of the present compound with phosphodiesterase (PDE) inhibitor such as methyl xanthanin including theophylline and aminophylline; selective PDE isoenzyme including PDE4 inhibitor, isoform PDE4D inhibitor or PDE5 inhibitor.

The present invention also encompasses a combination therapy of the present compound which is orally or parenterally administered with, for example, histamine H1 receptor antagonist such as cetirizine, loratadine, desloratadine, fexofenadine, acrivastin, terfenadine, astemizole, azelastine, levocabastine, chlorpheniramine, promethazine, cyclizine and mizolastine, etc.

The present invention also encompasses a combination therapy of the present compound with histamine type 4 receptor antagonists.
The present invention also encompasses a combination therapy of the present compound with α1/α2 adrenaline receptor agonist and vasoconstrictive sympathetic stimulant such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride, and ethyl norepinephrine hydrochloride.

The present invention also encompasses a combination therapy of the present compound with anticholinergic agent including muscarinic receptor (M1, M2 and M3) antagonist such as atropine, biotin, glycopyrrolate, ipratropium bromide; tiotropium bromide; oxytropium bromide; pirenzepine; and telenzepine.
The present invention also encompasses a combination therapy of the present compound with β-adrenaline receptor agonist including β receptor subtypes 1 to 4 such as isoprenaline, salbutamol, formoterol, salmeterol, terbutaline, orciprenaline, bitolterol mesylate and pirbuterol.
The present invention also encompasses a combination therapy of the present compound with chromone such as sodium cromoglycate and nedocromil sodium.

The present invention also encompasses a combination therapy of the present compound with insulin-like growth factor type 1 (IGF-1) mimic.
The present invention also encompasses a combination therapy of the present compound with inhaled glucocorticoid such as flunisolide, triamcinolone acetonide, beclomethasone dipropionate, budesonide, fluticasone propionate, ciclesonide and mometasone furoate.

The present invention also encompasses a combination therapy of the present compound with matrix metalloprotease inhibitor, specifically stromelysin, collagenase, gelatinase, aggrecanase, particularly collagenase-1 (MMP-1), collagenase-2 (MMP-8), collagenase-3 (MMP-13), stromelysin-1 (MMP-3), stromelysin-2 (MMP-10) and stromelysin-3 (MMP-11), MMP-9 and MMP-12 inhibitor.

The present invention also encompasses a combination therapy of the present compound with chemokine receptor regulators of antagonists of CCR1, CCR2, CCR2A, CCR2B, CCR3, CCR4, CCR5, CCR6, CCR7, CCR8, CCR9, CCR10 and CCR11 (CC family); CXCR1, CXCR2, CXCR3, CXCR4 and CXCR5 (C-X-C family); C-X3-C family such as CX3CR 1.

The present invention also encompasses a combination therapy of the present compound with cytokine function regulator including cytokine or medicaments acting on cytokine signaling pathway, for example α-, β- and γ-interferon, interleukin (IL) including IL1 to 15, and interleukin antagonist or inhibitor.

The present invention also encompasses a combination therapy of the present compound with antibodies and antagonists regulating Ig functions such as immunoglobulin (Ig), immunoglobulin preparations, anti IgE antibody (omalizumab).

The present invention also encompasses a combination therapy of the present compound with systemically- or locally-administered antiinflammatory drugs such as thalidomide and derivatives thereof, retinoid, dithranol and calcipotriol.

The present invention also encompasses a combination therapy of the present compound with antibacterial agents such as penicillin derivative, tetracycline, macrolide, β-lactam, fluoroquinolone, metronidazole and inhaled aminoglycoside; and antiviral agents including acyclovir, famciclovir, balacyclovir, ganciclovir, cidofovir, amantadine, rimantadine, ribavirin; zanamivir, oseltamivir; enzyme inhibitor such as indinavir, nelfinavir, ritonavir and saquinavir; nucleoside reverse transcriptase inhibitor such as didanosine, lamivudine, stavudine, zalcitabine, zidovudine; nonnucleoside reverse transcriptase inhibitor such as nevirapine and efavirenz.

The present invention also encompasses a combination therapy of the present compound with medicaments known as therapeutic agents for cancer. Preferable agents include the following (i) to (ix).
(i) Antiproliferative agents/antitumor agents and a combination thereof used as a therapeutic agent for tumors, for example alkylating agents (e.g., cisplatin, carboplatin, cyclophosphamide, nitrogen mustard, melphalan, chlorambucil, busulfan, and nitrosourea); antimetabolite (e.g., fluoropyrimidine such as 5-fluorouracil and tegafur, antifolate such as raltitrexed, methotrexate, cytosine arabinoside, hydroxyurea, gemcitabine and paclitaxel); antineoplastic antibiotics (e.g., anthracycline such as adriamycin, bleomycin, doxorubicin, daunomycin, epirubicin, idarubicin, mitomycin-C, dactinomycin, and mithramycin); antimitotic agents (e.g., vincaalkaloid such as vincristine, vinblastine, vindesine and vinorelbine, taxoid such as taxol and taxotere); and topoisomerase inhibitors (e.g., epipodophyllotoxine such as etoposide, teniposide, amsacrine, topotecan and camptothecin).

(ii) Cytostatic agents including antiestrogens (e.g., tamoxifen, toremifene, raloxifene, droloxifene and iodoxifene, etc.), estrogen receptor down regulators (e.g., fulvestrant), antiandrogenic agents (e.g., bicalutamide, flutamide, nilutamide, and cyproterone acetate), LHRH antagonists or LHRH agonists (e.g., goserelin, leuprorelin and buserelin), progestogen (e.g., megestrol acetate), aromatase inhibitors (e.g., anastrozole, letrozole, vorazole and exemestane) and 5α-reductase inhibitors (e.g., finasteride).
(iii) Inhibiting agents of invation of cancer cells (e.g., c-Src kinase family inhibitors such as 4-(6-chloro-2, 3-methylenedioxyanilino)-7-[2-(4-methyl piperazin-1-yl)ethoxy]-5-tetrahydropyran-4-yloxy quinazoline (AZD0530; WO01/94341) and N-(2- chloro - 6-methylphenyl)-2-{6-[4-(2-hydroxyethyl)piperazin-1-yl]-2-methylpyrimidine-4-ylamino}thiazol-5-carboxamido (dasatinib, BMS-354825; J. Med. Chem., 2004, 47, 6658-6661), metalloprotease inhibitors and inhibitors of urokinase plasminogen activating receptor functions such as marimastat, or heparanase antibody).
(iv) Growth factor function inhibitors, such as growth factor antibody and growth factor receptor antibody (e.g., anti-erbB2 antibody trastuzumab (Herceptin™) and anti-erbB 1 antibody cetuximab [Erbitux, C225], and growth factor antibody and growth factor receptor antibody as described in Sternet et. al., Critical reviews in oncology/haematology, 2005, 54, 11-29); tyrosine kinase inhibitors such as epidermal growth factor inhibitors (e.g., EGFR family tyrosine kinase inhibitors such as N-(3-chloro-4-fluorophenyl)-7-methoxy-6-(3-morpholinopropoxy)quinazoline-4-amine (Gefitinib, AZD 1839), N-(3-ethynylphenyl)-6, 7-bis(2-methoxyethoxy)quinazoline-4-amine (erlotinib, OSI-774) and 6-acrylamide-N-(3-chloro-4-fluorophenyl)-7-(3-morpholinopropoxy)quinazoline-4-amine (CI1033)); erbB2 tyrosine kinase inhibitors such as lapatinib, hepatocellular growth factor family inhibitors, platelet-derived growth factor family inhibitors such as imatinib, inhibitors for serine/threonine kinase activity (for example, Ras/Raf signaling inhibitors such as farnesyltransferase inhibitors, e.g., sorafenib (BAY4 3- 9006)), MEK and/or AKT kinase signaling inhibitors, c-kit inhibitors, abl kinase inhibitors, IGF(insulin-like growth factor)receptor kinase inhibitors; and aurora kinase inhibitors (e. g., AZD1152, PH739358, VX-680, MLN8054, R763, MP235, MP529, VX-528 and AX39459) and CDK2 and/or CDK4 inhibitors cyclin dependent kinase inhibitors,

(v) Antiangiogenic agents, for example inhibiting agents of activity of vascular endothelial cell growth factor (e.g., anti-vascular endothelial cell growth factor antibody bevacizumab (Avastin™), and VEGF receptor kinase inhibitors such as 4-(4-bromo-2-fluoroanilino-6-methoxy-7-(1-methylpiperidine-4-ylmethoxy)quinazoline (ZD6474; Example 2 of WO01/32651), 4-(4-fluoro-2-methylindole-5-yloxy)-6-methoxy-7-(3-pyrrolidin-1-ylpropoxy)quinazoline (AZD2171; Example 240 of WO00/47212), vatalanib (PTK787; WO98/35985) and SU11248 (sunitinib; WO01/60814), compounds disclosed in international publications: WO97/22596, WO97/30035, WO97/32856 and WO98/13354); and compounds acting in other mechanisms (e.g., linomid, integrin αvβ3 function inhibitors or angiostatin).
(vi) Vascular damaging agents such as combretastatin A4 and compounds disclosed in international publications: WO99/02166, WO00/40529, WO00/41669, WO01/92224, WO02/04434 and WO02/08213.
(vii) Antisense therapeutics, for example antisense, anti-ras antisense to the above targets such as ISIS2503.
(viii) Gene therapy, for example abnormal gene exchanging approach such as abnormal p53 and abnormal BRCA1 or BRCA2, GDEPT (Gene-directed enzyme pro-drug therapy) approach using cytosine deaminase, thymidine kinase or bacterial nitroreductase enzyme, approach enhancing patients' tolerance for chemical therapy or radiation therapy such as multidrug resistance gene therapy.
(ix) Immunotherapy approach, for example approach for enhancing immunity to cancer cells of patients by exposuring cytokine such as interleukin 2, interleukin 4 or Granulocyte-Macrophage Colony Stimulating Factor (GM-CSF) ex-vivo or in-vivo, T cell anergy reducing approach, approach transplanting immune cells such as cytokine exposuring dendritic cells, approach using cytokine exposuring tumor cell line, and approach using anti-idiotypic antibody, etc.

### [Example]

The present invention will be illustrated in more detail by the following Examples, but the present invention should not be construed to be limited thereto.

### Example 1: 6-Amino-2-butoxy-9-(4-morpholin-4-ylmethylbenzyl)-7,9-dihydropurin-8-one

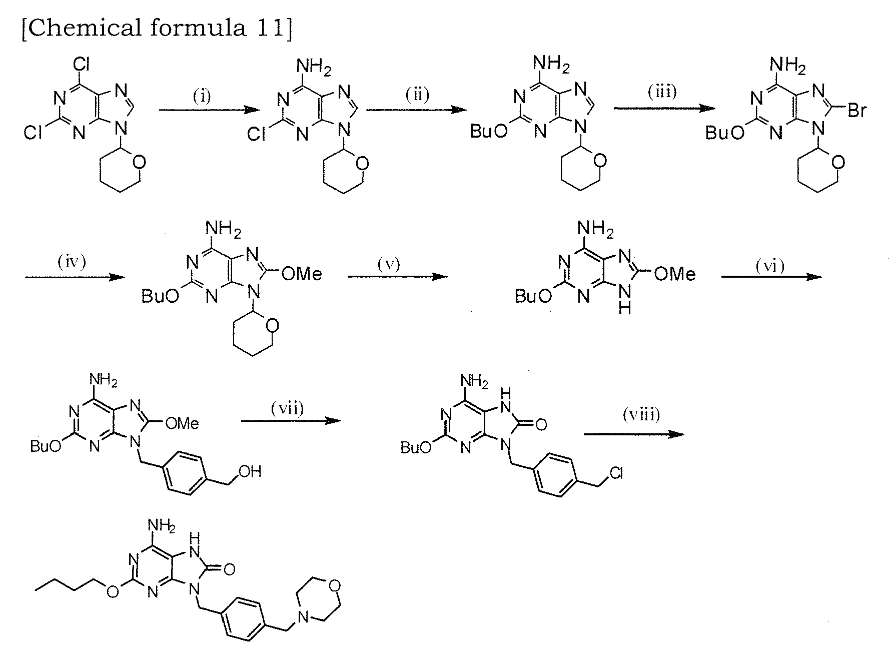

### Step (i): 2-Chloro-9-(tetrahydro-2H-pyran-2-yl)-9H-purin-6-amine

2,6-Dichloro-9-(tetrahydro-2H-pyran-2-yl)-9H-purine (55 g) was dissolved in 7N ammonia-methanol solution, and the mixture was heated at 100°C in the sealed flask for 6 hours. The reaction mixture was cooled to room temperature and allowed to stand overnight. The mixture was filtered to give the title compound. Yield: 40g, 80 % ¹H NMR (CDCl₃) δ 8.02 (1H, s), 5.94 (2H, bs), 5.71 (1H, dd), 4.15-4.22 (1H, m), 3.75-3.82 (1H, m), 1.27-2.12 (6H, m).

### Step (ii): 2-Butoxy-9-(tetrahydro-2H-pyran-2-yl)-9H-purin-6-amine

The compound obtained in Step (i) (40 g) was dissolved in a 19 % solution of sodium butoxide in butanol, and the mixture was heated under reflux for 6 hours. The obtained suspension was cooled to room temperature, diluted with water, and extracted with diethyl ether. The organic layer was washed with water, dried, and concentrated under reduced pressure. The residue was dissolved in a mixture of hexane and diethyl ether for crystallization, and the obtained crystals were collected by filtration to give the title compound. Yield: 19g, 64 %.
¹H NMR (CDCl₃) δ 7.87 (1H, s), 5.56-5.68 (3H, m), 4.31-4.35 (2H, t), 4.14-4.17 (1H, m), 3.76-3.80 (1H, m), 1.49-2.08 (10H, m), 0.98 (3H, t). Step (iii): 8-Bromo-2-butoxy-9-(tetrahydro-2H-pyran-2-yl)-9H-purin-6-amine

The compound obtained in Step (ii) (30 g) was dissolved in dichloromethane (200 ml), and thereto was added N-bromosuccinimide (27 g) in portions under stirring at room temperature, and then, the mixture was stirred at room temperature overnight. To the mixture was added a 20 % aqueous sodium thiosulfate, and the separated aqueous layer was extracted with dichloromethane. The organic layer was washed with a saturated aqueous sodium hydrogen carbonate solution and a saturated saline solution, and concentrated under reduced pressure. The residue was dissolved in ethyl acetate, washed with water and saturated saline solution, and dried. The obtained solution was filtered through silica gel, and concentrated under reduced pressure. The residue was dissolved in a mixture of hexane and diethyl ether for crystallization, and the obtained crystals were collected by filtration to give the crystals (26 g). The filtrate was concentrated, and the residue was purified by column chromatography (ethyl acetate: hexane) to give the crystals (2.5g). These crystals were combined to give the title compound as yellow solid. Yield: 28.5 g, 75 %, mp 148-150°C
¹H NMR (CDCl₃) δ 5.59-5.64 (3H, m), 4.32 (2H, m), 4.17 (1H, m), 3.74 (1H, m), 3.08 (1H, m), 2.13 (1H, d), 1.48-1.83 (8H, m), 0.98 (3H, t). Step (iv): 2-Butoxy-8-methoxy-9-(tetrahydro-2H-pyran-2-yl)-9H-purin-6-amine

Under nitrogen atmosphere, methanol (400 ml) was added sodium (3.7 g). To the obtained solution was added the compound obtained in Step (iii) (28.5 g), and the mixture was heated at 65°C for 9 hours. The reaction solution was concentrated under reduced pressure, and thereto was added water (500 ml). The separated aqueous layer was extracted with ethyl acetate, washed with a saturated saline solution, and concentrated. The residue was crystallized from diethyl ether to give the title compound. Yield: 14.2 g, 98 %.
¹H NMR (CDCl₃) δ 5.51(1H, dd), 5.28 (2H, bs), 4.29 (2H, t), 4.11-4.14 (4H, m), 3.70 (1H, m), 2.76-2.80 (1H, m), 2.05 (1H, d), 1.47-1.81 (8H, m), 0.97 (3H, t).

### Step (v): 2-Butoxy-8-methoxy-9H-purin-6-amine trifluoroacetic acid salt

The compound obtained in Step (iv) (24 g) was dissolved in methanol (300 ml), and thereto was added trifluoroacetic acid (30 ml). The mixture was stirred at room temperature for 72 hours, and concentrated under reduced pressure, and crystallized from a mixture of methanol and ethyl acetate to give the title compound as white solid. Yield: 21 g, 80 %.
¹H NMR (CD₃OD) δ 4.48 (2H, t), 4.15 (3H, s), 1.80 (2H, quintet), 1.50 (2H, sextet), 0.99 (3H, t).

### Step (vi): [4-(6-Amino-2-Butoxy-8-methoxypurin-9-ylmethyl)phenyl]-methanol

To a solution of 2-butoxy-8-methoxyadenine trifluoroacetic acid salt (10 g, 42.1 mmol) in DMF (90 ml) were added potassium carbonate (17.5 g, 126.4 mmol), (4-hydroxymethyl)benzyl chloride (7.3 g, 46.4 mmol), and the mixture was stirred at room temperature for 18 hours. The carbonate in the reaction system was removed by filtration, and the filtrate was concentrated. Water was added to the residue, and the mixture was extracted with 5 % methanol in chloroform (800 ml). The organic layer was washed successively with water and a saturated saline solution, and dried over sodium sulfate. To the residue were added chloroform (125 ml), methanol (25 ml) and diethyl ether (125 ml), and the insoluble solid was removed by filtration. The filtrate was concentrated under reduced pressure, and diethyl ether (150 ml) was added to the residue. The precipitated white solid was collected by filtration and dried to give the sub-title compound as white solid (7.2 g, 20.1 mmol). Yield: 71 %.
¹H,NMR (DMSO-d₆) δ 7.26 (2H, d, J = 8.2 Hz), 7.19 (2H, d, J = 8.2 Hz), 6.47 (2H, brs), 5.15 (1H, t, J = 5.6 Hz), 5.01 (2H, s), 4.44 (2H, d, J = 5.6 Hz), 4.17 (2H, t, J = 6.6 Hz), 4.03 (3H, s), 1.68-1.59 (2H, m), 1.44-1.34 (2H, m), 0.91 (3H, t, J = 7.4 Hz).

### Step (vii): 6-Amino-9-(4-chloromethylbenzyl)-2-butoxy-7,9-dihydropurin-8-one hydrochloride

To the compound obtained in Step (vi) (7.1 g, 19.6 mmol) was added dichloromethane (140 ml), and to the resulting suspension was added thionyl chloride (4.3 ml), and the mixture was stirred at 5° for 2 hours. To the mixture was added toluene (30 ml), and the solvent was evaporated. Toluene (100 ml) was added to the residue, and the solvent was evaporated, and dried under reduced pressure to give the sub-title compound as pale yellow solid (7.2 g, 19.6 mmol). Yield: 99 %.
¹H NMR (DMSO-d₆) δ 7.39 (2H, d, J = 8.2 Hz), 7.30 (2H, d, J = 8.2 Hz), 4.88 (2H, s), 4.73 (2H, s), 4.21 (2H, t, J = 6.6 Hz), 1.68-1.59 (2H, m), 1.43-1.32 (2H, m), 0.90 (3H, t, J = 7.4 Hz).

### Step (viii):

The compound obtained in Step (vii) (180 mg, 0.5 mmol), morpholine (0.3 mL) and diisopropylethylamine (1 mL) were added to DMF (5 mL), and the mixture was stirred at 80°C for 2 hours, and the solvent was evaporated. Water was added to the residue, and the precipitated solid was collected by filtration, purified by silica gel column chromatography (CHCl₃/MeOH=30/1) to give the title compound (163 mg, 79 %).
¹H NMR (DMSO-d₆) δ 9.94 (1H, s), 7.25 (4H, s), 6.45 (2H, s), 4.83 (2H, s), 4.14 (2H, t, J = 6.4 Hz), 3.54 (4H, t, J = 4.2 Hz), 3.40 (2H, s), 2.31 (4H, m), 1.62 (2H, m), 1.37 (2H, m), 0.91 (3H, t, J = 7.2 Hz).

In a similar manner to Example 1, the compounds of the following Examples 2 to 34 were obtained.

### Example 2: 6-Amino-2-butoxy-9-(4-piperidin-1-ylmethylbenzyl)-7,9-dihydropurin-8-one (139 mg, 82 %)

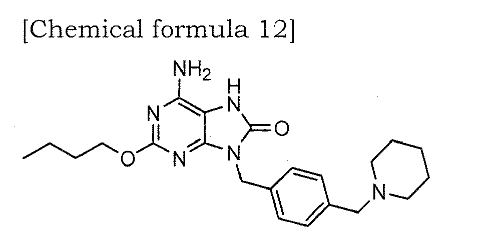

¹H NMR (DMSO-d₆) δ 9.96 (1H, s), 7.23 (4H, m), 6.43 (2H, s), 4.83 (2H, s), 4.15 (2H, t, J = 6.4 Hz), 3.36 (2H, s), 2.27 (4H, m), 1.62 (2H, m), 1.35-1.49 (8H, m), 0.91 (3H, t, J = 6.4 Hz).

### Example 3: 6-Amino-2-butoxy-9-[4-(4-methylpiperazin-1-ylmethyl)-benzyl]-7,9-dihydropurin-8-one (145 mg, 69 %)

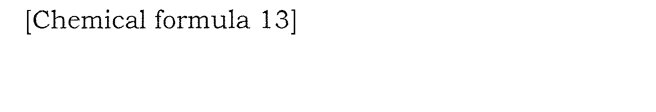

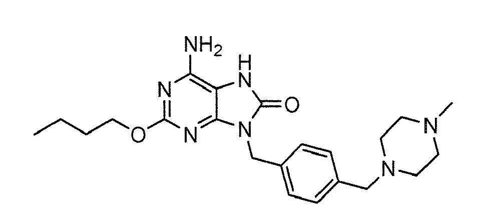

¹H NMR (DMSO-d₆) δ 9.96 (1H, s), 7.23 (4H, s), 6.46 (2H, s), 4.83 (2H, s), 4.14 (2H, t, J = 6.4 Hz), 3.38 (2H, s), 2.30 (8H, m), 2.12 (3H, s), 1.62 (2H, m), 1.36 (2H, m), 0.90 (3H, t, J = 7.2 Hz).

### Example 4: 6-Amino-2-butoxy-9-[4-(4-dimethylaminopiperidin-1-ylmethyl)benzyl]-7,9-dihydropurin-8-one (167 mg, 74 %)

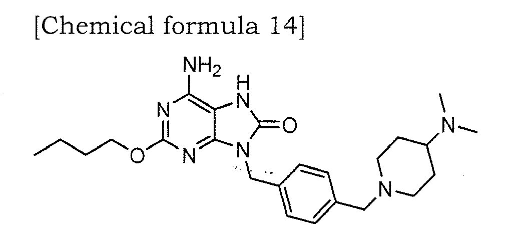

¹H NMR (DMSO-d₆) δ 9.96 (1H, s), 7.23 (4H, m), 6.46 (2H, s), 4.83 (2H, s), 4.14 (2H, t, J = 6.6 Hz), 3.17 (1H, d, J = 2.0 Hz), 2.75 (2H, d, J = 12.4 Hz), 2.12 (6H, s), 1.92 (1H, m), 1.85 (2H, m), 1.60-1.67 (4H, m), 1.30-1.40 (4H, m), 0.90 (3H, t, J = 7.4 Hz).

### Example 5: 6-Amino-2-butoxy-9-[4-(3-dimethylaminopyrrolidin-1-yl-methyl)benzyl]-7,9-dihydropurin-8-one (115 mg, 63 %)

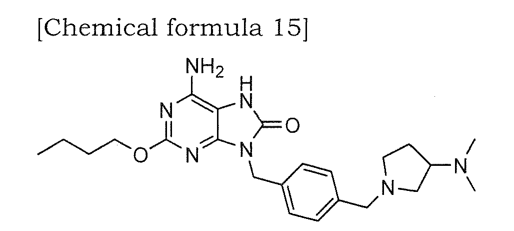

¹H NMR (DMSO-d₆) δ 9.95 (1H, s), 7.25 (4H, m), 6.45 (2H, s), 4.83 (2H, s), 4.14 (2H, t, J = 6.6 Hz), 3.49 (2H, d, J = 13.2 Hz), 3.39 (2H, d, J = 13.2 Hz), 2.62 (2H, m), 2.37 (1H, m), 2.20 (1H, m), 2.05 (6H, s), 1.80 (1H, m), 1.62 (3H, m), 1.37 (2H, m), 0.90 (3H, t, J = 6.8 Hz).

### Example 6: 6-Amino-2-butoxy-9-(4-{[methyl-(1-methylpyrrolidin-3-yl)amino]methyl}benzyl)-7,9-dihydropurin-8-one (22 mg, 12 %)

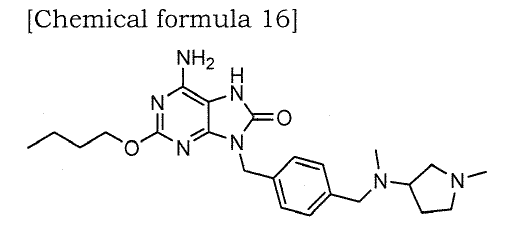

¹H NMR (DMSO-d₆) δ 9.91 (1H, brs), 7.25 (4H, m), 6.45 (2H, s), 4.83 (2H, s), 4.14 (2H, t, J = 7.0 Hz), 3.41 (2H, d, J = 13.6 Hz), 3.33 (2H, d, J = 13.6 Hz), 3.05 (1H, m), 2.55 (1H, m), 2.36-2.44 (3H, m), 2.20 (3H, s), 1.97 (3H, s), 1.86 (1H, m), 1.60-1.64 (3H, m), 1.38 (2H, m), 0.90 (3H, t, J = 7.4 Hz).

### Example 7: N-{1-[4-(6-Amino-2-butoxy-8-oxo-7,8-dihydropurin-9-ylmethyl)benzyl]piperidin-4-yl}acetamide (191 mg, 82 %)

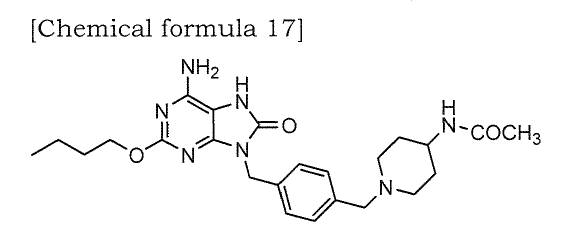

¹H NMR (DMSO-d₆) δ 9.95 (1H, s), 7.23 (4H, m), 6.45 (2H, s), 4.83 (2H, s), 4.14 (2H, t, J = 6.6 Hz), 3.47 (1H, m), 3.38 (3H, s), 2.70 (2H, m), 1.94 (2H, t, J = 10.8 Hz), 1.77 (3H, s), 1.60-1.67 (4H, m), 1.31-1.40 (4H, m), 0.90 (3H, t, J = 7.4 Hz).

### Example 8: 1-[4-(6-Amino-2-butoxy-8-oxo-7,8-dihydropurin-9-yl-methyl)benzyl]piperidine-4-carboxylic acid amide (135 mg, 60 %)

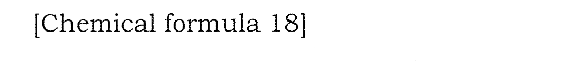

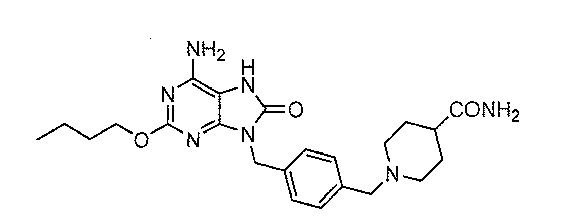

¹H NMR (DMSO-d₆) δ 9.95 (1H, s), 7.23 (4H, m), 6.70 (1H, s), 6.45 (2H, s), 4.83 (2H, s), 4.14 (2H, t, J = 6.6 Hz), 3.38 (2H, s), 3.38 (3H, s), 2.75 (2H, d, J = 11.2 Hz), 2.05 (1H, m), 1.85 (1H, q, J = 9.6 Hz), 1.40-1.64 (6H, m), 1.38 (2H, m), 0.91 (3H, t, J = 7.2 Hz).

### Example 9: 6-Amino-2-butoxy-9-[3-(4-methylpiperazin-1-ylmethyl)-benzyl]-7,9-dihydropurin-8-one) (171 mg)

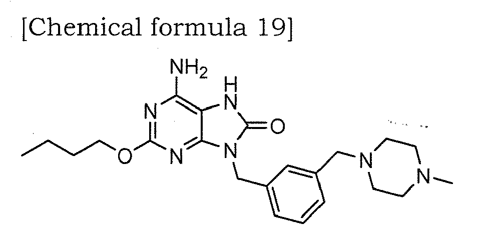

¹H NMR (DMSO-d₆) δ 9.95 (1H, s), 7.26 (2H, m), 7.16 (2H, m), 6.45 (2H, s), 4.84 (2H, s), 4.14 (2H, q, J = 6.6 Hz), 3.40 (2H, s), 2.29 (8H, m), 2.13 (3H, s), 1.63 (2H, m), 1.37 (2H, m), 0.91 (3H, t, J = 7.2 Hz).

### Example 10: 6-Amino-2-(2-methoxyethoxy)-9-(4-piperidin-1-ylmethylbenzyl)-7,9-dihydropurin-8-one) (210 mg, 88 %)

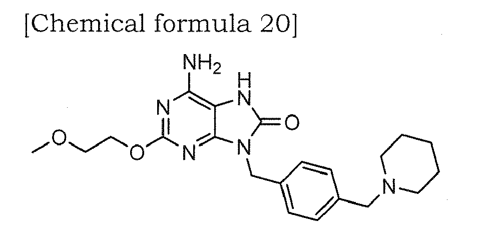

¹H NMR (DMSO-d₆) δ 9.98 (1H, s), 7.23 (4H, m), 6.48 (2H, s), 4.83 (2H, s), 4.26 (2H, t, J = 4.8 Hz), 3.59 (2H, t, J = 4.8 Hz), 3.35 (2H, s), 3.27 (3H, s), 2.26 (4H, m), 1.46 (4H, m), 1.36 (2H, m).

### Example 11: 6-Amino-2-(2-methoxyethoxy)-9-[4-(4-methylpiperazin-1-ylmethyl)benzyl]-7,9-dihydropurin-8-one (170 mg, 68 %)

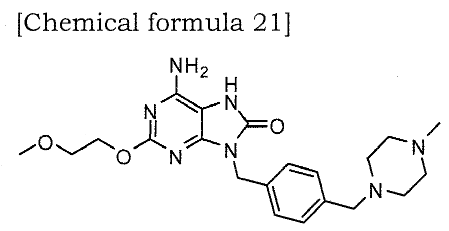

¹H NMR (DMSO-d₆) δ 9.98 (1H, s), 7.24 (4H, m), 6.49 (2H, s), 4.83 (2H, s), 4.26 (2H, t, J = 4.8 Hz), 3.58 (2H, t, J = 4.8 Hz), 3.38 (2H, s), 3.27 (3H, s), 2.29 (8H, m), 2.12 (3H, s).

### Example 12: 6-Amino-2-(2-methoxyethoxy)-9-[4-(4-phenylpiperazin-1-ylmethyl)benzyl]-7,9-dihydropurin-8-one (86 mg, 45 %)

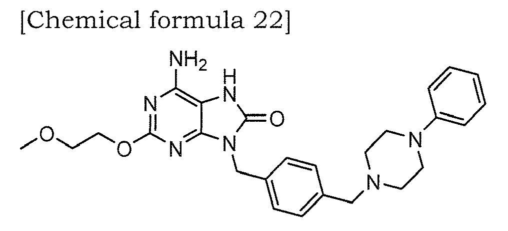

¹H NMR (DMSO-d₆) δ 9.97 (1H, s), 7.28 (4H, m), 7.19 (2H, m), 6.90 (2H, m), 6.76 (1H, t, J = 7.2 Hz), 6.49 (2H, s), 4.85 (2H, s), 4.27 (2H, t, J = 5.2 Hz), 3.59 (2H, t, J = 5.2 Hz), 3.47 (2H, s), 3.27 (3H, s), 3.10 (4H, m), 2.47 (4H, m).

### Example 13: 6-Amino-2-(2-methoxyethoxy)-9-[4-(4-phenoxypiperidin-1-ylmethyl)benzyl]-7,9-dihydropurin-8-one (170 mg, 45 %)

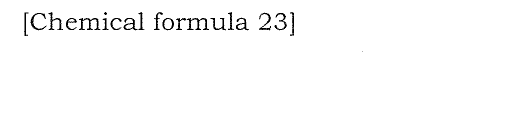

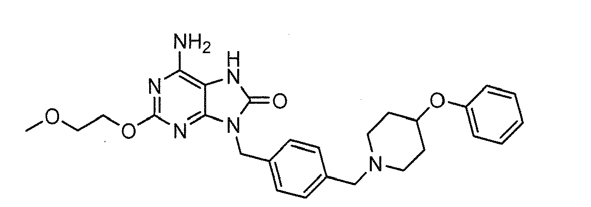

¹H NMR (DMSO-d₆) δ 9.97 (1H, s), 7.27 (6H, m), 6.91 (3H, m), 6.48 (2H, s), 4.84 (2H, s), 4.36 (1H, m), 4.26 (2H, t, J = 4.8 Hz), 3.59 (2H, t, J = 4.8 Hz), 3.44 (2H, s), 3.27 (3H, s), 2.64 (2H, m), 2.20 (2H, t, J = 9.2 Hz), 1.90 (2H, m), 1.60 (2H, m).

### Example 14: 6-Amino-9-(4-dimethylaminomethylbenzyl)-2-(2-methoxy-ethoxy)-7,9-dihydropurin-8-one (130 mg, 63 %)

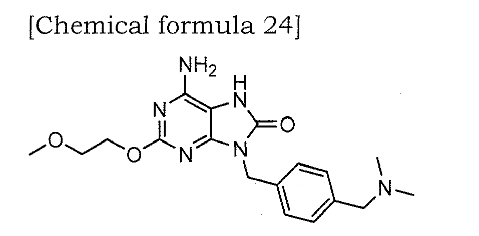

¹H NMR (DMSO-d₆) δ 9.97 (1H, s), 7.24 (4H, m), 6.48 (2H, s), 4.83 (2H, s), 4.26 (2H, t, J = 4.8 Hz), 3.58 (2H, t, J = 4.8 Hz), 3.33 (2H, s), 3.27 (3H, s), 2.29 (8H, m), 2.11 (6H, s).

### Example 15: 6-Amino-9-{4-[(diisopropylamino)methyl]benzyl}-2-(2-methoxyethoxy)-7,9-dihydropurin-8-one (51 mg, 29 %)

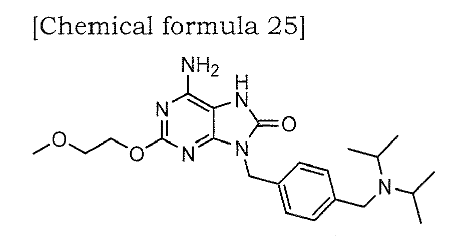

¹H NMR (DMSO-d₆) δ 9.95 (1H, s), 7.26 (4H, m), 6.47 (2H, s), 4.83 (2H, s), 4.27 (2H, t, J = 4.8 Hz), 3.59 (2H, t, J = 4.8 Hz), 3.56 (2H, s), 3.27 (3H, s), 2.93 (2H, sept, J = 6.8 Hz), 0.97 (6H, s), 0.95 (6H, s).

### Example 16: 6-Amino-2-(2-methoxyethoxy)-9-(4-{[(2-methoxyethyl)methylamino]methyl}benzyl)-7,9-dihydropurin-8-one (62 mg, 25 %)

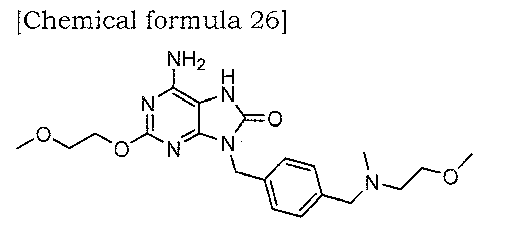

¹H NMR (DMSO-d₆) δ 9.97 (1H, s), 7.24 (4H, m), 6.48 (2H, s), 4.83 (2H, s), 4.26 (2H, t, J = 4.8 Hz), 3.58 (2H, t, J = 4.8 Hz), 3.42 (4H, m), 3.27 (3H, s), 3.21 (3H, s), 2.48 (2H, m), 2.10 (3H, s).

### Example 17: 6-Amino-9-{4-[(cyclohexylmethylamino)methyl]benzyl}-2-(2-methoxyethoxy)-7,9-dihydropurin-8-one (164 mg, 75%)

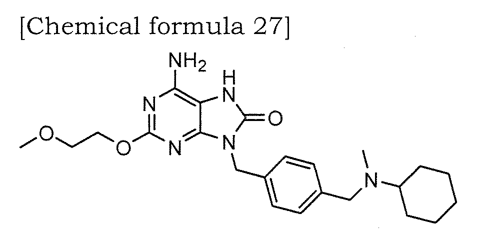

¹H NMR (DMSO-d₆) δ 9.95 (1H, s), 7.23 (4H, m), 6.45 (2H, s), 4.83 (2H, s), 4.27 (2H, t, J = 4.8 Hz), 3.59 (2H, t, J = 4.8 Hz), 3.49 (2H, s), 3.27 (3H, s), 2.36 (1H, m), 2.06 (3H, s), 1.75 (4H, m), 1.56 (1H, m), 1.05-1.25 (5H, m).

### Example 18: 6-Amino-9-(4-cyclohexylaminomethylbenzyl)-2-(2-methoxyethoxy)-7,9-dihydropurin-8-one (41 mg, 19 %)

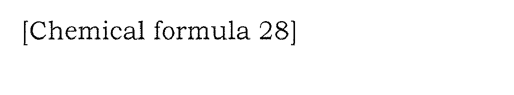

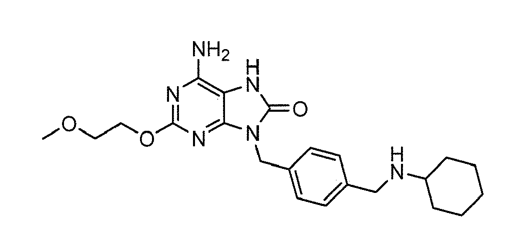

¹H NMR (DMSO-d₆) δ 9.95 (1H, brs), 7.26 (4H, m), 6.45 (2H, s), 4.82 (2H, s), 4.27 (2H, t, J = 4.8 Hz), 3.67 (2H, s), 3.59 (2H, t, J = 4.8 Hz), 3.27 (3H, s), 2.32 (1H, m), 1.80 (2H, m), 1.64 (2H, m), 1.53 (1H, m), 1.00-1.18 (5H, m).

### Example 19: 6-Amino-2-(2-methoxyethoxy)-9-{4-[(methylphenylamino)-methyl]benzyl}-7,9-dihydropurin-8-one (185 mg, 100 %)

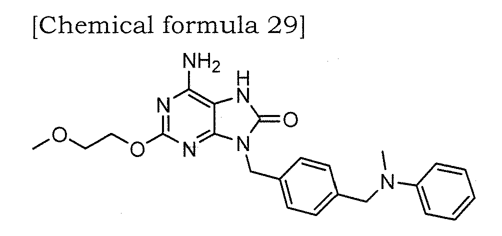

¹H NMR (DMSO-d₆) δ 9.98 (1H, s), 7.29-7.33 (2H, m), 7.10-7.16 (4H, m), 6.68 (2H, d, J = 8.0 Hz), 6.59 (1H, 7.2 Hz), 6.47 (2H, s), 4.86 (2H, s), 4.52 (2H, s), 4.25 (2H, t, J = 4.8 Hz), 3.58 (2H, t, J = 4.8 Hz), 3.27 (3H, s), 2.97 (3H, s).

### Example 20: 6-Amino-9-{4-[(benzylmethylamino)methyl]benzyl}-2-(2-methoxyethoxy)-7,9-dihydropurin-8-one (210 mg, 85 %)

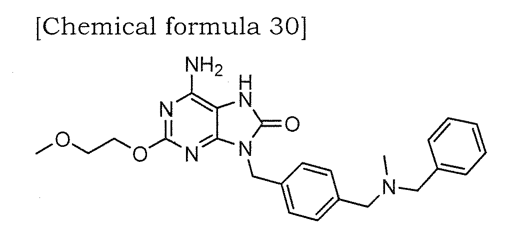

¹H NMR (DMSO-d₆) δ 10.00 (1H, s), 7.23-7.34 (10H, m), 6.49 (2H, s), 4.84 (2H, s), 4.26 (2H, t, J = 4.8 Hz), 3.58 (2H, t, J = 4.8 Hz), 3.45 (2H, s), 3.43 (2H, s), 3.26 (3H, s), 2.05 (3H, s).

### Example 21: 6-Amino-9-(4-morpholin-4-ylmethylbenzyl)-2-propoxy-7,9-dihydropurin-8-one (85 mg, 33 %)

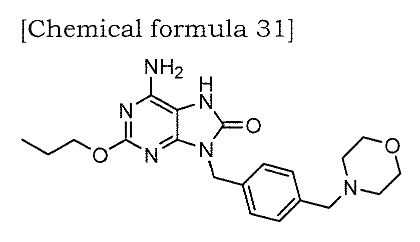

¹H NMR (DMSO-d₆) δ 9.96 (1H, s), 7.25 (4H, s), 6.46 (2H, s), 4.83 (2H, s), 4.09 (2H, t, J = 6.8 Hz), 3.54 (4H, t, J = 4.6 Hz), 3.40 (3H, s), 2.31 (4H, m), 1.65 (2H, m), 0.92 (3H, t, J = 7.2 Hz).

### Example 22: 6-Amino-2-cyclopropylmethoxy-9-(4-morpholin-4-ylmethylbenzyl)-7,9-dihydropurin-8-one) (58 mg, 42 %)

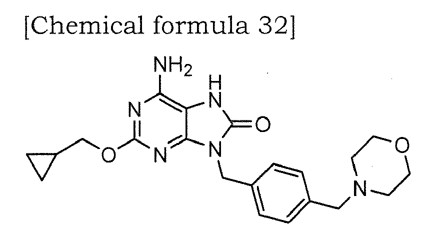

¹H NMR (DMSO-d₆) δ 9.96 (1H, s), 7.24 (4H, s), 6.44 (2H, s), 4.82 (2H, s), 3.97 (2H, d, J = 7.2 Hz), 3.53 (4H, t, J = 4.4 Hz), 3.40 (3H, s), 2.30 (4H, m), 1.17 (1H, m), 0.49 (2H, m), 0.26 (2H, m).

### Example 23: 6-Amino-9-(4-morpholin-4-ylmethylbenzyl)-2-(4, 4, 4-trifluorobutoxy)-7,9-dihydropurin-8-one (215 mg, 96 %)

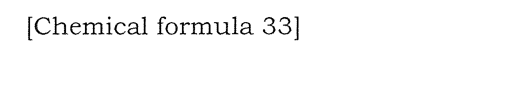

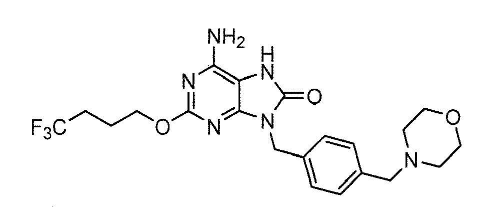

¹H NMR (DMSO-d₆) δ 10.01 (1H, s), 7.25 (4H, s), 6.50 (2H, s), 4.84 (2H, s), 4.21 (2H, t, J = 6.4 Hz), 3.54 (4H, t, J = 4.4 Hz), 3.40 (3H, s), 2.31-2.40 (6H, m), 1.86-1.92 (2H, m).

### Example 24: 6-Amino-9-[4-(4-methylpiperazin-1-ylmethyl)benzyl]-2-(4, 4, 4-trifluorobutoxy)-7,9-dihydropurin-8-one (84 mg, 49 %)

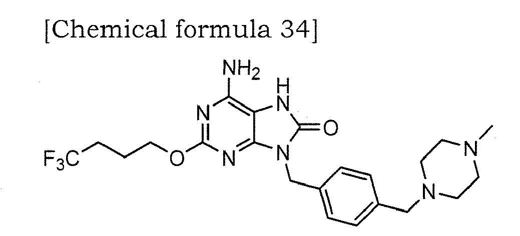

¹H NMR (DMSO-d₆) δ 9.98 (1H, s), 7.24 (4H, m), 6.50 (2H, s), 4.84 (2H, s), 4.21 (2H, t, J = 6.4 Hz), 3.39 (2H, s), 2.30-2.39 (10H, m), 2.12 (3H, s), 1.86-1.90 (2H, m).

### Example 25: 6-Amino-9-(4-{[(2-methoxyethyl)methylamino]methyl}-benzyl)-2-(4, 4, 4-trifluorobutoxy)-7,9-dihydropurin-8-one (64 mg, 57 %)

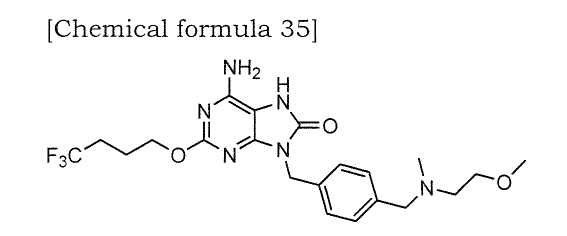

¹H NMR (DMSO-d₆) δ 9.89 (1H, s), 7.23 (4H, m), 6.50 (2H, s), 4.84 (2H, s), 4.21 (2H, t, J = 6.4 Hz), 3.43 (4H, m), 3.21 (3H, s), 2.49 (2H, m), 2.35 (2H, m), 2.12 (3H, s), 1.89 (2H, m).

### Example 26: 6-Amino-9-[4-(4-methoxypiperidin-1-ylmethyl)benzyl]-2-(2,2,2-trifluoroethoxy)-7,9-dihydropurin-8-one (140 mg, 77 %)

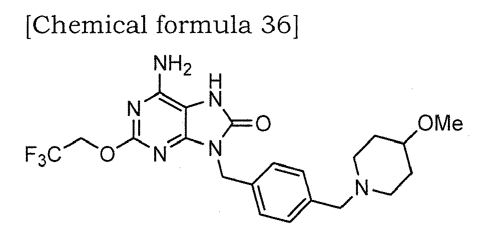

¹H NMR (DMSO-d₆) δ 10.08 (1H, s), 7.24 (4H, m), 6.65 (2H, s), 4.86 (4H, m), 3.38 (2H, s), 3.19 (3H, s), 3.15 (1H, m), 2.60 (2H, m), 2.03 (3H, t, J = 9.6 Hz), 1.41 (2H, m), 1.38 (2H, m).

### Example 27: 6-Amino-9-[4-(4-oxopiperidin-1-ylmethyl)benzyl]-2-(2,2,2-trifluoroethoxy)-7,9-dihydropurin-8-one (45 mg, 35 %)

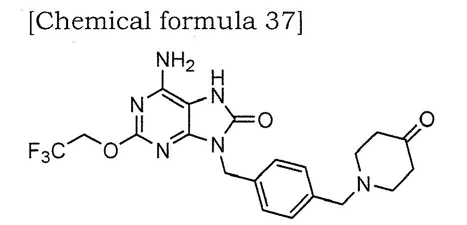

¹H NMR (DMSO-d₆) δ 10.09 (1H, s), 7.30 (4H, m), 6.66 (2H, s), 4.85 (4H, m), 3.56 (2H, s), 2.66 (4H, t, J = 6.0 Hz), 2.32 (4H, t, J = 6.0 Hz).

### Example 28: 6-Amino-2-butylamino-9-(4-dimethylaminomethylbenzyl)-7,9-dihydropurin-8-one (45 mg)

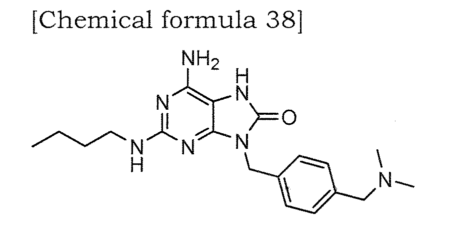

¹H NMR (DMSO-d₆) δ 9.62 (1H, s), 7.24 (2H, d, J = 8.4 Hz), 7.20 (2H, d, J = 8.4 Hz), 6.17 (1H, t, J = 5.8 Hz), 5.98 (2H, s), 4.79 (2H, s), 3.31 (2H, s), 3.16 (2H, 2H, q, J = 6.8 Hz), 2.12 (6H, s), 1.45 (2H, m), 1.29 (2H, m), 0.87 (3H, t, J = 7.2 Hz).

### Example 29: 6-Amino-2-butylamino-9-(4-piperidin-1-ylmethylbenzyl)-7,9-dihydropurin-8-one (48 mg)

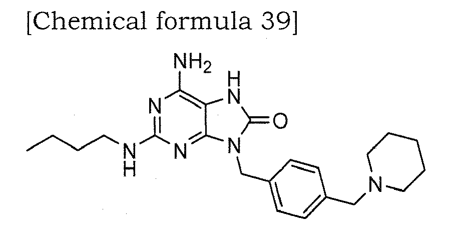

¹H NMR (DMSO-d₆) δ 9.63 (1H, s), 7.23 (2H, d, J = 8.4Hz), 7.20 (2H, d, J = 8.4Hz), 6.21 (1H, t, J = 5.6 Hz), 6.00 (2H, s), 4.78 (2H, s), 3.35 (2H, s), 3.16 (2H, q, J = 6.8 Hz), 2.26 (4H, m), 1.24-1.37 (10H, m), 0.87 (3H, t, J = 6.8 Hz).

### Example 30: 6-Amino-2-butylamino-9-(4-morpholin-4-ylmethylbenzyl)-7,9-dihydropurin-8-one (185 mg)

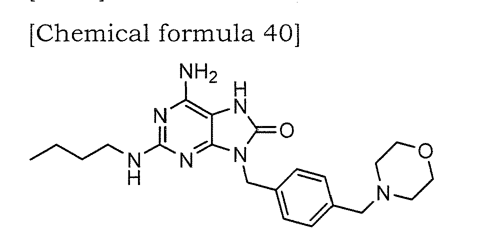

¹H NMR (DMSO-d₆) δ 9.62 (1H, s), 7.24 (4H, m), 6.17 (1H, t, J = 5.6 Hz), 5.98 (2H, s), 4.78 (2H, s), 3.54 (4H, t, J = 4.4 Hz), 3.41 (2H, s), 3.16 (2H, q, J = 6.8 Hz), 2.31 (4H, t, J = 4.0 Hz), 1.45 (2H, m), 1.30 (2H, m), 0.87 (3H, t, J = 7.2 Hz).

### Example 31: 6-Amino-2-butylamino-9-[4-(4-dimethylaminopiperidin-1-ylmethyl)benzyl]-7,9-dihydropurin-8-one (103 mg)

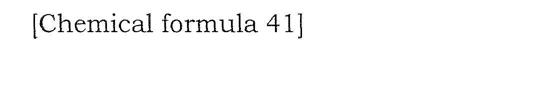

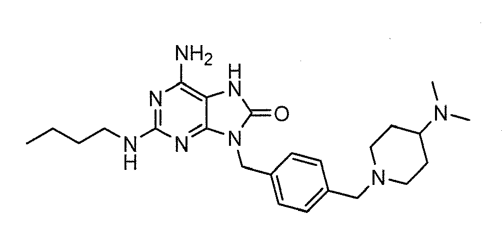

¹H NMR (DMSO-d₆) δ 9.63 (1H, s), 7.24 (2H, d, J = 8.4 Hz), 7.22 (2H, d, J = 8.4 Hz), 6.20 (1H, t, J = 6.0 Hz), 6.01 (2H, s), 4.79 (2H, s), 3.37 (2H, s), 3.15 (2H, m), 2.77 (2H, d, J = 11.6 Hz), 2.13 (6H, s), 1.95 (1H, m), 1.85 (2H, q, J = 8.0 Hz), 1.65 (2H, q, J = 8.0 Hz), 1.44 (2H, m), 1.26-1.33 (4H, m), 0.87 (3H, t, J = 7.2 Hz).

### Example 32: 6-Amino-2-butylamino-9-[4-(4-methylpiperazin-1-ylmethyl)benzyl]-7,9-dihydropurin-8-one (89 mg)

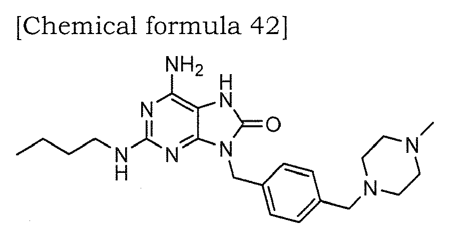

¹H NMR (DMSO-d₆) δ 9.62 (1H, s), 7.24 (2H, d, J = 8.4 Hz), 7.20 (2H, d, J = 8.4 Hz), 6.19 (1H, t, J = 5.6 Hz), 5.99 (2H, s), 4.78 (2H, s), 3.39 (2H, s), 3.16 (2H, q, J = 6.8 Hz), 2.30 (8H, m), 2.13 (3H, s), 1.45 (2H, m), 1.28 (2H, m), 0.87 (3H, t, J = 7.2 Hz).

### Example 33: 6-Amino-2-butylamino-9-(3-piperidin-1-ylmethylbenzyl)-7,9-dihydropurin-8-one (188 mg)

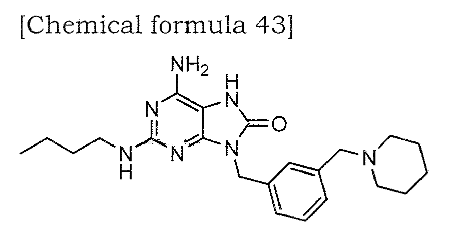

¹H NMR (DMSO-d₆) δ 9.63 (1H, s), 7.24 (2H, m), 7.16 (2H, m), 6.18 (1H, t, J = 5.6 Hz), 5.99 (2H, s), 4.79 (2H, s), 3.37 (2H, s), 3.16 (2H, q, J = 6.8 Hz), 2.34 (4H, m), 1.24-1.49 (10H, m), 0.87 (3H, t, J = 7.2 Hz). Example 34: 6-Amino-2-butoxy-9-(3-morpholin-4-ylmethylbenzyl)-7,9-dihydropurin-8-one (175 mg)

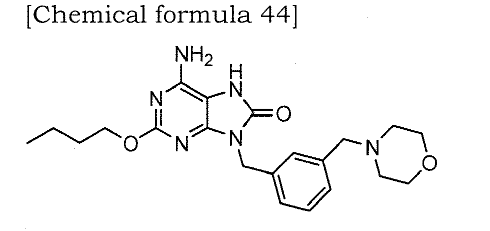

¹H NMR (DMSO-d₆) δ 9.95 (1H, s), 7.27 (2H, m), 7.18 (2H, m), 6.45 (2H, s), 4.85 (2H, s), 4.14 (2H, q, J = 6.4 Hz), 3.53 (4H, t, J = 4.4 Hz), 3.42 (2H, s), 2.31 (4H, m), 1.62 (2H, m), 1.37 (2H, m), 0.91 (3H, t, J = 7.2 Hz)

### Example 35: 6-Amino-9-[4-(4-amino-piperidin-1-ylmethyl)benzyl]-2-butoxy-7,9-dihydropurin-8-one

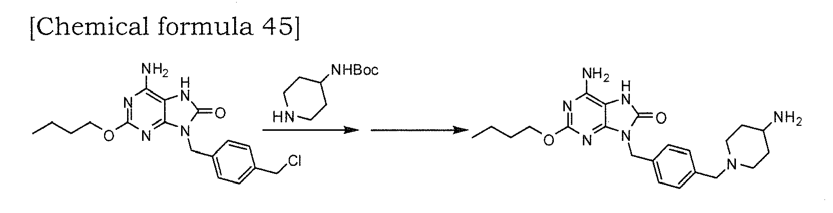

The compound obtained in Example 1, Step (vii) (150 mg, 0.42 mmol), 4-N-Boc-aminopiperidine (200 mg) and diisopropylethylamine (1 mL) were added to DMF (5 mL), and the mixture was heated at 80°C for 2 hours, and then, the solvent was evaporated. To the residue was added water, and the precipitated solid was collected by filtration, and purified by silica gel column chromatography (CHCl₃/MeOH=50/1) to give a white solid. The obtained solid was added to 10 % HCl/MeOH, and the mixture was stirred at room temperature overnight. The solvent was evaporated under reduced pressure, and the residue was dissolved in MeOH (3 mL), and thereto was added a 28 % aqueous ammonia. The precipitated solid was collected by filtration, and washed with water to give the title compound (114 mg, 64 %).
¹H NMR (DMSO-d₆) δ 7.25 (4H, m), 6.73 (2H, s), 4.83 (2H, s), 4.13 (2H, t, J = 6.4 Hz), 3.40 (2H, s), 2.91 (1H, m), 2.77 (2H, d, J = 11.6 Hz), 2.08 (1H, s), 1.81-1.96 (4H, m), 1.64 (2H, m), 1.32-1.53 (4H, m), 0.90 (3H, t, J = 7.2 Hz).

### Example 36: 6-Amino-2-butoxy-9-[4-(2-dimethylaminoethoxy)benzyl]-7,9-dihydropurin-8-one

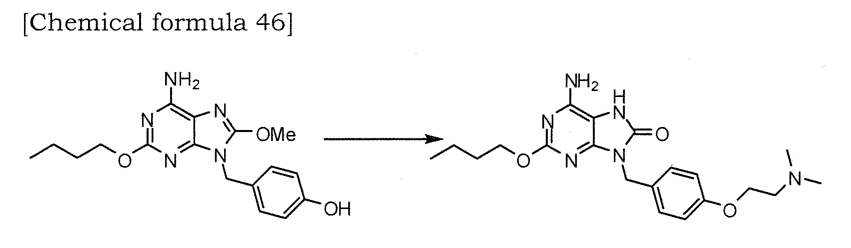

The compound obtained in Example 1, Step (vi) (100 mg, 0.3 mmol), N, N-dimethylaminoethylchloride hydrochloride (100 mg, 0.7 mmol) and potassium carbonate (140 mg, 1 mmol) were added to DMF (5 mL), and the mixture was stirred at room temperature for 2 days. The solvent was evaporated under reduced pressure, and chloroform and water were added to the residue for separation. The organic phase was dried over magnesium sulfate, and the solvent was evaporated under reduced pressure. To the residue was added 12N hydrochloric acid (5 mL), and the mixture was stirred at room temperature overnight, and then neutralized with a 28 % aqueous ammonia. The precipitated solid was collected by filtration, and washed with water to give the title compound (25 mg, 21 %).
¹H NMR (DMSO-d₆) δ 9.93 (1H, s), 7.23 (2H, d, J = 8.4 Hz), 6.87 (2H, d, J = 8.4 Hz), 6.45 (2H, s), 4.77 (2H, s), 4.15 (2H, t, J = 6.6 Hz), 3.99 (2H, t, J = 6.0 Hz), 2.57 (2H, t, J = 6.0 Hz, 2.18 (6H, s), 1.63 (2H, m), 1.38 (2H, m), 0.91 (3H, t, J = 7.4 Hz).
In a similar manner to Example 36, the compounds of the following Examples 37 to 39 were obtained.

### Example 37: 6-Amino-2-butoxy-9-[4-(3-dimethylaminopropoxy)benzyl]-7,9-dihydropurin-8-one (67 mg)

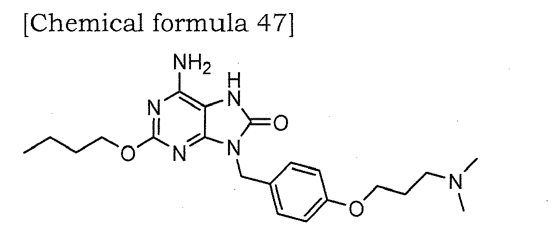

¹H NMR (DMSO-d₆) δ 9.94 (1H, s), 7.25 (2H, d, J = 8.4 Hz), 6.89 (2H, d, J = 8.4 Hz), 6.55 (2H, s), 4.78 (2H, s), 4.15 (2H, t, J = 6.6 Hz), 4.01 (2H, t, J = 6.0 Hz), 3.18 (2H, t, J = 8.0 Hz, 2.77 (6H, s), 2.02 (2H, m), 1.64 (2H, m), 1.39 (2H, m), 0.92 (3H, t, J = 7.2 Hz).

### Example 38: 6-Amino-2-(2-methoxyethoxy)-9-[4-(3-piperidin-1-yl-propoxy)benzyl]-7,9-dihydropurin-8-one) (114 mg)

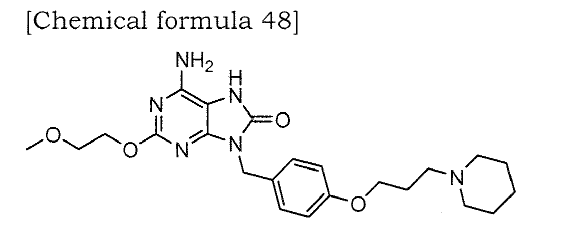

¹H NMR (DMSO-d₆) δ 9.92 (1H, s), 7.24 (2H, d, J = 8.8 Hz), 6.86 (2H, d, J = 8.4 Hz), 6.44 (2H, s), 4.77 (2H, s), 4.27 (2H, t, J = 4.8 Hz), 3.94 (2H, t, J = 6.4 Hz), 3.60 (2H, t, J = 4.8 Hz), 3.28 (3H, s), 2.34 (6H, m), 1.82 (2H, m), 1.46 (4H, m), 1.37 (2H, m).

### Example 39: 6-Amino-2-butylamino-9-[4-(3-morpholin-4-ylpropoxy)-benzyl]-7,9-dihydropurin-8-one (9 mg)

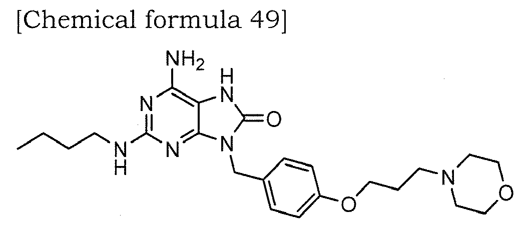

¹H NMR (DMSO-d₆) δ 9.62 (1H, s), 7.23 (2H, d, J = 8.4 Hz), 6.85 (2H, d, J = 8.4 Hz), 6.20 (1H, t, J = 6.0 Hz), 5.98 (2H, s), 4.69 (2H, s), 3.96 (2H, t, J = 6.0 Hz), 3.55 (4H, t, J = 4.6 Hz), 3.18 (2H, m), 2.37 (6H, s), 1.84 (2H, m), 1.45 (2H, m), 1.29 (4H, m), 0.88 (3H, t, J = 7.2 Hz).

### Example 40: 6-Amino-2-butoxy-9-[6-(4-methylpiperazin-1-yl)pyridin-3-ylmethyl]-7,9-dihydropurin-8-one

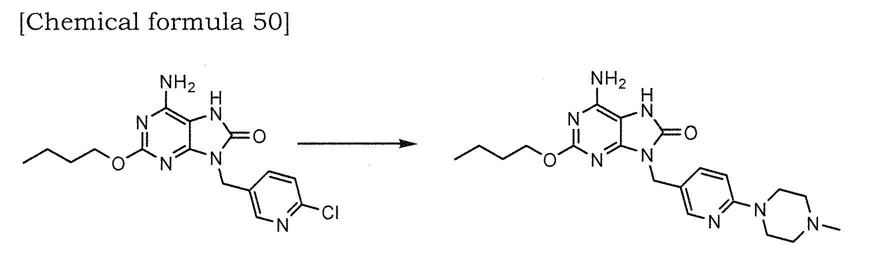

2-Butoxy-9-(6-chloropyridine-3-yl)methyl-8-oxoadenine (200 mg, 0.57 mmol), which was prepared in a similar manner to Example 1, and N-methylpiperazine (10 mL) were heated at 170°C for 10 hours. The solvent was evaporated under reduced pressure, and water was added thereto. The obtained solid was collected by filtration, and purified by silica gel column chromatography (CHCl₃/MeOH/28 % NH₃ = 100/3/1) to give the title compound (190 mg, 81 %).
¹H NMR (DMSO-d₆) δ 9.94 (1H, s), 8.11 (1H, d, J = 2.2 Hz), 7.50 (1H, dd, J = 2.4, 8.8 Hz), 6.77 (1H, d, J = 8.8 Hz), 6.44 (2H, s), 4.71 (2H, s), 4.16 (2H, t, J = 6.6 Hz), 3.42 (4H, t, J = 4.8 Hz), 2.35 (4H, t, J =4.8 Hz), 2.19 (3H, s), 1.65 (2H, m), 1.38 (2H, m), 0.92 (3H, t, J =7.2 Hz).
In a similar manner to Example 40, the compound of the following Examples 41 to 51 were obtained.

### Example 41: 6-Amino-2-butoxy-9-[6-(4-methyl-[1, 4]diazepan-1-yl)-pyridin-3-ylmethyl]-7,9-dihydropurin-8-one (125 mg, 64 %)

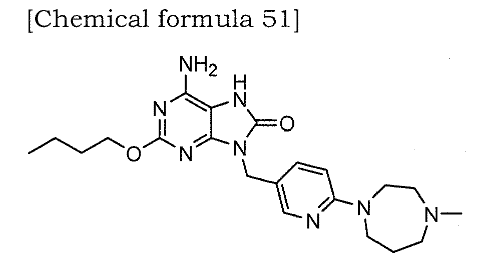

¹H NMR (DMSO-d₆) δ 9.92 (1H, s), 8.07 (1H, d, J = 2.2 Hz), 7.46 (1H, dd, J = 2.4, 8.8 Hz), 6.54 (1H, d, J = 8.8 Hz), 6.44 (2H, s), 4.69 (2H, s), 4.17 (2H, t, J = 6.6 Hz), 3.67 (2H, t, J = 4.8 Hz), 3.52 (2H, t, J = 6.2 Hz), 2.51 (2H, m), 2.40 (2H, t, J = 5.4 Hz), 2.21 (3H, s), 1.83 (2H, m), 1.66 (2H, m), 1.39 (2H, m), 0.92 (3H, t, J = 7.2 Hz).

### Example 42: 6-Amino-2-(2-methoxyethoxy)-9-[6-(4-methylpiperazin-1-yl)pyridin-3-ylmethyl]-7,9-dihydropurin-8-one (115 mg, 46 %)

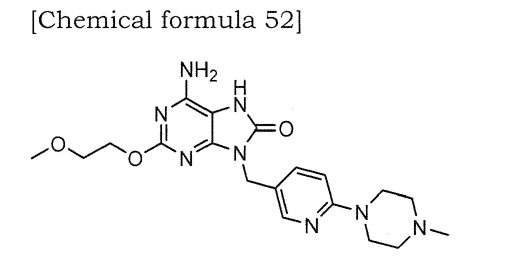

¹H NMR (DMSO-d₆) δ 9.93 (1H, s), 8.12 (1H, d, J = 2.2 Hz), 7.50 (1H, dd, J = 2.4, 8.8 Hz), 6.78 (1H, d, J = 9.2 Hz), 6.46 (2H, s), 4.72 (2H, s), 4.28 (2H, t, J = 4.8 Hz), 3.61 (4H, t, J = 4.8 Hz), 3.44 (4H, m), 3.29 (3H, s), 2.35 (4H, t, J = 5.0 Hz), 2.18 (3H, s).

### Example 43: 6-Amino-9-[6-(4-methylpiperazin-1-yl)pyridin-3-ylmethyl]-2-(4, 4, 4-trifluorobutoxy)-7,9-dihydropurin-8-one (99 mg, 43 %)

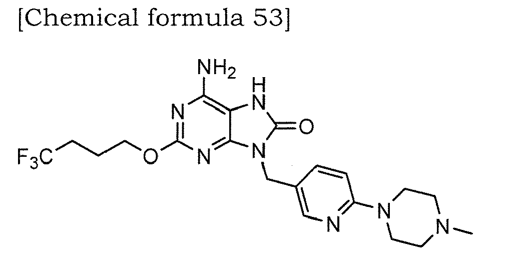

¹H NMR (DMSO-d₆) δ 9.97 (1H, s), 8.12 (1H, d, J = 2.1 Hz), 7.50 (1H, dd, J = 2.4, 8.8 Hz), 6.77 (1H, d, J = 8.8 Hz), 6.48 (2H, s), 4.72 (2H, s), 4.23 (2H, t, J = 6.4 Hz), 3.42 (4H, m), 2.37 (6H, m), 2.19 (3H, s), 1.90 (2H, m).

### Example 44: 6-Amino-2-ethoxy-9-[6-(4-methylpiperazin-1-yl)pyridin-3-ylmethyl]-7,9-dihydropurin-8-one (19 mg, 13 %)

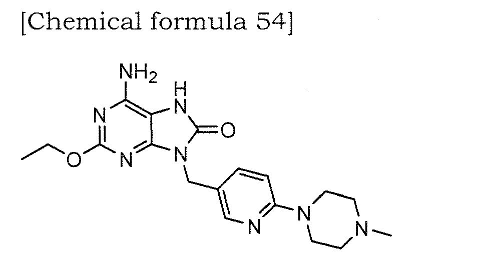

¹H NMR (DMSO-d₆) δ 9.91 (1H, s), 8.12 (1H, d, J = 2.1 Hz), 7.50 (1H, dd, J = 2.4, 8.8 Hz), 6.78 (1H, d, J = 8.8 Hz), 6.43 (2H, s), 4.71 (2H, s), 4.22 (2H, t, J = 6.8 Hz), 3.43 (4H, t, J = 4.8 Hz), 2.35 (4H, t, J = 5.0 Hz), 2.19 (3H, s), 1.26 (3H, t, J = 7.2 Hz).

### Example 45: 6-Amino-9-[6-(4-methylpiperazin-1-yl)pyridin-3-ylmethyl]-2-(2,2,2-trifluoroethoxy)-7,9-dihydropurin-8-one (104 mg, 39 %)

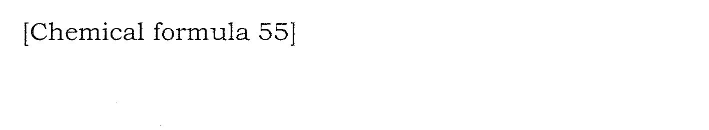

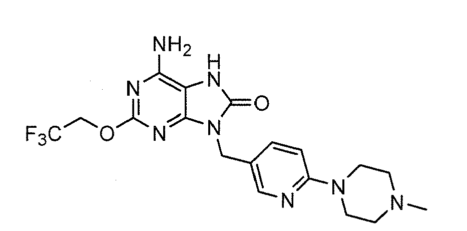

¹H NMR (DMSO-d₆) δ 10.05 (1H, s), 8.13 (1H, d, J = 2.1 Hz), 7.52 (1H, dd, J = 2.4, 8.8 Hz), 6.77 (1H, d, J = 8.8 Hz), 6.63 (2H, s), 4.89 (2H, q, J = 9.0 Hz), 4.74 (2H, s), 3.43 (4H, t, J = 4.8 Hz), 2.35 (4H, t, J = 5.0 Hz), 2.19 (3H, s).

### Example 46: 6-Amino-2-butylamino-9-[6-(4-methylpiperazin-1-yl)pyridin-3-ylmethyl]-7,9-dihydropurin-8-one (109 mg, 46 %)

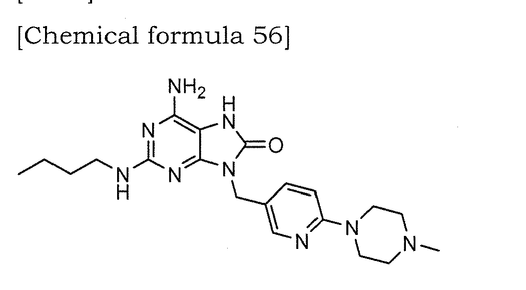

¹H NMR (DMSO-d₆) δ 9.59 (1H, s), 8.11 (1H, d, J = 2.1 Hz), 7.50 (1H, dd, J = 2.4, 8.8 Hz), 6.76 (1H, d, J = 8.8 Hz), 6.21 (1H, t, J = 5.4 Hz), 5.98 (2H, s), 4.65 (2H, s), 3.42 (4H, t, J = 4.8 Hz), 3.17 (2H, q, J = 6.8 Hz), 2.34 (4H, t, J = 5.0 Hz), 2.18 (3H, s), 1.46 (2H, m), 1.30 (2H, m), 0.88 (3H, t, J = 7.2 Hz).

### Example 47: 6-Amino-2-butylamino-9-[6-(4-methyl-[1, 4]diazepan-1-yl)-pyridin-3-ylmethyl]-7,9-dihydropurin-8-one (81 mg, 39 %)

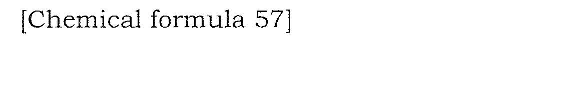

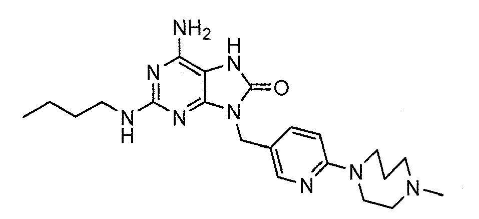

¹H NMR (DMSO-d₆) δ 9.58 (1H, s), 8.07 (1H, d, J = 2.1 Hz), 7.47 (1H, dd, J = 2.4, 8.0 Hz), 6.53 (1H, d, J = 8.8 Hz), 6.21 (1H, t, J = 5.6 Hz), 5.98 (2H, s), 4.64 (2H, s), 3.68 (4H, t, J = 4.8 Hz), 3.52 (2H, q, J = 6.0 Hz), 3.19 (2H, m), 2.53 (2H, m), 2.41 (2H, t, J = 5.2 Hz), 2.22 (3H, s), 1.84 (2H, m), 1.48 (2H, m), 1.31 (2H, m), 0.89 (3H, t, J = 7.2 Hz).

### Example 48: 6-Amino-2-butylamino-9-(6-piperazin-1-ylpyridin-3-ylmethyl)-7,9-dihydropurin-8-one (79 mg, 35 %)

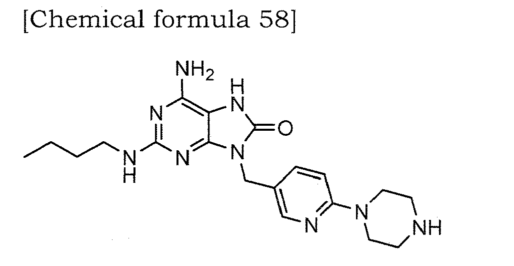

¹H NMR (DMSO-d₆) δ 9.59 (1H, brs), 8.11 (1H, d, J = 2.1 Hz), 7.50 (1H, dd, J = 2.4, 8.8 Hz), 6.72 (1H, d, J = 8.8 Hz), 6.17 (1H, t, J = 5.8 Hz), 5.97 (2H, s), 4.66 (2H, s), 3.34 (2H, m), 3.19 (4H, m), 2.74 (4H, t, J = 4.8 Hz), 1.47 (2H, m), 1.30 (2H, m), 0.89 (3H, t, J = 7.2 Hz).

### Example 49: 6-Amino-2-butylamino-9-[6-(4-dimethylaminopiperidin-1-yl)pyridin-3-ylmethyl]-7,9-dihydropurin-8-one (96 mg, 45 %)

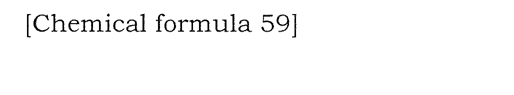

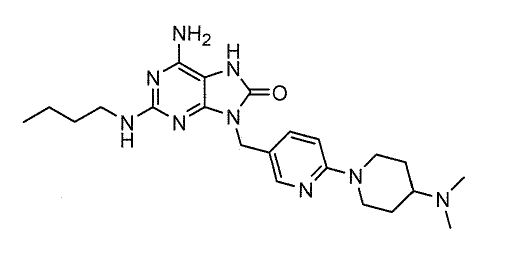

¹H NMR (DMSO-d₆) δ 9.59 (1H, s), 8.101 (1H, d, J = 2.2 Hz), 7.49 (1H, dd, J = 2.4, 8.8 Hz), 6.77 (1H, d, J = 8.8 Hz), 6.21 (1H, t, J = 5.6 Hz), 5.98 (2H, s), 4.65 (2H, s), 4.24 (2H, d, J = 13.2 Hz), 3.20 (2H, q, J = 6.8 Hz), 2.75 (2H, m), 2.27 (1H, m), 2.15 (6H, s), 1.76 (2H, d, J = 11.0 Hz), 1.46 (2H, m), 1.26-1.34 (4H, m), 0.89 (3H, t, J = 7.2 Hz).

### Example 50: 6-Amino-2-butylamino-9-{6-[(3-dimethylaminopropyl)-methylamino]pyridin-3-ylmethyl}-7,9-dihydropurin-8-one (32 mg, 15 %)

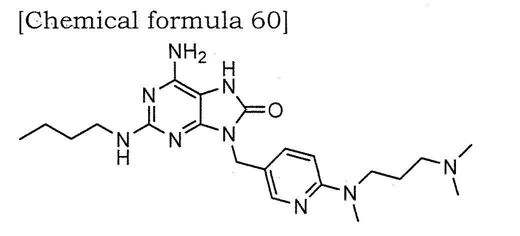

¹H NMR (DMSO-d₆) δ 9.58 (1H, brs), 8.08 (1H, d, J = 2.1 Hz), 7.48 (1H, dd, J = 2.4, 8.8 Hz), 6.53 (1H, d, J = 8.8 Hz), 6.21 (1H, t, J = 5.8 Hz), 5.97 (2H, s), 4.64 (2H, s), 3.46 (2H, t, J = 7.2 Hz), 3.19 (2H, q, J = 6.8 Hz), 2.94 (3H, s), 2.17 (2H, t, J = 7.0 Hz), 2.10 (6H, s), 1.60 (2H, m), 1.47 (2H, m), 1.31 (2H, m), 0.90 (3H, t, J = 7.2 Hz).

### Example 51: 6-Amino-2-butylamino-9-[6-(3-dimethylaminopyrrolidin-1-yl)pyridin-3-ylmethyl]-7,9-dihydropurin-8-one (135 mg, 69 %)

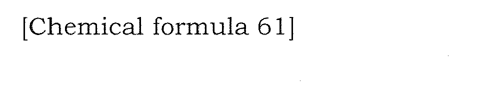

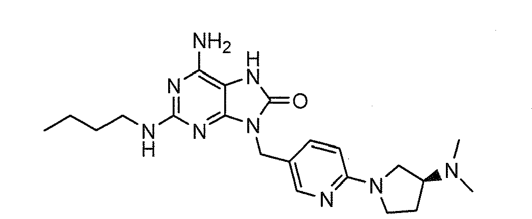

¹H NMR (DMSO-d₆) δ 9.60 (1H, brs), 8.11 (1H, d, J = 2.1 Hz), 7.50 (1H, dd, J = 2.4, 8.8 Hz), 6.37 (1H, d, J = 8.8 Hz), 6.16 (1H, t, J = 5.8 Hz), 5.94 (2H, s), 4.65 (2H, s), 3.61 (1H, m), 3.50 (1H, m), 3.17-3.27 (3H, m), 3.05 (1H, m), 2.17 (6H, s), 2.09 (1H, m), 1.76 (1H, m), 1.48 (2H, m), 1.31 (2H, m), 0.90 (3H, t, J = 7.2 Hz).

### Example 52: 6-Amino-2-butoxy-9-[6-(2-morpholin-4-ylethoxy)pyridin-3-ylmethyl]-7,9-dihydropurin-8-one

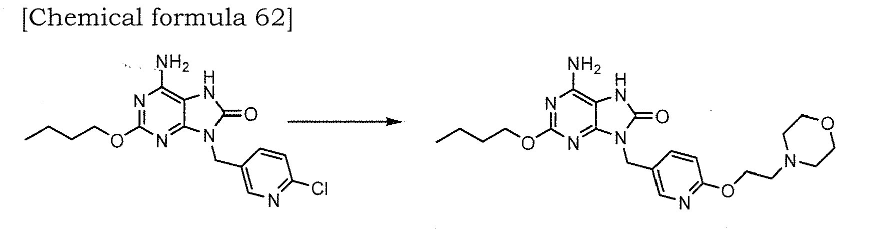

2-Butoxy-9-(6-chloropyridine-3-yl)methyl-8-oxoadenine (200 mg, 0.57 mmol) and sodium (100 mg) were added to morpholinoethanol (5 mL), and the mixture was heated at 140°C for 6 hours. The mixture was neutralized with 12N hydrochloric acid, and extracted with a mixture of CHCl₃/EtOH=3/1. The extract was dried by adding thereto magnesium sulfate, and the solvent was evaporated under reduced pressure. The residue was purified by (CHCl₃/MeOH=100/3) to give the title compound (115 mg, 45 %).
¹H NMR (DMSO-d₆) δ 9.95 (1H, s), 8.14 (1H, d, J = 2.4 Hz), 7.65 (1H, dd, J = 2.4, 8.4 Hz), 6.77 (1H, d, J = 8.8 Hz), 6.45 (2H, s), 4.80 (2H, s), 4.33 (2H, t, J = 6.0 Hz), 4.15 (2H, t, J = 6.4 Hz), 3.54 (4H, t, J = 4.4 Hz), 2.64 (2H, t, J = 5.6 Hz), 2.42 (4H, m), 1.63 (2H, m), 1.38 (2H, m), 0.91 (3H, t, J = 7.2 Hz).
In a similar manner to Example 52, the compounds of Examples 53 to 55 were obtained.

### Example 53: 6-Amino-2-butylamino-9-[6-(2-morpholin-4-ylethoxy)-pyridin-3-ylmethyl]-7,9-dihydropurin-8-one (108 mg, 57 %)

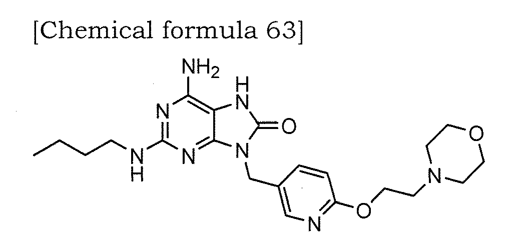

¹H NMR (DMSO-d₆) δ 9.62 (1H, brs), 8.13 (1H, d, J = 2.2 Hz), 7.65 (1H, dd, J = 2.4, 8.4 Hz), 6.76 (1H, d, J = 8.4 Hz), 6.22 (1H, t, J = 5.6 Hz), 6.00 (2H, s), 4.74 (2H, s), 4.33 (2H, t, J = 5.8 Hz), 3.55 (4H, m), 3.17 (2H, q, J = 6.8 Hz), 2.64 (2H, t, J = 5.8 Hz), 2.43 (4H, t, J = 4.4 Hz), 1.45 (2H, m), 1.30 (2H, m), 0.88 (3H, t, J = 7.2 Hz).

### Example 54: 6-Amino-2-butylamino-9-[6-(2-dimethylaminoethoxy)-pyridin-3-ylmethyl]-7,9-dihydropurin-8-one (27 mg, 13 %)

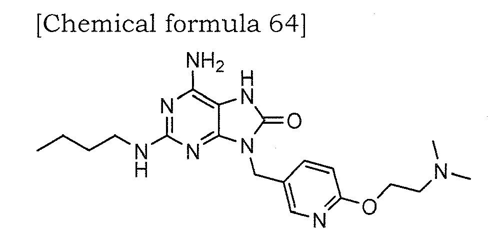

¹H NMR (DMSO-d₆) δ 9.64 (1H, brs), 8.13 (1H, d, J = 2.2 Hz), 7.65 (1H, dd, J = 2.4, 8.4 Hz), 6.76 (1H, d, J = 8.4 Hz), 6.26 (1H, t, J = 5.6 Hz), 6.02 (2H, s), 4.74 (2H, s), 4.33 (2H, t, J = 5.8 Hz), 3.17 (2H, q, J = 6.8 Hz), 2.56 (2H, t, J = 5.8 Hz), 2.17 (6H, s), 1.45 (2H, m), 1.30 (2H, m), 0.88 (3H, t, J = 7.2 Hz).

### Example 55: 6-Amino-2-butylamino-9-[6-(4-dimethylaminobutoxy)-pyridin-3-ylmethyl]-7,9-dihydropurin-8-one (24 mg, 15 %)

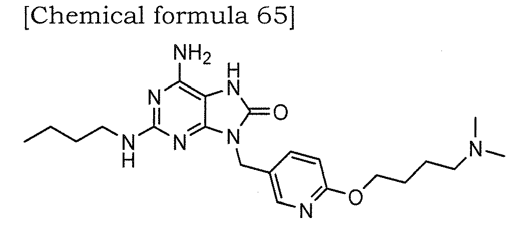

¹H NMR (DMSO-d₆) δ 9.63 (1H, brs), 8.13 (1H, d, J = 2.2 Hz), 7.64 (1H, dd, J = 2.4, 8.8 Hz), 6.75 (1H, d, J = 8.8 Hz), 6.23 (1H, t, J = 5.6 Hz), 6.00 (2H, s), 4.74 (2H, s), 4.21 (2H, t, J = 6.8 Hz), 3.17 (2H, q, J = 6.8 Hz), 2.09 (6H, s), 1.67 (2H, m), 1.44-1.52 (4H, m), 1.30 (2H, m), 0.88 (3H, t, J = 7.2 Hz).

### Example 56: 6-Amino-2-butylamino-9-[5-chloro-6-(4-methylpiperazin-1-yl)pyridin-3-ylmethyl]-7,9-dihydropurin-8-one

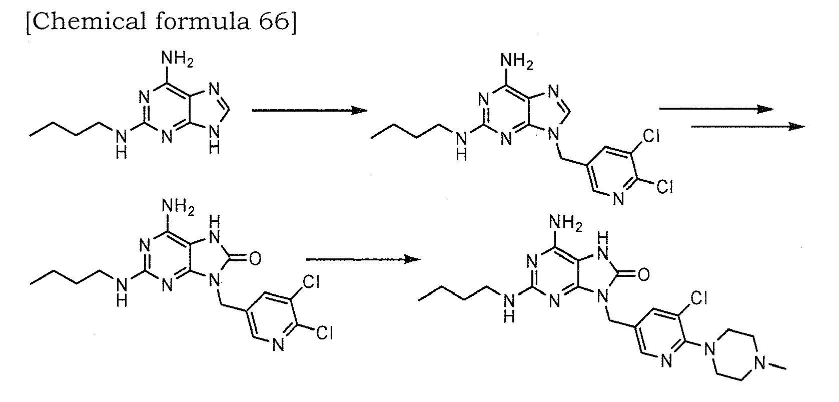

2-Butylaminoadenine (300 mg, 1.5 mmol), 5-chloromethyl-2, 3-dimethylpyridine (400 mg, 2 mmol) and potassium carbonate (420 mg, 3 mmol) were added to DMF (5 mL), and the mixture was heated at 60°C for 4 hours, and the solvent was evaporated. To the residue were added water and chloroform, and the mixture was separated. The organic phase was dried over magnesium sulfate. The residue was purified by silica gel column chromatography (CHC1₃/MeOH=50/1) to give a white solid (480 mg). The obtained solid and bromine (320 mg, 2 mmol) were added to chloroform (10 mL), and the mixture was stirred under ice-cooling for 2 hours. The precipitated yellow solid was collected by filtration, and thereto was added 12N hydrochloric acid, and the mixture was refluxed for 6 hours. The solvent was evaporated under reduced pressure, and neutralized with a 28 % aqueous ammonia to give 2-butylamino-9-(5, 6-dichloropyridin-3-ylmethyl)-8-oxoadenine as a white solid (350 mg). This solid (200 mg, 0.5 mmol) and N-methylpiperazine (5 mL) were heated at 130° for 3 hours. The solvent was evaporated under reduced pressure, and thereto was added water. The precipitated solid was collected by filtration, and purified by silica gel column chromatography (CHCl₃/MeOH=100/3) to give the title compound (114 mg, 50 %).
¹H NMR (DMSO-d₆) δ 9.64 (1H, s), 8.19 (1H, d, J = 1.8 Hz), 7.73 (1H, d, J = 1.8 Hz), 6.24 (1H, t, J = 5.6 Hz), 6.02 (2H, s), 4.75 (2H, s), 3.18 (4H, m), 2.44 (4H, t, J = 4.4 Hz), 2.21 (3H, s), 1.47 (2H, m), 1.30 (2H, m), 0.88 (3H, t, J = 7.2 Hz).

### Example 57: 6-Amino-9-[5-chloro-6-(4-methylpiperazin-1-yl)pyridin-3-ylmethyl]-2-ethoxy-7,9-dihydropurin-8-one

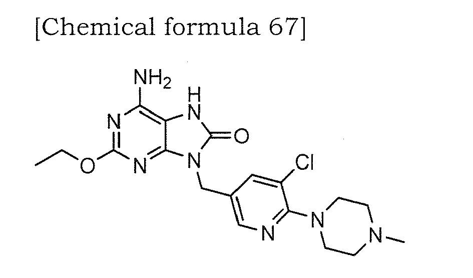

The title compound was obtained in a similar manner to Example 56 (78 mg, 41 %).
¹H NMR (DMSO-d₆) δ 9.97 (1H, brs), 8.19 (1H, d, J = 2.2 Hz), 7.74 (1H, d, J = 2.2 Hz), 6.48 (2H, s), 4.81 (2H, s), 4.21 (2H, q, J = 7.0 Hz), 3.17 (4H, m), 2.43 (4H, t, J = 4.4 Hz), 2.21 (3H, s), 1.27 (3H, t, J = 7.0 Hz). Example 58: 6-Amino-2-butylamino-9-[5-chloro-6-(2-dimethylamino-ethoxy)pyridin-3-ylmethyl]-7,9-dihydropurin-8-one

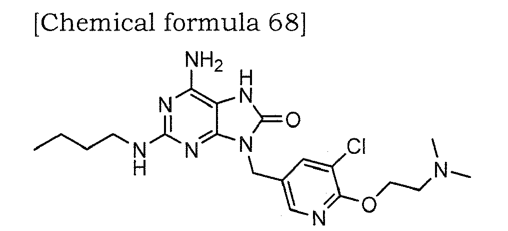

Using 2-butylamino-9-(5,6-dichloropyridin-3-ylmethyl)-8-oxoadenine obtained in Example 56, the title compound was obtained in a similar manner to Example 52 (104 mg, 54 %).
¹H NMR (DMSO-d₆) δ 9.65 (1H, s), 8.10 (1H, d, J = 1.8 Hz), 7.84 (1H, d, J = 1.8 Hz), 6.23 (1H, t, J = 5.8 Hz), 6.02 (2H, s), 4.77 (2H, s), 4.40 (2H, t, J = 5.8 Hz), 3.19 (2H, q, J = 6.8 Hz), 2.62 (2H, t, J = 5.8 Hz), 2.20 (6H, s), 1.46 (2H, m), 1.30 (2H, m), 0.88 (3H, t, J = 7.2 Hz).

### Example 59: 6-Amino-2-butylamino-9-[5-chloro-6-(2-morpholin-4-ylethoxy)pyridin-3-ylmethyl]-7,9-dihydropurin-8-one

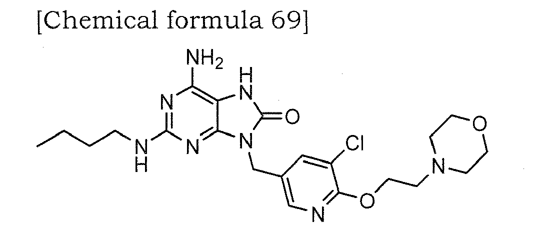

The title compound was obtained in a similar manner to Example 58 (78 mg, 31%).
¹H NMR (DMSO-d₆) δ 9.66 (1H, s), 8.09 (1H, d, J = 2.0 Hz), 7.84 (1H, d, J = 2.0 Hz), 6.25 (1H, t, J = 5.6 Hz), 6.02 (2H, s), 4.84 (2H, s), 4.43 (2H, t, J = 5.8 Hz), 3.54 (4H, t, J = 4.6 Hz), 3.18 (2H, q, J = 6.8 Hz), 2.68 (2H, t, J = 5.8 Hz), 2.46 (4H, t, J = 4.4 Hz), 1.45 (2H, m), 1.30 (2H, m), 0.88 (3H, t, J = 7.2 Hz).

### Example 60: 6-Amino-2-butylamino-9-[4-(4-methylpiperazin-1-yl)-3-nitrobenzyl]-7,9-dihydropurin-8-one

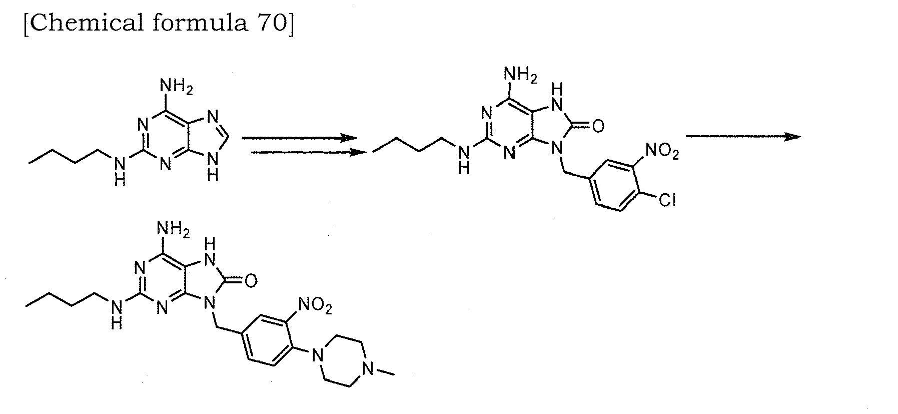

The title compound was obtained in a similar manner to Example 56 (390 mg, 100%).
¹H NMR (DMSO-d₆) δ 9.66 (1H, s), 7.75 (1H, d, J = 1.8 Hz), 7.48 (1H, dd, J = 2.0, 8.6 Hz), 7.27 (1H, d, J = 8.4 Hz), 6.21 (1H, t, J = 5.6 Hz), 6.02 (2H, s), 4.79 (2H, s), 3.16 (2H, q, J = 6.8 Hz), 2.95 (4H, t, J = 4.8 Hz), 2.20 (3H, s), 1.45 (2H, m), 1.29 (2H, m), 0.86 (3H, t, J = 7.2 Hz).

### Example 61: 6-Amino-9-[3-amino-4-(4-methylpiperazin-1-yl)benzyl]-2-butylamino-7,9-dihydropurin-8-one

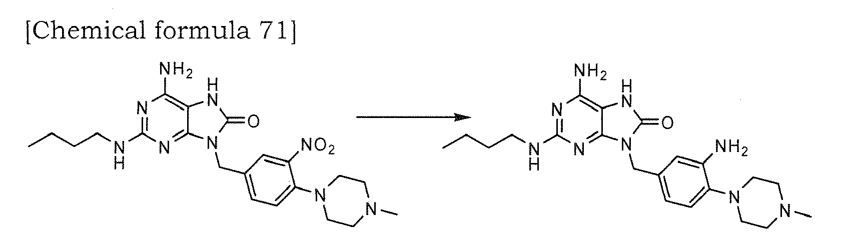

The compound of Example 60 (240 mg, 0.5 mmol) and 20 % Pd(OH)₂/C (50 mg) were added to methanol (10 mL), and the mixture was stirred under hydrogen atmosphere for 5 hours. The catalyst was removed, and water was added thereto, and the precipitated solid was collected by filtration to give the title compound (170 mg, 76 %).
¹H NMR (DMSO-d₆) δ 9.59 (1H, s), 6.80 (1H, d, J = 4.0 Hz), 6.60 (1H, s), 6.49 (1H, d, J = 8.0 Hz), 6.15 (1H, t, J = 5.6 Hz), 5.97 (2H, s), 4.71 (2H, s), 4.63 (2H, s), 3.16 (2H, q J = 6.8 Hz), 2.75 (4H, m), 2.49 (4H, m), 2.22 (3H, s), 1.45 (2H, m), 1.30 (2H, m), 0.87 (3H, t, J = 7.2 Hz).

### Example 62: 6-Amino-2-ethoxy-9-(3-methoxy-4-morpholin-4-ylmethylbenzyl)-7,9-dihydropurin-8-one

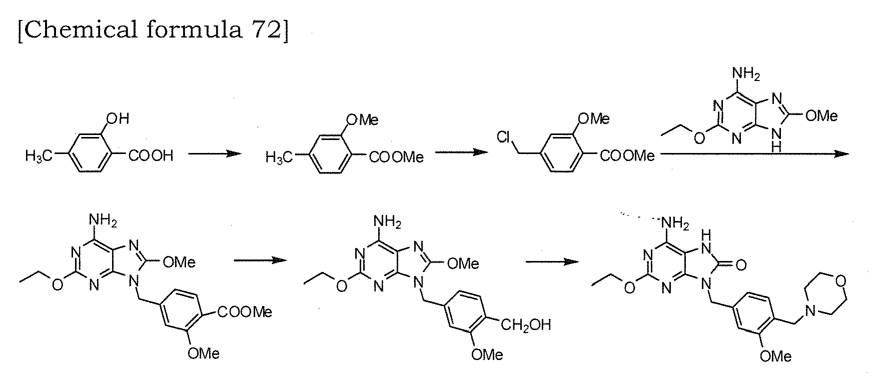

2-Hydroxy-4-methylbenzoic acid (600 mg, 4 mmol), methyl iodide (1.4 g, 10 mmol) and potassium carbonate (1.4 g, 10 mmol) were added to DMF (10 mL), and the mixture was stirred at room temperature for 3 hours. The solvent was evaporated under reduced pressure, and to the residue were added ethyl acetate and water. The mixture was separated, and dried over magnesium sulfate. The solvent was evaporated under reduced pressure, and to the residue were added NBS (700 mg, 5 mmol), benzoyl peroxide (100 mg) and carbon tetrachloride (6 mL), and the mixture was stirred at 80°C for 5 hours. The insoluble materials were separated by filtration, and the filtrate was washed with sodium thiosulfate and water, and dried over magnesium sulfate. The solvent was evaporated under reduced pressure, and the obtained residue was added to 2-ethoxy-8-methoxyadenine TFA salt (250 mg, 0.8 mmol), potassium carbonate (420 mg, 3 mmol) and DMF (5 mL), and the mixture was stirred at room temperature overnight. The solvent was removed by evaporation, and water and chloroform were added to the residue, and the mixture was separated. The organic phase was dried over magnesium sulfate, and purified by silica gel column chromatography (CHCl₃/MeOH=50/1) to give a white solid (310 mg). This solid and lithium aluminum hydride (74 mg, 2 mmol) were added to THF (10 mL), and the mixture was stirred under ice-cooling for 2 hours. The insoluble materials were removed by filtration, and the filtrate was concentrated under reduced pressure. To the residue were added thionyl chloride (0.3 mL) and dichloromethane (5 mL), and the mixture was stirred at 40°C for 2 hours. The solvent was removed by evaporation, and to the residue were added morpholine (0.2 mL), diisorpopylethylamine (0.5 mL) and DMF (5mL), and the mixture was heated at 80°C with stirring for 2 hours. The solvent was removed by evaporation, and the residue was purified by silica gel column chromatography (CHCl₃/MeOH/28 % NH₃=100/3/2) to give the title compound (46 mg).
¹H NMR (DMSO-d₆) δ 9.95 (1H, s), 7.22 (1H, d, J = 8.0 Hz), 7.00 (1H, d, J = 1.2 Hz), 6.78 (1H, dd, J = 1.4, 8.0 Hz), 6.46 (2H, s), 4.83 (2H, s), 4.20 (2H, q, J = 7.0 Hz), 3.74 (3H, s), 3.54 (4H, t, J = 4.6 Hz), 3.40 (3H, s), 2.34 (4H, m), 1.25 (3H, t, J = 7.0 Hz).

### Example 63: 6-Amino-9-(4-dimethylaminomethylbenzyl)-2-ethoxy-7,9-dihydropurin-8-one

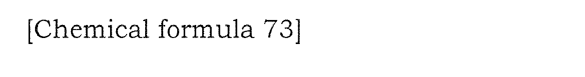

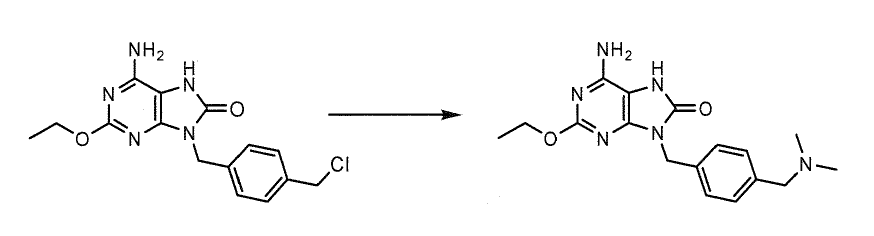

6-Amino-9-(4-chloromethylbenzyl)-2-ethoxy-7,9-dihydropurin-8-one hydrochloride (0.15 g, 0.41 mmol), which was obtained in a similar manner to Example 1, was dissolved in DMF (10 ml), and thereto was added a 40 % aqueous dimethylamine solution (2 ml), and the mixture was stirred at room temperature for 10 minutes. The mixture was concentrated by an evaporator, and thereto was added a 1 % aqueous ammonia (5 ml). The precipitated solid was collected by filtration, dried, and recrystallized from methanol/acetonitrile = 1/1 to give the title compound (0.080 g) as a white solid. Yield: 57 %.
¹H NMR (DMSO-d₆) δ 9.96 (1H, s), 7.25-7.20 (4H, m), 6.45 (2H, brs), 4.82 (2H, s), 4.18 (2H, q, J = 7.0 Hz), 3.32 (2H, s), 2.10 (s, 6H), 1.24 (3H, t, J = 7.0 Hz).

### Example 64: 6-Amino-9-(4-diethylaminomethylbenzyl)-2-ethoxy-7,9-dihydropurin-8-one

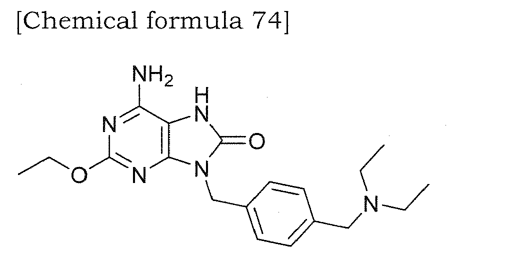

The starting compound of Example 63 (0.15 g, 0.41 mmol) was dissolved in DMF (10 ml), and thereto was added diethylamine (0.22 ml, 2.1 mmol), and the mixture was stirred at room temperature for 8 hours. The mixture was evaporated by an evaporator, and thereto was added 1 % aqueous ammonia (10 ml). The precipitated solid was collected by filtration, dried, and recrystallized from methanol/acetonitrile = 1/1 to give the title compound (0.12 g) as a white solid. Yield: 81 %.
¹H NMR (DMSO-d₆) δ 9.94 (1H, s), 7.25-7.20 (4H, m), 6.44 (2H, brs), 4.82 (2H, s), 4.18 (2H, q, J = 7.0 Hz), 3.46 (2H, s), 2.40 (4H, q, J = 7.1 Hz), 1.24 (3H, t, J = 7.0 Hz), 0.94 (6H, t, J = 7.1 Hz).
In a similar manner to Example 64, the compounds of the following Examples 65 to 70 were obtained.

### Example 65: 6-Amino-9-(4-diisopropylaminomethylbenzyl)-2-ethoxy-7,9-dihydropurin-8-one, white solid (Yield: 0.098 g, Yield: 61 %)

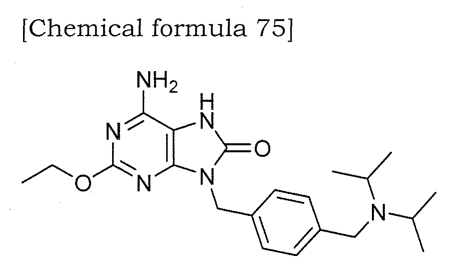

¹H NMR (DMSO-d₆) δ 9.93 (1H, s), 7.27 (2H, d, J = 8.1 Hz), 7.20 (2H, d, J = 8.1 Hz), 6.43 (2H, brs), 4.81 (2H, s), 4.18 (2H, q, J = 7.0 Hz), 3.55 (2H, s), 2.92 (2H, sep, J = 6.6 Hz), 1.24 (3H, t, J = 7.0 Hz), 0.95 (12H, d, J = 6.6 Hz).

### Example 66: 6-Amino-2-ethoxy-9-(4-piperidin-1-ylmethylbenzyl)-7,9-dihydropurin-8-one), White solid, Yield: 0.14 g (90 %).

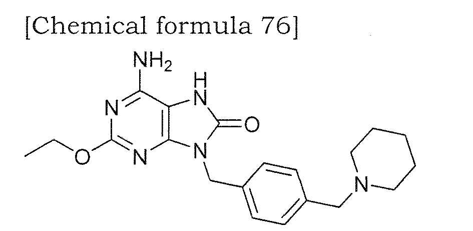

¹H NMR (DMSO-d₆) δ 9.97 (1H, s), 7.25-7.20 (4H, m), 6.45 (2H, brs), 4.82 (2H, s), 4.18 (2H, q, J = 7.0 Hz), 3.35 (2H, s), 2.33-2.24 (4H, m), 1.48-1.42 (4H, m), 1.37-1.34 (2H, m), 1.24 (3H, t, J = 7.0 Hz).

### Example 67: 6-Amino-2-ethoxy-9-[4-(4-methoxypiperidin-1-ylmethyl)benzyl]-7,9-dihydropurin-8-one, White solid, Yield: 0.10 g (62 %).

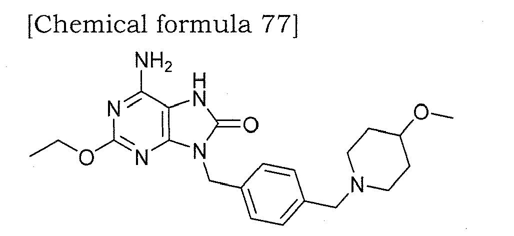

Using the compound obtained in Example 63, Step (ii) (0.15 g, 0.41 mmol), the title compound was obtained in a similar manner to Example 64.
¹H NMR (DMSO-d₆) δ 9.95 (1H, s), 7.26-7.20 (4H, m), 6.44 (2H, brs), 4.82 (2H, s), 4.18 (2H, q, J = 7.0 Hz), 3.38 (2H, s), 3.19 (3H, s), 3.16-3.11 (1H, m), 2.60-2.56 (2H, m), 2.05-1.99 (2H, m), 1.79-1.76 (2H, m), 1.42-1.35 (2H, m), 1.24 (3H, t, J = 7.0 Hz).

### Example 68: 6-Amino-2-ethoxy-9-(4-morpholin-4-ylmethylbenzyl)-7,9-dihydropurin-8-one), White solid, Yield: 0.11 g (68 %).

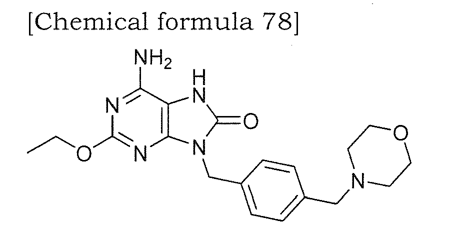

¹H NMR (DMSO-d₆) δ 9.95 (1H, s), 7.25-7.23 (4H, m), 6.45 (2H, brs), 4.82 (2H, s), 4.18 (2H, q, J = 7.0 Hz), 3.53 (4H, t, J = 4.6 Hz), 3.40 (2H, s), 2.30 (4H, t, J = 4.6 Hz), 1.24 (3H, t, J = 7.0 Hz).

### Example 69: 6-Amino-2-ethoxy-9-(4-thiomorpholin-4-ylmethylbenzyl)-7,9-dihydropurin-8-one, White solid, Yield: 0.13 g (80 %).

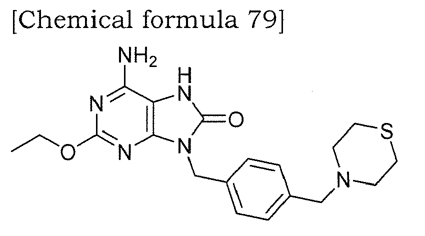

¹H NMR (DMSO-d₆) δ 9.96 (1H, s), 7.26-7.21 (4H, m), 6.45 (2H, brs), 4.82 (2H, s), 4.18 (2H, q, J = 7.0 Hz), 3.43 (2H, s), 2.57-2.54 (8H, m), 1.24 (3H, t, J = 7.0 Hz).

### Example 70: 6-Amino-2-ethoxy-9-[4-(4-methylpiperazin-1-ylmethyl)-benzyl]-7,9-dihydropurin-8-one, White solid, Yield: 0.094 g (58 %).

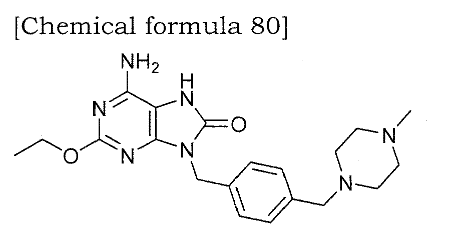

¹H NMR (DMSO-d₆) δ 9.95 (1H, s), 7.26-7.20 (4H, m), 6.45 (2H, brs), 4.82 (2H, s), 4.18 (2H, q, J = 7.0 Hz), 3.38 (2H, s), 2.50-2.10 (8H, m), 2.12 (3H, s), 1.24 (3H, t, J = 7.0 Hz).

### Example 71: 6-Amino-2-butyl-9-(4-dimethylaminomethylbenzyl)-7,9-dihydropurin-8-one

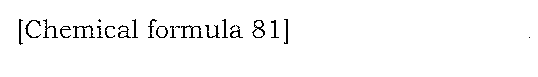

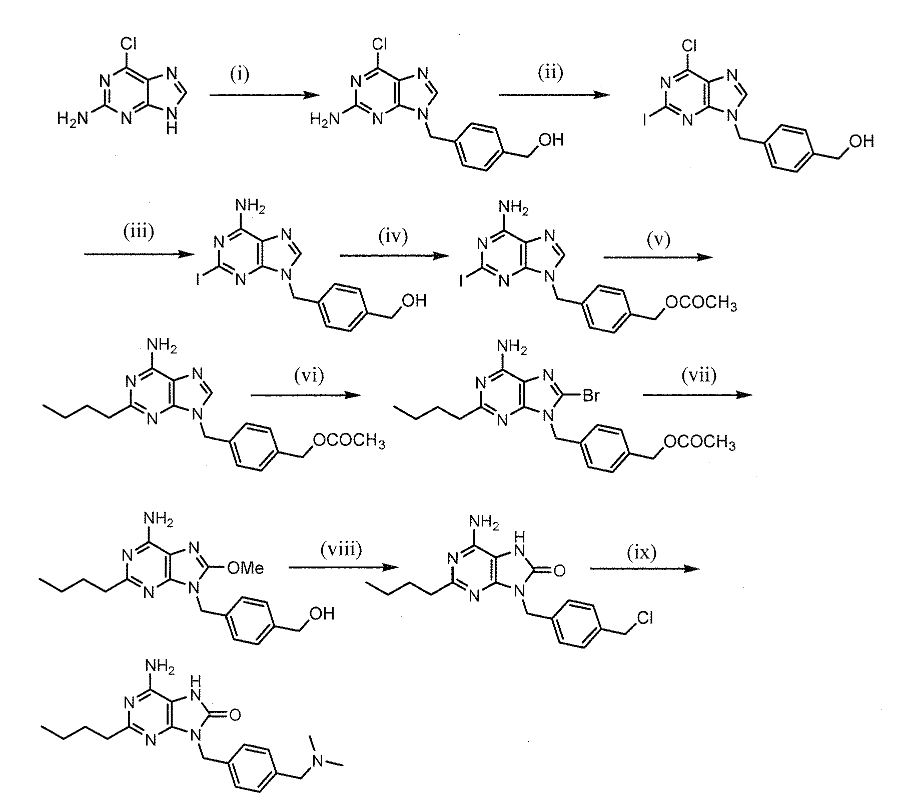

### Step (i): [4-(2-Amino-6-chloropurin-9-ylmethyl)phenyl]methanol

To a suspension of 2-amino-6-chloropurine (1.51 g, 8.9 mmol) in DMF (30 ml) were added potassium carbonate (3.69 g, 26.7 mmol) and (4-hydroxymethyl)benzyl chloride (2.09 g, 13.4 mmol), and the mixture was stirred at room temperature for 18 hours. The carbonate within the reaction system was removed by filtration, and the filtrate was concentrated. To the residue was added water, and the solid was collected by filtration, and further thereto were added chloroform (30 ml) and acetonitrile (15 ml). The mixture was stirred under reflux for 30 minutes, and cooled to 0°C. The crystals were collected by filtration to give the sub-title compound (2.15 g, 7.43 mmol) as a white solid. Yield: 84 %.
¹H NMR (DMSO-d₆) δ 8.22 (1H, s), 7.27 (2H, d, J = 8.2 Hz), 7.21 (2H, d, J = 8.2 Hz), 6.93 (2H, brs), 5.26 (2H, s), 5.16 (1H, t, J = 5.7 Hz), 4.45 (2H, d, J = 5.7 Hz).

### Step (ii): [4-(6-Chloro-2-iodopurin-9-ylmethyl)phenyl]methanol

To the compound obtained in Step (i) (1.87 g. 6.44 mmol) was added THF (65 ml), and to the resulting suspension were added copper (I) iodide (1.23 g, 6.44 mmol), diiodomethane (2.64 ml, 32.8 mmol), and isoamyl nitrite ( 2.59 ml, 19.3 mmol), and the mixture was stirred at 60°C for 3 hours. The mixture was concentrated, and purified by silica gel column (chloroform/methanol = 100/0 to 100/1) to give the sub-title compound (1.34 g, 3.35 mmol) as a white solid. Yield: 52 %.
¹H NMR (CDCl₃) δ 7.97 (1H, s), 7.42 (2H, d, J = 8.2 Hz), 7.33 (2H, d, J = 8.2 Hz), 5.42 (2H, s), 4.74 (2H, s), 1.74 (1H, brs).

### Step (iii): [4-(6-Amino-2-iodopurin-9-ylmethyl)phenyl]methanol

The compound obtained in Step (ii) (1.34 g, 3.35 mmol) was dissolved in THF (100 ml), and thereto was added a 28 % aqueous ammonia (33 ml), and the mixture was stirred at room temperature for 5 days. After concentration, water was added to the residue, and the precipitated solid was collected by filtration, and dried to give the sub-title compound (1.17 g, 3.06 mmol) as a white solid. Yield: 92 %.
¹H NMR (DMSO-d₆) δ 8.13 (1H, s), 7.67 (2H, brs), 7.28 (2H, d, J = 8.1 Hz), 7.21 (2H, d, J = 8.2 Hz), 5.29 (2H, s), 5.16 (1H, t, J = 5.6 Hz), 4.45 (2H, d, J = 5.6 Hz).

### Step (iv): 4-(6-Amino-2-iodopurin-9-ylmethyl)benzyl acetate

The compound obtained in Step (iii) (1.17 g, 3.06 mmol) was dissolved in DMF (10 ml), and thereto were added acetic anhydride (0.58 ml, 6.12 mmol) and triethylamine (0.85 ml, 6.12 mmol), and the mixture was stirred at room temperature for 16 hours. After concentration, water was added to the residue, and the precipitated solid was collected by filtration and dried to give the sub-title compound (1.30 g, 3.06 mmol) as a white solid. Yield: 100 %.
¹H NMR (DMSO-d₆) δ 8.15 (1H, s), 7.68 (2H, brs), 7.34 (2H, d, J = 8.1 Hz), 7.25 (2H, d, J = 8.1 Hz), 5.32 (2H, s), 5.03 (2H, s), 2.04 (3H, s).

### Step (v): 4-(6-Amino-2-butylpurin-9-ylmethyl)benzyl acetate

The compound obtained in Step (iv) (1.30 g, 3.06 mmol) was dissolved in DMF (15 ml), and thereto were added tetrakistriphenyl phosphine palladium (0.18 g, 0.15 mmol) and, zinc butylbromide (0.5M THF solution, 42 ml, 21 mmol), and the mixture was stirred at room temperature for 1 hour. The reaction was quenched with 0.5M hydrochloric acid, and then extracted with chloroform three times. The organic layer was concentrated, and purified by silica gel column (chloroform/methanol=100/0 to 100/1) to give the sub-title compound (0.87 g, 2.45 mmol) as a white solid. Yield: 80 %.
¹H NMR (CDCl₃) δ 7.93 (1H, s), 7.37 (2H, d, J = 8.1 Hz), 7.32 (2H, d, J = 8.1 Hz), 6.21 (2H, brs), 5.37 (2H, s), 5.11 (2H, s), 2.87 (2H, t, J = 7.8 Hz), 2.11 (3H, s), 1.90-1.77 (2H, m), 1.49-1.38 (2H, m), 0.97 (3H, t, J = 7.4 Hz).

### Step (vi): 4-(6-Amino-8-bromo-2-butylpurin-9-ylmethyl)benzyl acetate

The compound obtained in Step (v) (0.84 g, 2.38 mmol) was dissolved in chloroform (20 ml), and the mixture was cooled to 0°C. To the mixture was added sodium acetate (0.98 g, 11.9 mmol), and thereto was slowly added dropwise a solution of bromine (0.76 g, 4.76 mmol) in chloroform (5 ml). The mixture was warmed to room temperature, and stirred for 3 hours. The mixture was cooled to 0°, and thereto were added a saturated sodium hydrogen carbonate solution, a saturated aqueous sodium thiosulfate solution, and the mixture was extracted with chloroform three times. The organic layer was concentrated and purified by silica gel column (chloroform) to give the sub-title compound (0.77 g, 1.78 mmol) as a pale yellow solid. Yield: 75 %.
¹H NMR (CDCl₃) δ 7.35 (2H, d, J = 8.2 Hz), 7.30 (2H, d, J = 8.2 Hz), 5.43 (2H, brs), 5.37 (2H, s), 5.07 (2H, s), 2.80 (2H, t, J = 7.8 Hz), 2.09 (3H, s), 1.83-1.75 (2H, m), 1.44-1.36 (2H, m), 0.95 (3H, t, J = 7.4 Hz).

### Step (vii): [4-(6-Amino-2-butyl-8-methoxypurin-9-ylmethyl)phenyl]-methanol

The compound obtained in Step (vi) (0.77 g, 1.78 mmol) was suspended in methanol (30 ml), and thereto was added 2M aqueous sodium hydroxide solution (15 ml), and the mixture was stirred under reflux for 1.5 hour. The mixture was cooled to 0°C, and the mixture was neutralized with 1 M hydrochloric acid. The mixture was extracted with chloroform three times to give the sub-title compound (0.61 g, 1.78 mmol) as a pale brown solid. Yield: 100 %.
¹H NMR (DMSO-d₆) δ 7.25 (2H, d, J = 8.1 Hz), 7.18 (2H, d, J = 8.1 Hz), 6.69 (2H, brs), 5.14 (1H, t, J = 4.5 Hz), 5.07 (2H, s), 4.43 (2H, d, J = 4.5 Hz), 4.05 (3H, s), 2.60 (2H, t, J = 7.6 Hz), 1.72-1.62 (2H, m), 1.36-1.25 (2H, m), 0.88 (3H, t, J = 7.3 Hz).

### Step (viii): 6-Amino-2-butyl-9-(4-chloromethylbenzyl)-7,9-dihydropurin-8-one hydrochloride

Using the compound obtained in Step (vii) (0.61 g, 1.78 mmol), the sub-title compound was obtained in a similar manner to Example 1 (0.64 g, 1.67 mmol) as a white solid. Yield: 93 %.
¹H NMR (DMSO-d₆) δ 10.89 (1H, brs), 7.38 (2H, d, J = 8.2 Hz), 7.29 (2H, d, J = 8.2 Hz), 6.80 (2H, brs), 4.98 (2H, s), 4.73 (2H, s), 2.69 (2H, t, J = 7.5 Hz), 1.72-1.62 (2H, m), 1.36-1.24 (2H, m), 0.88 (3H, t, J = 7.3 Hz). Step (ix): 6-Amino-2-butyl-9-(4-dimethylaminomethylbenzyl)-7,9-dihydropurin-8-one

Using the compound obtained in Step (viii) (0.12 g, 0.32 mmol), the title compound was obtained in a similar manner to Example 64 (0.076 g, 0.21 mmol) as a white solid. Yield: 68 %.
¹H NMR (DMSO-d₆) δ 10.13 (1H, brs), 7.24 (2H, d, J = 8.3 Hz), 7.20 (2H, d, J = 8.3 Hz), 6.35 (2H, brs), 4.87 (2H, s), 3.32 (2H, s), 2.56 (2H, t, J = 7.6 Hz), 2.09 (6H, s), 1.67-1.58 (2H, m), 1.31-1.22 (2H, m), 0.86 (3H, t, J = 7.4 Hz).

### Example 72: 6-Amino-2-butyl-9-(4-morpholin-4-ylmethylbenzyl)-7,9-dihydropurin-8-one

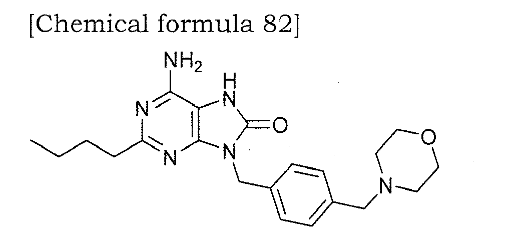

Using the compound obtained in Example 71, Step (viii) (0.13 g, 0.35 mmol), the title compound (0.095 g, 0.24 mmol) was obtained in a similar manner to Example 64 as a white solid. Yield: 68 %.
¹H NMR (DMSO-d₆) δ10.12 (1H, brs), 7.30-7.20 (4H, m), 6.35 (2H, brs), 4.87 (2H, s), 3.53 (4H, t, J = 4.6 Hz), 3.43 (2H, s), 2.56 (2H, t, J = 7.6 Hz), 2.35-2.28 (4H, m), 1.67-1.58 (2H, m), 1.33-1.21 (2H, m), 0.86 (3H, t, J = 7.4 Hz).

### Example 73: 6-Amino-2-butyl-9-[4-(4-methoxypiperidin-1-ylmethyl)-benzyl]-7,9-dihydropurin-8-one)

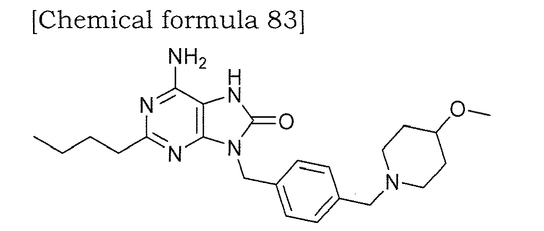

Using the compound obtained in Example 71, Step (viii) (0.13 g, 0.35 mmol), the title compound (0.090 g, 0.21 mmol) was obtained in a similar manner to Example 64 as a white solid. Yield: 60 %.
¹H NMR (DMSO-d₆) δ10.11 (1H, brs), 7.26-7.18 (4H, m), 6.35 (2H, brs), 4.87 (2H, s), 3.36 (2H, s), 3.19 (3H, s), 3.19-3.10 (1H, m), 2.60-2.50 (4H, m), 2.04-1.98 (2H, m), 1.80-1.73 (2H, m), 1.67-1.60 (2H, m), 1.38-1.29 (2H, m), 1.29-1.23 (2H, m), 0.86 (3H, t, J = 7.4 Hz).

### Example 74: 6-Amino-2-butoxy-9-[3-(4-dimethylaminomethylphenoxy)-propyl]-7,9-dihydropurin-8-one

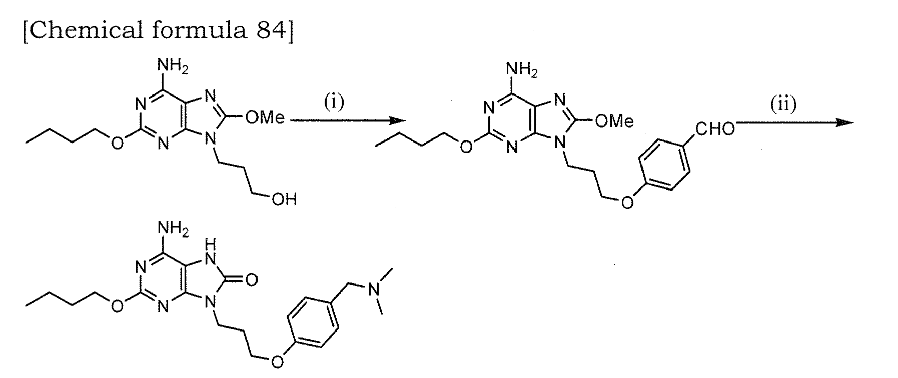

### Step (i): 4-[3-(6-Amino-2-butoxy-8-methoxypurin-9-yl)propoxy]benzaldehyde

2-Butoxy-9-(3-hydroxypropyl)-8-methoxyadenine (0.50 g, 1.69 mmol) was dissolved in THF (10 ml), and the mixture was cooled to 0°C. To the mixture were added 4-hydroxybenzaldehyde (0.22 g, 1.77 mmol) and triphenylphosphine (0.49 g, 1.86 mmol), diethyl azodicarboxylate (2.2M toluene solution, 0.85 ml, 1.86 mmol), and the mixture was stirred for 4 hours. Water was added to the mixture, and the mixture was extracted with ethyl acetate three times. The organic layer was washed with a saturated saline solution three times, dried over magnesium sulfate, concentrated, and the residue was purified by silica gel column (ethyl acetate /hexane = 2 / 1 to 1 / 0) to give the sub-title compound (0.67 g) as a white solid. Yield: 99 %.
¹H NMR (CDCl₃) δ 9.81 (1H, s), 7.75 (2H, d, J = 8.8 Hz), 6.85 (2H, d, J = 8.8 Hz), 5.25 (2H, brs), 4.13-4.06 (4H, m), 3.98 (2H, t, J= 5.8 Hz), 3.93 (3H, s), 2.23-2.18 (2H, m), 1.67-1.56 (2H, m), 1.40-1.32 (2H, m), 0.87 (3H, t, J = 7.4 Hz).

### Step (ii): 6-Amino-2-butoxy-9-[3-(4-dimethylaminomethylphenoxy)-propyl]-7,9-dihydropurin-8-one

The compound obtained in Step (i) (0.18 g, 0.45 mmol) was dissolved in THF (8 ml), and thereto were added at 0°C dimethylamine (2M THF solution, 1.70 ml, 3.40 mmol) and sodium triacetate borohydride (0.21 g, 1.00 mmol), and the mixture was stirred at room temperature for 18 hours. The mixture was cooled to 0°C, and thereto was added a saturated sodium hydrogen carbonate solution, and the mixture was extracted with chloroform twice. The extract was dried over magnesium sulfate, concentrated, and purified by silica gel column (chloroform/methanol/28 % aqueous ammonia = 100/2/0 to 100/3/1). Then, to the residue were added methanol (2 ml) and 4N hydrochloric acid/dioxane (2 ml), and the mixture was stirred at room temperature for one hour. The mixture was cooled to 0°C, and neutralized with 4 %aqueous ammonia. The precipitated solid was collected by filtration and washed with water. The obtained solid was purified by silica gel column (chloroform/methanol/28 % aqueous ammonia = 100/4/0 to 100/4/1) to give the title compound (0.080 g, 0.19 mmol) as a white solid. Yield: 43 %.
¹H NMR (DMSO-d₆) δ 9.85 (1H, brs), 7.13 (2H, d, J = 8.5 Hz), 6.79 (2H, d, J = 8.5 Hz), 6.39 (2H, brs), 4.05 (2H, t, J= 6.6 Hz), 3.96 (2H, t, J= 5.9 Hz), 3.84 (2H, t, J= 6.7 Hz), 3.27 (2H, s), 2.09 (6H, s), 2.09-2.03 (2H, m), 1.60-1.53 (2H, m), 1.38-1.30 (2H, m), 0.89 (3H, t, J = 7.4 Hz).

### Example 75: 6-Amino-2-butoxy-9-(5-dimethylaminomethylfuran-2-yl-methyl)-7, 9-dihydropurin-8-one

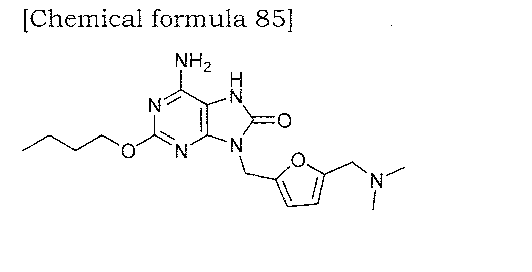

The title compound was obtained in a similar manner to Example 1.
¹H NMR (CDCl₃) δ 10.73 (1H, brs), 6.84 (2H, brs), 6.32 (1H, d, J = 2.7 Hz), 6.11 (1H, d, J = 2.7 Hz), 5.01 (2H, s), 4.28 (2H, t, J = 6.6 Hz), 3.37 (2H, s), 2.29 (6H, s), 1.83-1.73 (2H, m), 1.55-1.44 (2H, m), 0.97 (3H, t, J = 7.3 Hz).

### Example 76: 6-Amino-9-(4-dimethylaminomethylbenzyl)-2-[(pyridin-4-ylmethyl)amino]-7,9-dihydropurin-8-one

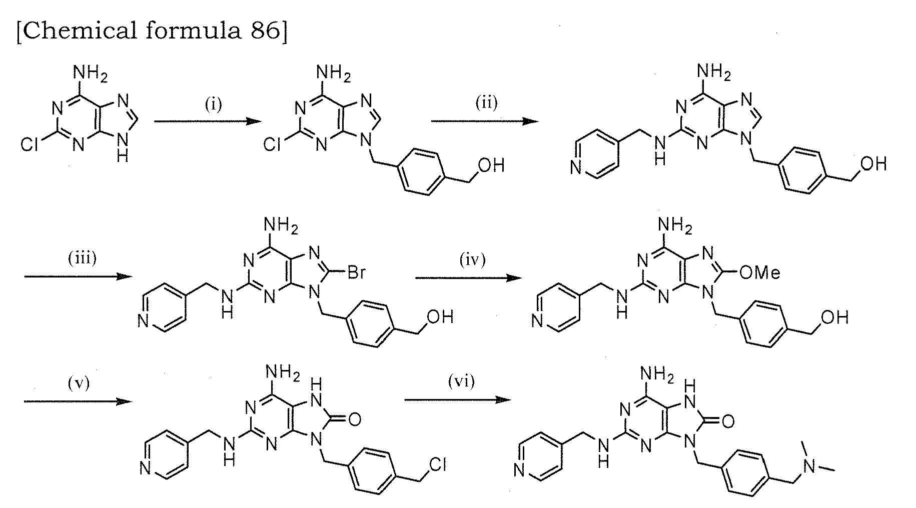

### Step (i): 2-Chloro-9-(4-hydroxymethylbenzyl)adenine

To a solution of 2-chloro-6-aminopurine (1.69 g, 10 mmol) in DMF (50 mL, 0.2M) were added K₂CO₃ (4.15 g, 3.0 eq) and 4-chloromethylbenzyl alcohol (2.34 g, 1.5 eq.), and the mixture was stirred at room temperature for 24 hours. Water (1.0 L) and 10 % MeOH-CHCl₃ (1.0 L x 2) were added thereto, and the mixture was separated. The organic layers were combined, and washed with water and a saturated saline solution (500 mL). The solvent was removed by evaporation, and to the resulting residue were added ethyl acetate (20 mL) and hexane (200 mL), and the mixture was stirred at room temperature for one hour so that the crystallization was completed. The precipitated crystals were collected by filtration, washed with hexane (200 mL), and dried in vacuo at 40° to give the sub-title compound as pale brown crystals (2.32 g). Yield: 80 %.
¹H NMR (DMSO-d₆) δ8.25 (1H, s), 7.80 (2H, brs), 7.29 (2H, d), 7.23 (2H, d), 5.31 (2H, s), 5.18 (1H, t), 4.46 (2H, d).

### Step (ii): 2-(4-Pyridylmethylamino)-9-(4-hydroxymethylbenzyl)adenine

To a solution of the compound obtained in Step (i) (2.90 g, 10 mmol) in NMP (20 mL, 0.5M) were added iPr₂EtN (3.88 g, 3.0 eq) and 4-pyridylmethylamine (5.0 mL, 25 %v/v.), and the mixture was stirred at 180°C for 20 hours under stirring in an autoclave. After confirming the disappearance of the starting compound by LCMS, the mixture was cooled to room temperature, and water (500 mL) and 5 % MeOH-CHCl₃ (1.0 L x 2) were added thereto and separated. The organic layers were combined and washed with a saturated saline solution (500 mL). The solvent was removed by evaporation, and the residue was isolated by column chromatography (SiO₂: the developing solvent: CHCl₃ → 2 % MeOH-CHCl₃ → 5 % MeOH-CHCl₃), and acetone (20 mL) was added to the obtained pale brown amorphous for repulp washing. The obtained crystals were collected by filtration, dried in vacuo to give the sub-title compound (900 mg) as white crystals. Yield: 25 %.
¹H NMR (DMSO-d₆) δ 8.44 (2H, dd), 7.79 (1H, s), 7.30 (2H, dd), 7.14-7.19 (4H, m), 6.97 (1H, t), 6.72 (2H, brs), 5.13 (1H, t), 5.09 (2H, s), 4.48 (2H, d), 4.43 (2H, d).

### Step (iii): 8-Bromo-2-(4-pyridylmethylamino)-9-(4-hydroxymethylbenzyl)adenine

To a solution of the compound obtained in Step (ii) (460 mg, 1.27 mmol) in 10 % MeOH-chloroform (46 mL) was added dropwise a solution of bromine (203 mg, 1.27 mmol, 1.0 eq.) in chloroform (12.7 mL, 0.1 M) at 0°C over a period of 30 minutes. After confirming the disappearance of the starting compound by LCMS, to the mixture were added a saturated sodium hydrogen carbonate solution (100 mL) and a saturated sodium thiosulfate solution (100 mL) so that the reaction was quenched. This reaction solution was extracted by separation into water (100 mL) and a 25 % EtOH-chloroform (500 ml x 2), and the solvent was removed by evaporation. To the resulting pale pink crsytals was added ethyl acetate (5 mL), and the mixture was stirred at room temperature for one hour for repulp washing. The residue was collected by filtration and dried in vacuo (40°C) to give the sub-title compound (548 mg) as pale pink crystals. Yield: 98.0 %.
¹H NMR (DMSO-d₆) δ 8.44 (2H, dd), 7.29 (2H, d), 7.16-7.19 (3H, m), 7.09 (2H, d), 6.97 (2H, brs), 5.18 (1H, t), 5.09 (2H, s), 4.47 (2H, d), 4.43 (2H, d).

### Step (iv): 8-Methoxy-2-(4-pyridylmethylamino)-9-(4-hydroxymethylbenzyl)adenine

To a suspension of the compound obtained in Step (iii) (352 mg, 0.8 mmol) in methanol (200 mL, ca. 5.0 x 10⁻³M) was added potassium methoxide (1.12 g, 20 eq.), and the mixture was heated with stirring in an autoclave at 120° for 12 hours. After confirming the disappearance of the starting compound by LCMS, the mixture was cooled to room temperature, and water (300 mL) and a 25 % EtOH-chloroform (500 mL x 2) were added thereto. The mixture was separated and the solvent was removed by evaporateion. To the residue was added ethyl acetate (5 mL), and the mixture was subjected to crystallization with ultrasonic, and then, the mixture was stirred at room temperature for one hour for repulp washing. The mixture was filtered, and the residue was dried in vacuo (40°C) to give the sub-title compound (250 mg) as white crystals. Yield: 80 %.
¹H NMR (DMSO-d₆) δ 8.44 (2H, dd), 7.29 (2H, d), 7.18 (2H, d), 7.08 (2H, d), 6.87 (1H, t), 6.41 (2H, brs), 5.15 (1H, t), 4.89 (2H, brs), 4.42-4.45 (4H, m), 3.98 (3H, s).

### Step (v): 2-(4-Pyridylmethylamino)-9-(4-chloromethylbenzyl)-8-oxo-adenine

To a suspension of the compound obtained in Step (iv) (200 mg, 0.512 mmol) in CHCl₃ (51 mL, 0.01M) was added SOCl₂ (1.8 mL, 50 eq.), and the mixture was stirred at room temperature for 3 hours. After confirming the disappearance of the starting compound, the solvent was removed by evaporation to give the sub-title compound (205 mg) as white crystals. Yield: 100 %.
¹H NMR (DMSO-d₆) δ 11.1 (1H, brs), 8.80 (2H, d), 8.17-8.55 (2H, brs), 7.97 (2H, s), 7.25 (2H, d), 7.04 (2H, d), 4.80 (2H, s), 4.74 (2H, s), 4.71 (2H, s).

### Step (vi): 6-Amino-9-(4-dimethylaminomethylbenzyl)-2-[(pyridin-4-ylmethyl)amino]-7,9-dihydropurin-8-one

The compound obtained in Step (v) (74 mg, 0.19 mmol) was dissolved in DMF (1 ml), and thereto was added dimethylamine (2.0M THF solution, 3 ml), and the mixture was stirred at room temperature for 3 hours. The mixture was concentrated by an evaporator, and the thereto was added at 0°C 1 % aqueous ammonia (6 ml). The precipitated solid was collected by filtration and purified by silica gel column to give the title compound (20 mg) as a white solid. Yield: 27 %. ¹H NMR (DMSO-d₆) δ 9.65 (1H, brs), 8.42 (2H, d, J = 5.7 Hz), 7.25 (2H, d, J = 5.7 Hz), 7.20-7.10 (4H, m), 6.96 (1H, t, J = 6.2 Hz), 6.07 (2H, brs), 4.73 (2H, s), 4.40 (2H, d, J = 6.2 Hz), 3.31 (2H, s), 2.10 (6H, s).

In a similar manner to Example 1, the compounds of the following Examples 77 to 81 were obtained.

### Example 77: 6-Amino-2-(2-methoxyethoxy)-9-[4-(4-pyridin-4-yl-piperazin-1-ylmethyl)benzyl]-7,9-dihydropurin-8-one (165 mg, 82 %)

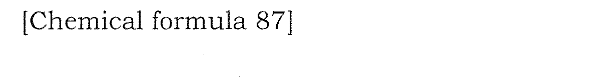

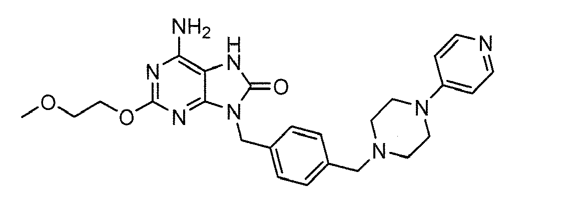

¹H NMR (DMSO-d₆) δ 9.97 (1H, s), 8.14 (2H, d, J = 6.4 Hz), 7.28 (4H, m), 6.78 (2H, d, J = 6.4 Hz), 6.49 (2H, s), 4.85 (2H, s), 4.26 (2H, t, J = 4.8 Hz), 3.59 (2H, t, J = 4.8 Hz), 3.47 (2H, s), 3.28 (7H, m), 2.44 (4H, m).

### Example 78: 6-Amino-9-(4-{[bis-(2-methoxyethyl)amino]methyl}benzyl)-2-(2-methoxyethoxy)-7,9-dihydropurin-8-one (108 mg, 56 %)

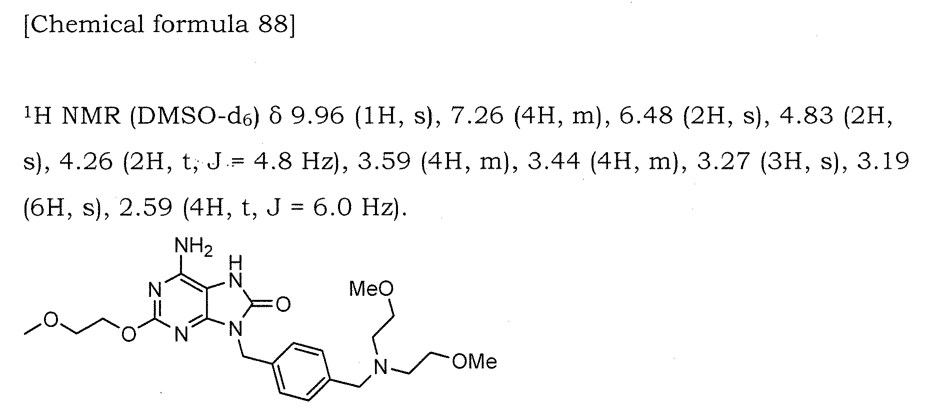

### Example 79: 6-Amino-9-(4-{[bis(2-hydroxyethyl)amino]methyl}benzyl)-2-butoxy-7,9-dihydropurin-8-one (94 mg, 39 %)

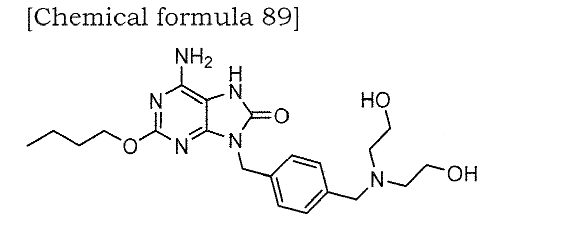

¹H NMR (DMSO-d₆) δ 9.93 (1H, s), 7.27 (2H, d, J = 8.4 Hz), 7.22 (2H, d, J = 8.4 Hz), 6.44 (2H, s), 4.82 (2H, s), 4.32 (2H, t, J = 5.2Hz), 4.14 (2H, t, J = 6.4 Hz), 3.58 (2H, s), 3.42 (4H, m), 1.62 (2H, m), 1.36 (2H, m), 0.90 (3H, t, J = 6.4 Hz).

### Example 80: 6-Amino-2-butoxy-9-(4-{[(2, 3-dihydroxypropyl)methyl-amino]methyl}benzyl)-7,9-dihydropurin-8-one (136 mg, 57 %)

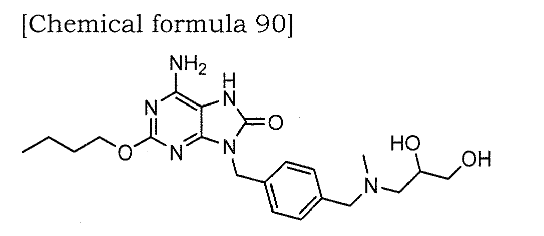

¹H NMR (DMSO-d₆) δ 9.94 (1H, s), 7.24 (4H, m), 6.44 (2H, s), 4.83 (2H, s), 4.47 (1H, m), 4.37 (1H, d, J = 4.0 Hz), 4.13 (2H, t, J = 6.4 Hz), 3.62 (2H, m), 3.44 (2H, q, J = 12.2 Hz), 2.39 (1H, q, J = 5.6 Hz), 2.26 (1H, q, J = 5.6 Hz), 2.10 (3H, s), 1.62 (2H, m), 1.36 (2H, m), 0.90 (3H, t, J = 6.4 Hz).

### Example 81: 6-Amino-2-butoxy-9-(4-{[(2-dimethylaminoethyl)methyl-amino]methyl}benzyl)-7,9-dihydropurin-8₋one (92 mg, 47 %)

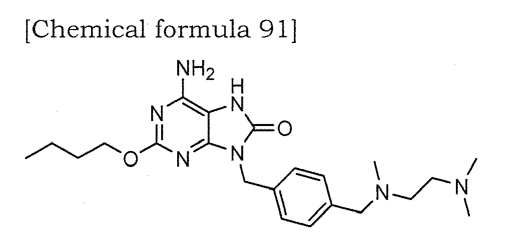

¹H NMR (DMSO-d₆) δ 9.95 (1H, s), 7.22 (4H, m), 6.44 (2H, s), 4.82 (2H, s), 4.13 (2H, t, J = 6.4 Hz), 3.41 (1H, s), 2.30-2.40 (4H, m), 2.09 (3H, s), 2.08 (6H, s), 1.62 (2H, m), 1.35 (2H, m), 0.89 (3H, t, J = 7.2 Hz). Example 82: 6-Amino-9-[6-(2-dimethylaminoethoxy)pyridin-3-ylmethyl]-2-(2-methoxyethoxy)-7,9-dihydropurin-8-one

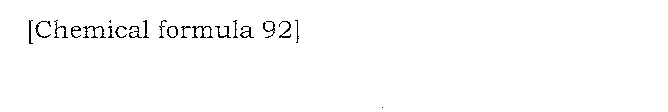

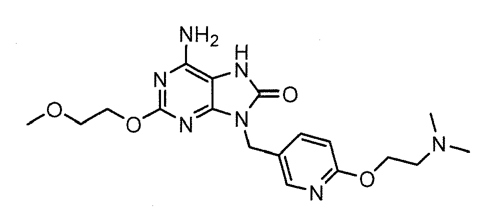

The title compound was obtained in a similar manner to Example 52 (74 mg, 38 %).
¹H NMR (DMSO-d₆) δ 9.96 (1H, s), 8.14 (1H, d, J = 2.4 Hz), 7.64 (1H, dd, J = 2.4, 8.4 Hz), 6.76 (1H, d, J = 8.4 Hz), 6.47 (2H, s), 4.79 (2H, s), 4.28 (4H, m), 3.59 (2H, m), 3.27 (3H, s), 2.56 (2H, t, J = 6.0 Hz), 2.19 (6H, s).
In a similar manner to Example 1, the compounds of the following Examples 83 to 84 were obtained.

### Example 83: 6-Amino-2-butoxy-9-(4-dimethylaminomethylbenzyl)-7,9-dihydropurin-8-one

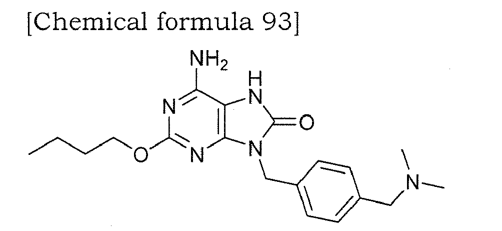

¹H NMR (DMSO-d₆) δ 9.97 (1H, brs), 7.28-7.18 (4H, m), 6.45 (2H, brs), 4.83 (2H, s), 4.14 (2H, t, J = 6.5 Hz), 3.30 (2H, s), 2.10 (6H, s), 1.67-1.55 (2H, m), 1.44-1.28 (2H, m), 0.90 (3H, t, J = 7.3 Hz).

### Example 84: 6-Amino-2-butoxy-9-[4-(3-hydroxyazetidin-1-ylmethyl)-benzyl]-7,9-dihydropurin-8-one

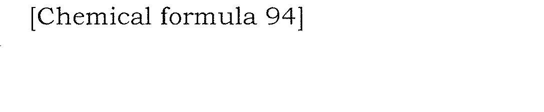

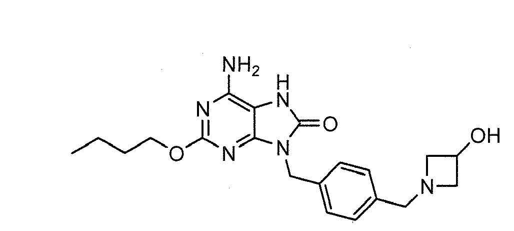

¹H NMR (DMSO-d₆) δ 9.94 (1H, s), 7.22 (4H, m), 6.45 (2H, s), 5.27 (1H, d, J = 6.8 Hz), 4.81 (2H, s), 4.15 (3H, m), 3.49 (2H, s), 3.44 (2H, m), 2.71 (2H, m), 1.62 (2H, m), 1.36 (2H, m), 0.90 (3H, t, J = 7.2 Hz).

### Example 85: Human TLR7 reporter assay

HEK 293 cells, to which human TLR7 or rat TLR7 plasmid and reporter plasmid (NF-kB-SEAP) were stably introduced, were suspended in DMEM medium (10 % FBS, 1 % NEAA, 10 ug/mL blastocidin S HC1, 100 ug/mL Zeocin), and seeded into a 96-well plate in an amount of 90 µl/well (hTLR7/seap-293: 20, 000 cells/well, rTLR7/seap-293: 25, 000 cells/well).
A test compound (DMSO stock solution (2 µl) was diluted into 100-fold with the medium (200 µl)) was added into the cells seeded in the 96-well plate in an amount of 10 µl/well (final concentration: 1nM to 10µM, common ratio: 3). The side of the plate was lightly patted for stirring the content of the plate, and the plate was incubated for 20 hours in a CO₂ incubator. To the cells that were stimulated with a test compound was added a substrate for reporter assay (substrate for SEAP, pNPP) in an amount of 50 µl/well. The minute later after the addition of the substrate, the solution for reaction quenching (4N NaOH) was added to the plate in an amount of 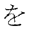 50 µl/well in order to quench the enzyme reaction. A top seal A was applied to the plate, and the absorbance at 405 nm was measured by a microplate reader.
The human TLR7 binding activity (EC₅₀) of each compound is shown in Table 1.

**[Table 1]**

| Compound | EC₅₀ (nM) |
|---|---|
| Example 1 | 7.2 |
| Example 2 | 0.9 |
| Example 3 | 1.8 |
| Example 4 | 2.5 |
| Example 5 | 2.7 |
| Example 13 | 18.7 |
| Example 23 | 14.8 |
| Example 29 | 7.6 |
| Example 37 | 1.1 |
| Example 56 | 1.3 |
| Example 58 | 3.7 |
| Example 68 | 5.5 |

### Example 86:

In a similar manner to Example 1, the compounds as listed in Table 2 may be prepared.

**[Table 2]**

| | | |
|---|---|---|
| 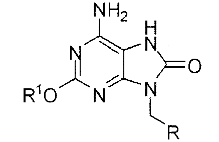 | | |

| Compound No. | R¹- | -R |
|---|---|---|
| Compound 86-1 | 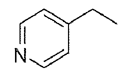 | 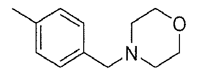 |
| Compound 86-2 | 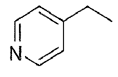 | 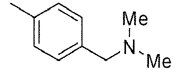 |
| Compound 86-3 | 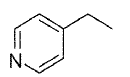 | 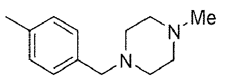 |
| Compound 86-4 | 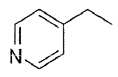 | 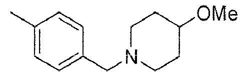 |
| Compound 86-5 | 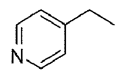 | 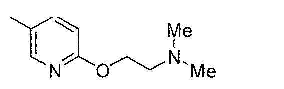 |
| Compound 86-6 | Bu- | 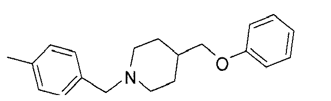 |
| Compound 86-7 | Bu- | 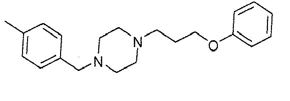 |

### Example 87:

In a similar manner to Example 76, the compounds as listed in Table 3 may be prepared.

**[Table 3]**

| | |
|---|---|
| 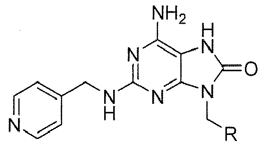 | |

| Compound No. | -R |
|---|---|
| Compound 87-1 | 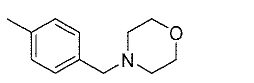 |
| Compound 87-2 | 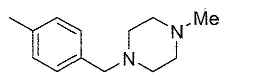 |
| Compound 87-3 | 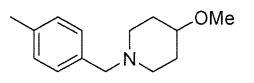 |
| Compound 87-4 | 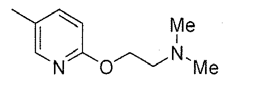 |

### Example 88:

The compounds as listed in Table 4 may be prepared by a method disclosed in the present description.

**[Table 4]**

| | |
|---|---|
| 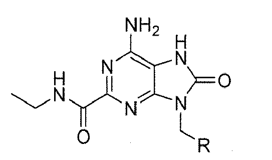 | |
| Compound No. | -R |
| Compound 88-1 | 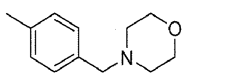 |
| Compound 88-2 | 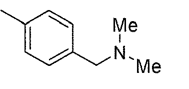 |
| Compound 88-3 | 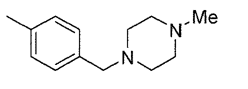 |
| Compound 88-4 | 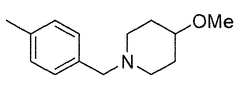 |
| Compound 88-5 | 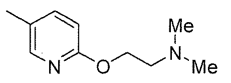 |

### Example 89: 2-Butoxy-7,8-dihydro-9-[(3-[{N-methyl-N-benzyl}amino]-propoxy)benzyl]-8-oxoadenine

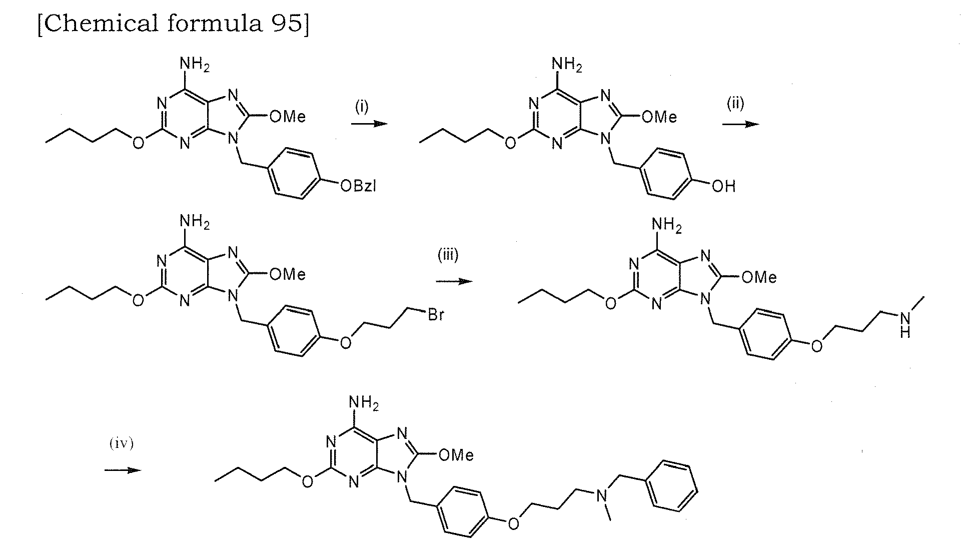

### Step (i): 2-Butoxy-9-(4-hydroxybenzyl)-8-methoxyadenine

9-(4-Benzyloxybenzyl)-2-butoxy-8-methoxyadenine (9.04 g, 20.9 mmol), which was synthesized using 2-butoxyadenine (7.2 g, 34.8 mmol) in a similar manner to Example 1, was dissolved in THF (150 ml), and thereto was added 20 % Pd(OH)₂/C (2.0 g), and the mixture was stirred under hydrogen atmosphere for 9 hours. The mixture was filtered through celite, and the filtrate was concentrated. The precipitated solid was washed with hexane to give the sub-title compound (7.18 g) as a white solid. Yield: 100 %
¹H NMR (DMSO-d₆) δ9.44 (1H, s), 7.09 (2H, d, J = 8.5 Hz), 6.83 (2H, brs), 6.69 (2H, d, J = 8.5 Hz), 4.89 (2H, s), 4.17 (2H, t, J = 6.6 Hz), 4.03 (3H, s), 1.65 (2H, tt, J = 7.5 Hz, 6.6 Hz), 1.40 (2H, tq, J = 7.5 Hz, 7.3 Hz), 0.92 (3H, t, J = 7.3 Hz).

### Step (ii): 9-[4-(3-Bromopropoxy)benzyl]-2-butoxy-8-methoxyadenine

The compound obtained in Step (i) (1.50 g, 4.37 mmol) was dissolved in DMF (50 ml), and thereto were added 1, 3-dibromopropane (4.4 ml, 43.7mmol) and potassium carbonate (0.60 g, 4.37mmol), and the mixture was stirred at 70°C for 6 hours. The solvent was removed by evaporation, and water was added thereto. The mixture was extracted with chloroform and the organic layer was dried over anhydrous magnesium sulfate, and concentrated under reduced pressure. The residue was purified by silica gel column chromatography to give the sub-title compound (0.48 g) as a white solid. Yield: 24 %
¹H NMR (CDCl₃) δ7.29 (2H, d, J = 8.6 Hz), 6.81 (2H, d, J = 8.6 Hz), 5.26 (2H, brs), 5.03 (2H, s), 4.31 (2H, t, J = 6.6 Hz), 4.09 (3H, s), 4.07 (2H, t, J = 5.8 Hz), 3.58 (2H, t, J = 6.4 Hz), 2.29 (2H, tt, J = 6.4 Hz, 5.8 Hz), 1.78 (2H, tt, J = 7.5 Hz, 6.6 Hz), 1.50 (2H, tq, J = 7.5 Hz, 7.4 Hz), 0.97 (3H, t, J = 7.4 Hz).

### Step (iii): 2-Butoxy-8-methoxy-9-[4-(3-methylaminopropoxy)benzyl]-adenine

The compound obtained in Step (ii) (0.15 g, 0.32 mmol) was dissolved in THF (3 ml), and thereto was added a solution of 30 % methylamine/methanol solution (3 ml), and the mixture was stirred at room temperature for 9 hours. The solvent was removed by evaporation, and the residue was purified by silica gel column chromatography to give the sub-title compound (0.13 g) as a white solid. Yield: 100 %
¹H NMR (CDCl₃) δ7.26 (2H, d, J = 8.6 Hz), 6.78 (2H, d, J = 8.6 Hz), 5.51 (2H, brs), 5.00 (2H, s), 4.28 (2H, t, J = 6.8 Hz), 4.09 (3H, s), 4.03 (2H, t, J = 5.8 Hz), 3.20 (2H, t, J = 7.4 Hz), 2.72 (3H, s), 2.37 (2H, tt, J = 7.4 Hz, 5.8 Hz), 1.76 (2H, tt, J = 7.6 Hz, 6.8 Hz), 1.47 (2H, tq, J = 7.6 Hz, 7.4 Hz), 0.96 (3H, t, J = 7.4 Hz).

### Step (iv):

The title compound can be prepared by reacting the compound obtained in Step (iii) with benzyl bromide in acetonitrile in the presence of potassium carbonate.
In addition, the compounds as listed in the following Table 5 can be prepared in a similar manner to the method disclosed in the above Steps (i) to (iv) and Example 52.

**[Table 5]**

| | |
|---|---|
| 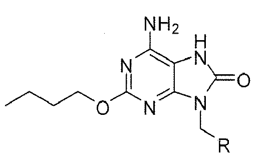 | |

| Compound No. | -R |
|---|---|
| Compound 89-1 | 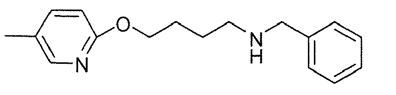 |
| Compound 89-2 | 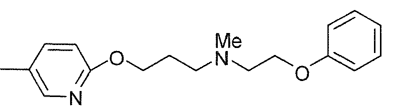 |
| Compound 89-3 | 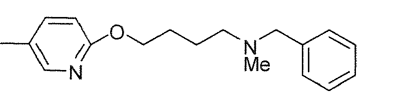 |
| Compound 89-4 | 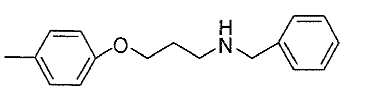 |
| Compound 89-5 | 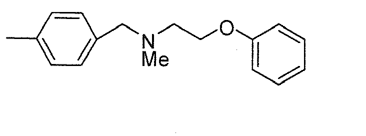 |

### Example 90

### 2-Butoxy-7,8-dihydro-9-(4-{N-(3-hydroxypropyl)-N-benzylaminomethyl}-benzyl)-8-oxoadenine

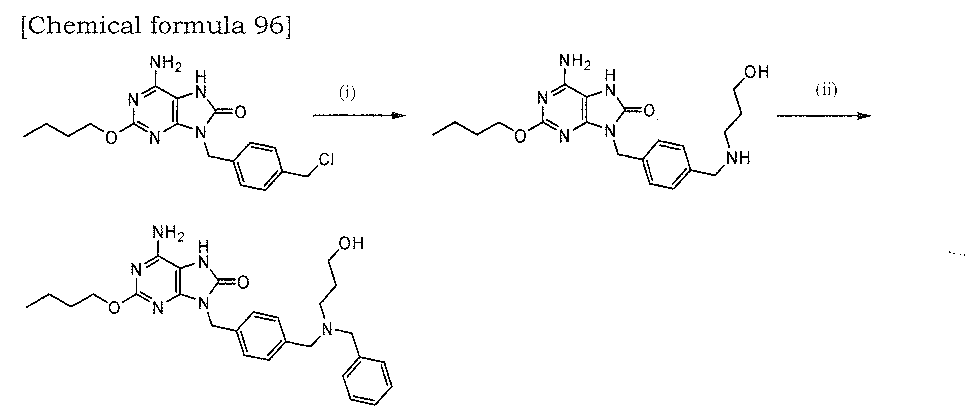

### Step (i): 2-Butoxy-7,8-dihydro-9-{4-[N-(3-hydroxypropyl)aminomethyl]-benzyl}-8-oxoadenine

To 2-butoxy-7,8-dihydro-9-{4-chloromethylbenzyl}-8-oxoadenine (7.2 g, 19.6mmol) was added DMF (140 ml), and thereto was added aminopropanol (15g, 199 mmol), and the mixture was stirred at room temperature for 15 hours. Water (320 ml) was added thereto, and the precipitated solid was collected by filtration and dried to give the sub-title compound (7.8 g, 19.6 mmol) as white yellow solid. Yield: 99 %
¹H NMR (DMSO-d₆)δ 7.25 (2H, bs) (2H, d, J = 8.2 Hz), 7.22 (2H, d, J = 8.2 Hz), 6.57 (2H, brs), 4.81 (2H, s), 4.13 (2H, t, J = 6.6 Hz), 3.61 (2H, s), 3.45 (2H, t, J = 6.3 Hz), 1.66-1.58 (2H, m), 1.58-1.51 (2H, m), 2.52-2.48 (2H, m), 1.42-1.32 (2H, m), 0.90 (3H, t, J = 7.4 Hz).

### Step (ii): 2-Butoxy-7,8-dihydro-9-(4-{N-(3-hydroxypropyl)-N-benzyl-aminomethyl}benzyl)-8-oxoadenine

The title compound can be prepared by reacting the compound obtained in Step (i) with benzyl bromide in DMF in the presence of potassium carbonate.
The compounds 88-2 and 88-3 as listed in the following Table 6 can be obtained in a similar manner to the above Step (i), (ii).

### Example 91

### 2-Butoxy-9-(4-{N-(3-chloropropyl)-N-benzylaminomethyl}benzyl)-7,8-dihydro-8-oxoadenine

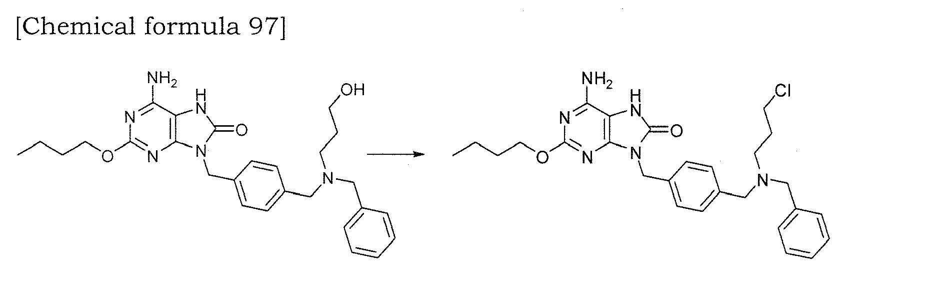

The title compound may be prepared by reacting the compound obtained in Example 90 with thionyl chloride in dichloromethane.

### Example 92

### 2-Butoxy-7,8-dihydro-9-(4-{N-benzyl-N-(3-morpholin-4-ylpropyl)aminomethyl}benzyl)-8-oxoadenine

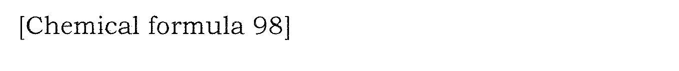

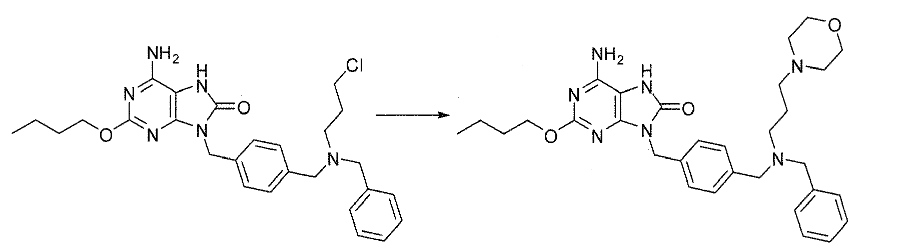

The title compound can be prepared by reacting the compound of Example 91 with morpholine.
In a similar manner to Example 1, the compounds of Examples 93 to 103 were obtained.

### Example 93: 6-Amino-9-(4-{[bis(2-diethylaminoethyl)amino]methyl}-benzyl)-2-butoxy-7,9-dihydropurin-8-one

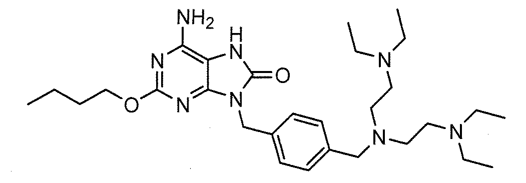

¹H NMR (DMSO-d₆) δ 9.96 (1H, s), 7.25 (4H, m), 6.44 (2H, s), 4.83 (2H, s), 4.14 (2H, t, J = 6.6 Hz), 3.54 (2H, s), 2.35-2.46 (16H, m), 1.62 (2H, m), 1.37 (8H, m), 0.85-0.93 (15H, m).

### Example 94: 6-Amino-2-butoxy-9-{4-[4-(2-dimethylaminoacetyl)-piperazin-1-ylmethyl]benzyl}-7,9-dihydropurin-8-one)

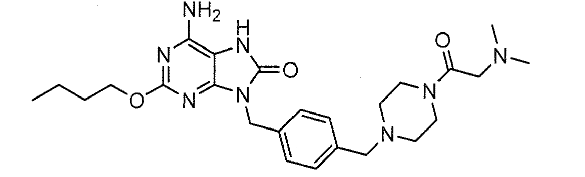

¹H NMR (DMSO-d₆) δ 9.95 (1H, s), 7.25 (4H, m), 6.45 (2H, s), 4.84 (2H, s), 4.14 (2H, t, J = 6.6 Hz), 3.49 (2H, m), 3.41 (4H, m), 3.02 (2H, s), 2.33 (4H, m), 2.26 (2H, m), 2.15 (6H, s), 1.63 (2H, m), 1.37 (2H, m), 0.91 (3H, t, J = 6.4 Hz).

### Example 95: 2-{4-[4-(6-Amino-2-butoxy-8-oxo-7,8-dihydropurin-9-yl-methyl)benzyl]piperazin-1-yl}-N, N-dimethylacetamide

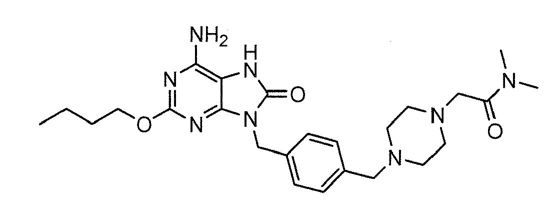

¹H NMR (DMSO-d₆) δ 9.95 (1H, s), 7.23 (4H, m), 6.45 (2H, s), 4.83 (2H, s), 4.14 (2H, t, J = 6.6 Hz), 3.39 (2H, s), 3.08 (2H, s), 2.99 (3H, s), 2.78 (3H, s), 2.33 (8H, m), 1.63 (2H, m), 1.37 (2H, m), 0.90 (3H, t, J = 6.4 Hz).

### Example 96: 6-Amino-2-(2-methoxyethoxy)-9-[4-(4-methoxypiperidin-1-ylmethyl)benzyl]-7,9-dihydropurin-8-one

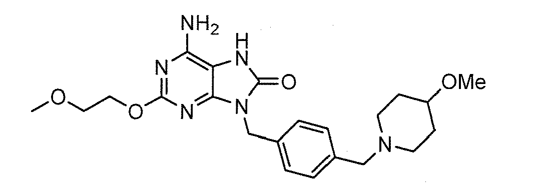

¹H NMR (DMSO-d₆) δ 9.96 (1H, s), 7.23 (4H, m), 6.47 (2H, s), 4.83 (2H, s), 4.26 (2H, t, J = 4.8 Hz), 3.59 (2H, t, J = 4.8 Hz), 3.39 (2H, s), 3.27 (3H, s), 3.20 (3H, s), 3.14 (1H, m), 2.59 (2H, m), 2.03 (2H, m), 1.78 (2H, m), 1.38 (2H, m).

### Example 97: 6-Amino-9-{4-[(butylmethylamino)methyl]benzyl}-2-(2-methoxyethoxy)-7,9-dihydropurin-8-one

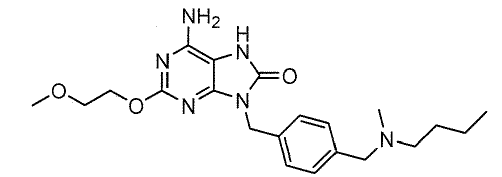

¹H NMR (DMSO-d₆) δ 9.96 (1H, s), 7.23 (4H, m), 6.47 (2H, s), 4.83 (2H, s), 4.26 (2H, t, J = 4.8 Hz), 3.58 (2H, t, J = 4.8 Hz), 3.38 (2H, s), 3.27 (3H, s), 2.27 (2H, t, J = 7.2 Hz), 2.06 (3H, s), 1.41 (2H, m), 1.26 (2H, m), 0.84 (3H, t, J = 7.2 Hz).

### Example 98: 4-({4-[6-Amino-2-(2-methoxyethoxy)-8-oxo-7,8-dihydropurin-9-ylmethyl]benzyl}methylammo)butyronitrile

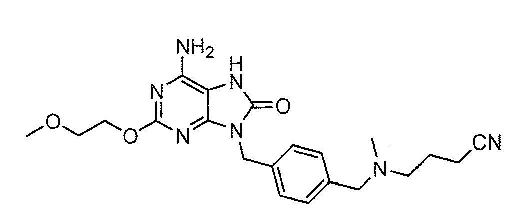

¹H NMR (DMSO-d₆) δ 9.96 (1H, s), 7.26 (4H, m), 6.47 (2H, s), 4.84 (2H, s), 4.26 (2H, t, J = 4.8 Hz), 3.59 (2H, t, J = 4.8 Hz), 3.49 (2H, s), 3.27 (3H, s), 2.68 (2H, t, J = 6.2 Hz), 2.58 (2H, t, J = 6.2 Hz), 2.13 (3H, m).

### Example 99: N-(1-{4-[6-Amino-2-(2-methoxyethoxy)-8-oxo-7,8-dihydropurin-9-ylmethyl]benzyl}pyrrolidin-3-yl)-N-methylacetamide

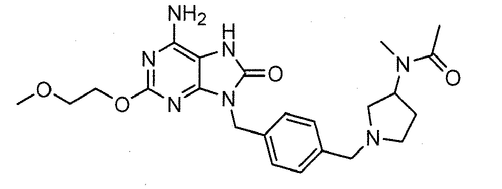

¹H NMR (DMSO-d₆) δ 9.85 (1H, s), 7.23 (4H, m), 6.48 (2H, s), 5.04 (0.5H, m), 4.84 (2H, s), 4.44 (0.5H, m), 4.26 (2H, t, J = 4.8 Hz), 3.44-3.59 (4H, m), 3.26 (3H, s), 2.90 (2H, s), 2.73 (2H, m), 2.25 (2H, m), 2.08 (2H, s), 2.00 (2H, s), 1.92 (2H, s), 1.60 (1H, m).

### Example 100: 6-Amino-9-(4-{[ethyl(tetrahydropyran-4-yl)amino]methyl}-benzyl)-2-(2-methoxyethoxy)-7,9-dihydropurin-8-one

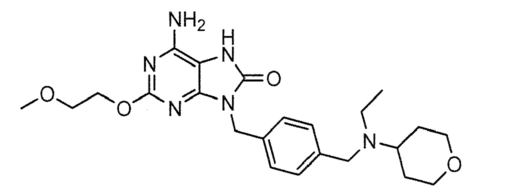

¹H NMR (DMSO-d₆) δ 9.96 (1H, s), 7.27 (2H, d, J = 8.0 Hz), 7.22 (2H, d, J = 8.0Hz), 6.48 (2H, s), 4.82 (2H, s), 4.26 (2H, t, J = 4.6 Hz), 3.85 (2H, dd, J = 4.0, 10.8 Hz), 3.59 (2H, t, J = 4.6 Hz), 3.55 (2H, s), 3.27 (3H, s), 3.22 (2H, m), 2.67 (1H, m), 2.47 (2H, m), 1.61 (2H, m), 1.45-1.49 (2H, m), 0.92 (3H, t, J = 6.8 Hz).

### Example 101: 6-Amino-9-[4-(4,4-difluoropiperidin-1-ylmethyl)benzyl]-2-(2-methoxyethoxy)-7,9-dihydropurin-8-one

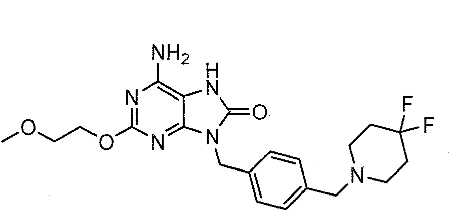

¹H NMR (DMSO-d₆) δ 10.01 (1H, s), 7.26 (4H, s), 6.51 (2H, s), 4.84 (2H, s), 4.26 (2H, t, J = 4.8 Hz), 3.58 (2H, t, J = 4.8 Hz), 3.49 (2H, s), 3.27 (3H, s), 2.33 (4H, m), 1.92 (4H, m).

### Example 102: 6-Amino-9-[4-(4-cyclopentylpiperazin-1-ylmethyl)benzyl]-2-(2-methoxyethoxy)-7,9-dihydropurin-8-one

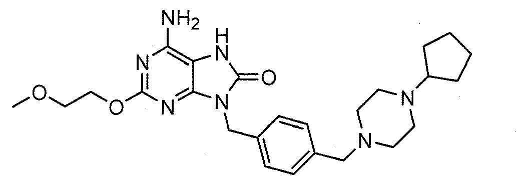

¹H NMR (DMSO-d₆) δ 9.97 (1H, s), 7.23 (4H, m), 6.49 (2H, s), 4.83 (2H, s), 4.26 (2H, t, J = 4.8 Hz), 3.58 (2H, t, J = 4.8 Hz), 3.38 (2H, s), 3.27 (3H, s), 2.36 (9H, m), 1.72 (2H, m), 1.57 (2H, m), 1.45-1.48 (4H, m), 1.26 (2H, m).

### Example 103: 6-Amino-9-(4-{[isopropyl(2-methoxyethyl)amino]methyl}-benzyl)-2-(2-methoxyethoxy)-7,9-dihydropurin-8-one

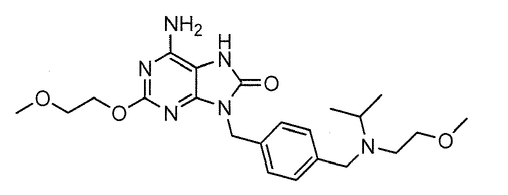

¹H NMR (DMSO-d₆) δ 9.96 (1H, s), 7.27 (2H, d, J = 8.0 Hz), 7.22 (2H, d, J = 8.0Hz), 6.48 (2H, s), 4.82 (2H, s), 4.26 (2H, t, J = 4.8 Hz), 3.59 (2H, t, J = 4.8 Hz), 3.52 (2H, s), 3.35 (2H, s), 3.27 (5H, m), 3.15 (3H, s), 2.82 (1H,m), 0.93 (6H, m).

### Example 104: 6-Amino-2-butoxy-9-{6-[(2-dimethylaminoethyl)methylamino]pyridin-3-ylmethyl}-7,9-dihydropurin-8-one

The following compound was synthesized in a similar manner to Example 40. 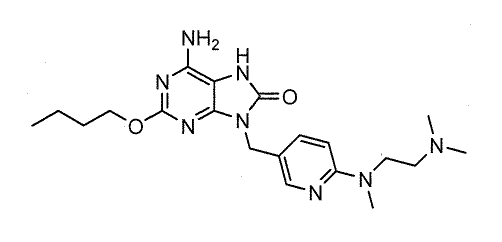 ¹H NMR (DMSO-d₆) δ 9.89 (1H, s), 8.07 (1H, d, J = 2.2 Hz), 7.47 (1H, dd, J = 2.4, 8.8 Hz), 6.52 (1H, d, J = 8.8 Hz), 6.42 (2H, s), 4.70 (2H, s), 4.17 (2H, t, J = 7.2 Hz), 3.56 (2H, t, J = 7.2 Hz), 2.95 (3H, s), 2.34 (2H, t, J = 6.6 Hz), 2.15 (3H, s), 1.65 (2H, m), 1.39 (2H, m), 0.93 (3H, t, J = 7.2 Hz).

### Example 105: 6-Amino-9-[5-chloro-6-(4-methylpiperazin-1-yl)pyridin-3-ylmethyl]-2-(2-methoxyethoxy)-7,9-dihydropurin-8-one

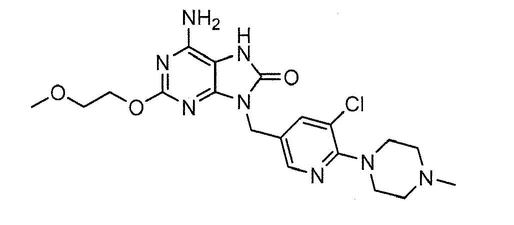

The title compound was obtained in a similar manner to Example 56.
¹H NMR (DMSO-d₆) δ 9.97 (1H, brs), 8.19 (1H, d, J = 2.0 Hz), 7.73 (1H, d, J = 2.2 Hz), 6.49 (2H, s), 4.81 (2H, s), 4.29 (2H, t, J = 4.8 Hz), 3.60 (2H, t, J = 4.8 Hz), 3.28 (3H, s), 3.32 (4H, m), 2.44 (4H, t, J = 4.4 Hz), 2.21 (3H, s).

### Example 106: 6-Amino-9-[5-chloro-6-(4-methyl-[1,4)diazepan-1-yl)-pyridin-3-ylmethyl]-2-(2-methoxyethoxy)-7,9-dihydropurin-8-one

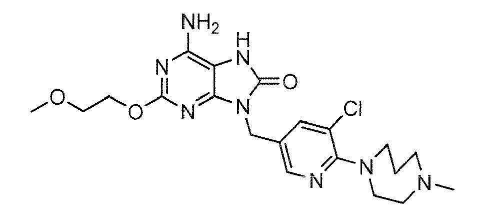

The title compound was obtained in a similar manner to Example 56.
¹H NMR (DMSO-d₆) δ 9.80 (1H, brs), 8.09 (1H, d, J = 2.0 Hz), 7.64 (1H, d, J = 2.2 Hz), 6.49 (2H, s), 4.77 (2H, s), 4.29 (2H, t, J = 4.8 Hz), 3.54-3.62 (6H, m), 3.29 (3H, s), 2.65 (2H, m), 2.25 (3H, s), 1.87 (2H, m).
The compounds of Examples 107 to 117 were synthesized in a similar manner to Example 52.

### Example 107: 6-Amino-2-butoxy-9-(6-{2-[(2-hydroxyethyl)methylamino]ethoxy}pyridin-3-ylmethyl)-7,9-dihydropurin-8-one

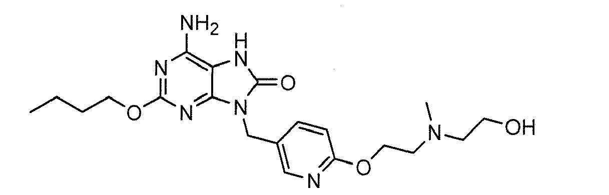

¹H NMR (DMSO-d₆) δ 9.95 (1H, brs), 8.14 (1H, d, J = 2.2 Hz), 7.65 (1H, dd, J = 2.4, 8.4 Hz), 6.76 (1H, d, J = 8.4 Hz), 6.45 (2H, s), 4.80 (2H, s), 4.29 (3H, m), 4.16 (2H, t, J = 6.6 Hz), 3.44 (2H, q, J = 6.0 Hz), 2.71 (2H, q, J = 6.0 Hz), 2.47 (2H, t, J = 6.6 Hz), 2.24 (3H, m), 1.64 (2H, m), 1.38 (2H, m), 1.18 (3H, s), 0.92 (3H, t, J = 7.2 Hz). 0.78 (2H, d, J = 7.4 Hz).

### Example 108: 6-Amino-2-butoxy-9-[6-(2-dimethylamino-1-dimethyl-aminomethylethoxy)pyridin-3-ylmethyl]-7,9-dihydropurin-8-one

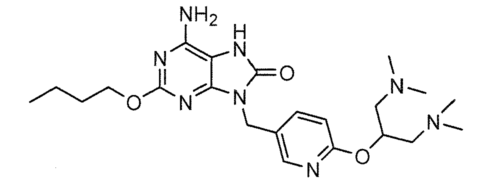

¹H NMR (DMSO-d₆) δ 9.95 (1H, brs), 8.12 (1H, d, J = 2.2 Hz), 7.64 (1H, dd, J = 2.4, 8.4 Hz), 6.70 (1H, d, J = 8.4 Hz), 6.45 (2H, s), 5.43 (1H, m), 4.80 (2H, s), 4.16 (2H, t, J = 6.6 Hz), 2.45 (4H, m), 2.16 (12H, s), 1.64 (2H, m), 1.38 (2H, m), 0.92 (3H, t, J = 7.2 Hz).

### Example 109: 6-Amino-2-(2-methoxyethoxy)-9-[6-(2-piperidin-1-ylethoxy)pyridin-3-ylmethyl]-7,9-dihydropurin-8-one

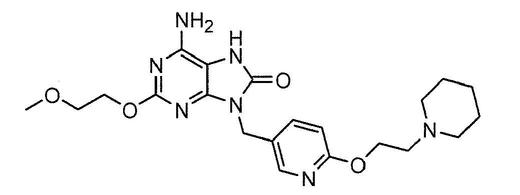

¹H NMR (DMSO-d₆) δ 9.96 (1H, brs), 8.14 (1H, d, J = 2.2 Hz), 7.65 (1H, dd, J = 2.4, 8.4 Hz), 6.77 (1H, d, J = 8.4 Hz), 6.47 (2H, s), 4.90 (2H, s), 4.25-4.32 (4H, m), 3.60 (2H, t, J = 4.8 Hz), 3.28 (3H, s), 2.59 (2H, t, J = 6.0 Hz), 2.33 (4H, m), 1.46 (4H, m), 1.36 (2H, m).

### Example 110: 6-Amino-9-[6-(3-dimethylamino-2,2-dirnethylpropoxy)-pyridin-3-ylmethyl]-2-(2-methoxyethoxy)-7,9-dihydropurin-8-one)

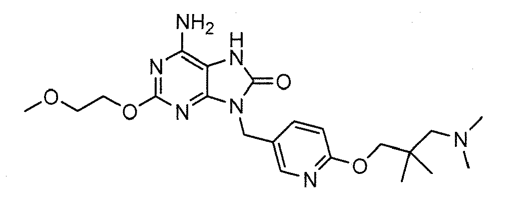

¹H NMR (DMSO-d₆) δ 9.97 (1H, s), 8.14 (1H, d, J = 2.2 Hz), 7.64 (1H, dd, J = 2.4, 8.4 Hz), 6.78 (2H, d, J = 8.4 Hz), 6.49 (2H, s), 4.80 (2H, s), 4.27 (2H, t, J = 4.8 Hz), 3.95 (2H, s), 3.60 (2H, q, J = 4.8 Hz), 3.28 (3H, s), 2.23 (2H, s), 2.18 (6H, s), 0.91 (6H, s).

### Example 111: 6-Amino-2-(2-methoxyethoxy)-9-[6-(1-methylpiperidin-3-ylmethoxy)pyridin-3-ylmethyl]-7,9-dihydropurin-8-one

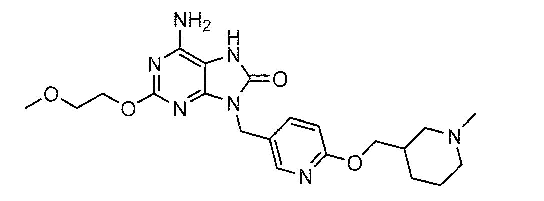

¹H NMR (DMSO-d₆) δ 9.97 (1H, brs), 8.14 (1H, d, J = 2.2 Hz), 7.65 (1H, dd, J = 2.4, 8.4 Hz), 6.77 (1H, d, J = 8.4 Hz), 6.49 (2H, s), 4.80 (2H, s), 4.27 (2H, t, J = 4.8 Hz), 4.13 (1H, m), 4.03 (1H, dd, J = 7.6, 10.8 Hz), 3.60 (2H, t, J = 4.8 Hz), 3.28 (3H, s), 2.74 (1H, d, J = 10.8 Hz), 2.57 (1H, d, J = 10.8 Hz), 2.12 (3H, s), 1.90 (1H, m), 1.85 (1H, m), 1.59-1.72 (3H, m), 1.44 (1H, m), 0.98 (1H, m).

### Example 112: 6-Amino-2-(2-methoxyethoxy)-9-[6-(1-methylpiperidin-4-yloxy)pyridin-3-ylmethyl]-7,9-dihydropurin-8-one

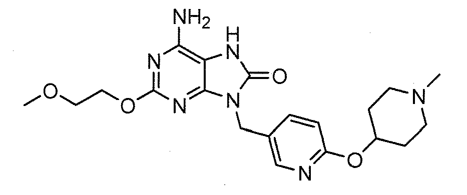

¹H NMR (DMSO-d₆) δ 9.96 (1H, brs), 8.13 (1H, d, J = 2.2 Hz), 7.64 (1H, dd, J = 2.4, 8.4 Hz), 6.74 (1H, d, J = 8.4 Hz), 6.48 (2H, s), 4.93 (1H, m), 4.79 (2H, s), 4.28 (2H, t, J = 6.6 Hz), 3.60 (2H, t, J = 6.6 Hz), 3.28 (3H, s), 2.60 (2H, m), 2.12 (5H, m), 1.92 (2H, m), 1.60 (2H, m).

### Example 113: 6-Amino-9-[6-(2-dimethylaminoethoxy)pyridin-3-yl-methyl]-2-ethoxy-7,9-dihydropurin-8-one

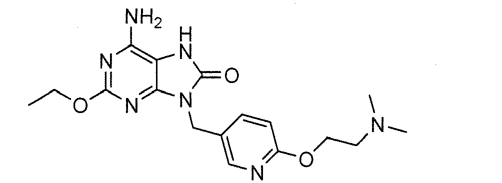

¹H NMR (DMSO-d₆) δ 9.96 (1H, brs), 8.14 (1H, d, J = 2.2 Hz), 7.65 (1H, dd, J = 2.4, 8.4 Hz), 6.77 (1H, d, J = 8.4 Hz), 6.46 (2H, s), 4.80 (2H, s), 4.29 (2H, t, J = 6.0 Hz), 4.20 (2H, q, J = 6.6 Hz), 2.68 (2H, t, J = 6.0 Hz), 2.17 (6H, s), 1.26 (3H, t, J = 6.6 Hz).

### Example 114: 6-Amino-2-ethoxy-9-{6-[2-(4-methylpiperazin-1-yl)-ethoxy]pyridin-3-ylmethyl}-7,9-dihydropurin-8-one

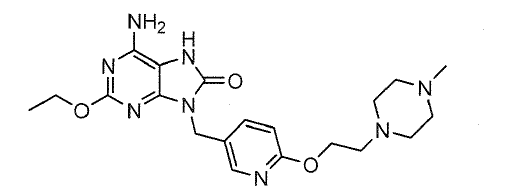

¹H NMR (DMSO-d₆) δ 9.95 (1H, brs), 8.14 (1H, d, J = 2.2 Hz), 7.65 (1H, dd, J = 2.4, 8.4 Hz), 6.77 (1H, d, J = 8.4 Hz), 6.46 (2H, s), 4.80 (2H, s), 4.37 (2H, t, J = 5.0 Hz), 4.20 (2H, q, J = 7.0 Hz), 2.62 (2H, t, J = 6.0 Hz), 2.49 (4H, m), 2.33 (4H, m), 2.12 (3H, s), 1.26 (3H, t, J = 6.8 Hz).

### Example 115: 6-Amino-2-ethoxy-9-{6-[3-(4-methylpiperazin-1-yl)-propoxy]pyridin-3-ylmethyl}-7,9-dihydropurin-8-one

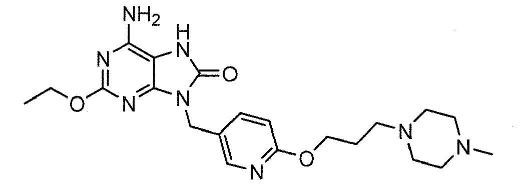

¹H NMR (DMSO-d₆) δ 9.96 (1H, brs), 8.14 (1H, d, J = 2.2 Hz), 7.65 (1H, dd, J = 2.4, 8.4 Hz), 6.76 (1H, d, J = 8.4 Hz), 6.46 (2H, s), 4.79 (2H, s), 4.18-4.23 (4H, m), 2.28-2.38 (10H, m), 2.13 (3H, s), 1.26 (3H, t, J = 6.8 Hz).

### Example 116: 6-Amino-2-butylamino-9-[6-(3-dimethylaminopropoxy)-pyridin-3-ylmethyl]-7,9-dihydropurin-8-one

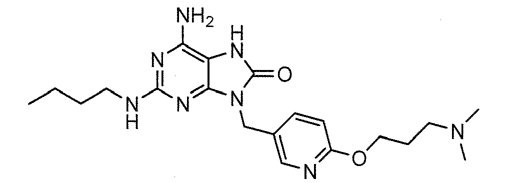

¹H NMR (DMSO-d₆) δ 9.63 (1H, brs), 8.13 (1H, d, J = 2.2 Hz), 7.65 (1H, dd, J = 2.4, 8.4 Hz), 6.75 (1H, d, J = 8.4 Hz), 6.23 (1H, t, J = 5.6 Hz), 6.00 (2H, s), 4.74 (2H, s), 4.22 (2H, t, J = 6.6 Hz), 3.17 (2H, q, J = 6.6 Hz), 2.30 (2H, t, J = 6.8 Hz), 2.11 (6H, s), 1.81 (2H, m), 1.44 (2H, m), 1.29 (2H, m), 0.88 (3H, t, J = 6.8 Hz).

### Example 117: 6-Amino-2-(2-methoxyethoxy)-9-[6-(1-methylpiperidin-4-ylmethoxy)pyridin-3-ylmethyl]-7,9-dihydropurin-8-one

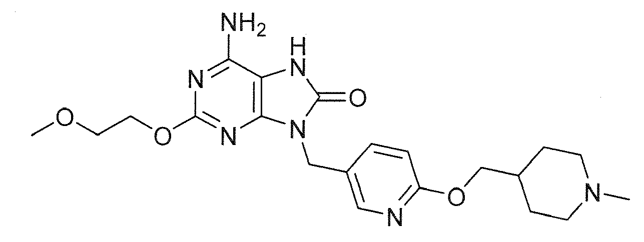

¹H NMR (DMSO-d₆) δ 10.04 (1H, brs), 8.13 (1H, d, J = 2.3 Hz), 7.64 (1H, dd, J = 8.5 Hz, 2.3 Hz), 6.75 (1H, d, J = 8.5 Hz), 6.51 (2H, brs), 4.79 (2H, s), 4.27 (2H, t, J = 4.6 Hz), 4.05 (2H, d, J = 6.1 Hz), 3.59 (2H, t, J = 4.6 Hz), 3.27 (3H, s), 2.74 (2H, d, J = 11.1 Hz), 2.13 (3H, s), 1.86-1.78 (2H, m), 1.68-1.64 (3H, m), 1.30-1.20 (2H, m).
The compounds of Examples 118 to 123 were synthesized in a similar manner to Example 58.

### Example 118: 6-Amino-9-[5-chloro-6-(2-dimethylaminoethoxy)pyridin-3-ylmethyl]-2-(2-methoxyethoxy)-7,9-dihydropurin-8-one

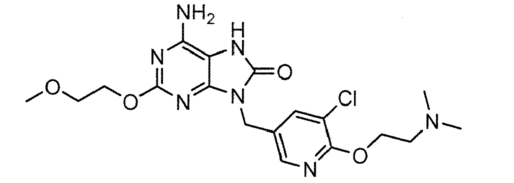

¹H NMR (DMSO-d₆) δ 9.98 (1H, s), 8.10 (1H, d, J = 1.8 Hz), 7.84 (1H, d, J = 1.8 Hz), 6.49 (2H, s), 4.83 (2H, s), 4.40 (2H, t, J = 5.8 Hz), 4.28 (2H, m), 3.60 (2H, q, J = 5.0 Hz), 3.28 (3H, s), 2.62 (2H, t, J = 6.0 Hz), 2.20 (6H, s).

### Example 119: 6-Amino-9-[5-chloro-6-(3-dimethylaminopropoxy)pyridin-3-ylmethyl]-2-(2-methoxyethoxy)-7,9-dihydropurin-8-one

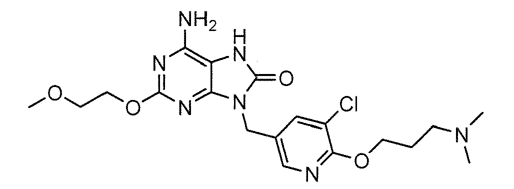

¹H NMR (DMSO-d₆) δ 9.99 (1H, s), 8.10 (1H, d, J = 1.8 Hz), 7.83 (1H, d, J = 1.8 Hz), 6.51 (2H, s), 4.83 (2H, s), 4.27-4.35 (4H, m), 3.60 (2H, q, J = 4.8 Hz), 3.28 (3H, s), 2.31 (2H, t, J = 6.4 Hz), 2.12 (6H, s), 1.84 (2H, m).

### Example 120: 6-Amino-9-[5-chloro-6-(3-dimethylamino-2,2-dimethylpropoxy)pyridin-3-ylmethyl]-2-(2-methoxyethoxy)-7,9-dihydropurin-8-one

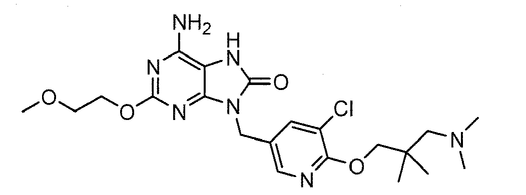

¹H NMR (DMSO-d₆) δ 9.99 (1H, s), 8.10 (1H, d, J = 1.8 Hz), 7.83 (1H, d, J = 1.8 Hz), 6.51 (2H, s), 4.83 (2H, s), 4.28 (2H, t, J = 4.8 Hz), 4.00 (2H, s), 3.60 (2H, q, J = 4.8 Hz), 3.28 (3H, s), 2.23 (2H, s), 2.19 (6H, s), 0.92 (6H, s).

### Example 121: 6-Amino-9-[5-chloro-6-(2-pyrrolidin-1-ylethoxy)pyridin-3-ylmethyl]-2-(2-methoxyethoxy)-7,9-dihydropurin-8-one

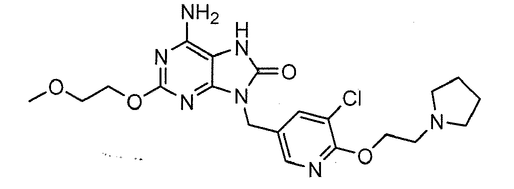

¹H NMR (DMSO-d₆) δ 10.00 (1H, s), 8.10 (1H, d, J = 1.8 Hz), 7.84 (1H, d, J = 1.8 Hz), 6.51 (2H, s), 4.83 (2H, s), 4.41 (2H, t, J = 5.6 Hz), 4.28 (2H, t, J = 4.4 Hz), 3.60 (2H, q, J = 4.8 Hz), 3.28 (3H, s), 2.77 (2H, t, J = 6.0 Hz), 1.65 (4H, m).

### Example 122: 6-Amino-9-{5-chloro-6-[3-(4-methylpiperazin-1-yl)-propoxy]pyridin-3-ylmethyl}-2-(2-methoxyethoxy)-7,9-dihydropurin-8-one

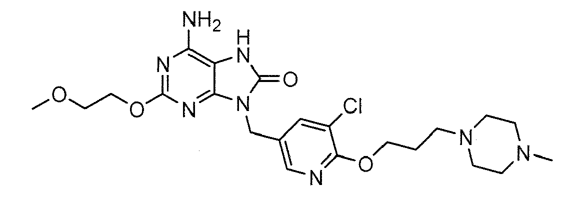

¹H NMR (DMSO-d₆) δ 9.99 (1H, s), 8.10 (1H, d, J = 1.8 Hz), 7.83 (1H, d, J = 1.8 Hz), 6.50 (2H, s), 4.82 (2H, s), 4.26-4.46 (4H, m), 3.59 (2H, q, J = 4.8 Hz), 3.27 (3H, s), 2.35 (10H, m), 2.10 (3H, s), 1.84 (2H, m).

### Example 123: 6-Amino-9-[5-chloro-6-(1-methylpiperidin-4-yloxy)-pyridin-3-ylmethyl]-2-(2-methoxyethoxy)-7,9-dihydropurin-8-one

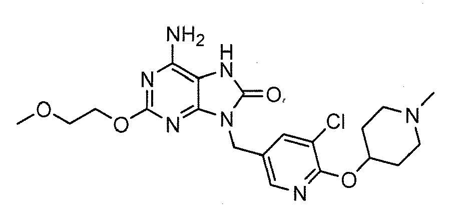

¹H NMR (DMSO-d₆) δ 10.00 (1H, brs), 8.21 (1H, d, J = 2.0 Hz), 7.83 (1H, d, J = 2.0 Hz), 6.51 (2H, s), 5.05 (1H, m), 4.82 (2H, s), 4.28 (2H, t, J = 4.8 Hz), 3.60 (2H, t, J = 4.8 Hz), 3.28 (3H, s), 2.67 (2H, m), 2.16 (5H, m), 1.92 (2H, m), 1.68 (2H, m).

### Example 124: 6-amino-2-(2-methoxyethoxy)-9-[6-(3-morpholin-4-yl-propyl)pyridin-3-ylmethyl]-7,9-dihydropurin-8-one

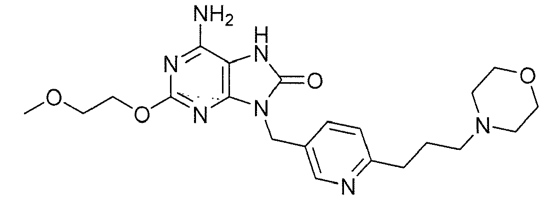

### Step (i) : Methanesulfonic acid 6-bromopyridin-3-ylmethyl ester

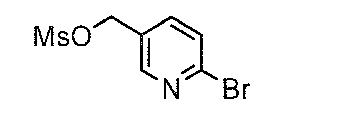

6-Bromopyridin-3-yl)methanol (2.58 g, 13.7 mmol) was dissolved in THF (25 mL) and cooled to 0°C. The solution was added sequentially with diisopropylmethylamine (2.36 mL, 13.7 mmol) and methanesulfonyl chloride (1.06 mL, 13.7 mmol), stirred at 0°C for 1 hour. The mixture was concentrated by evaporator, and thereto added water and extracted with chloroform, and then dried over magnesium sulphate and concentrated to give the sub-title compound as pale orange oil (3.64 g, 13.7 mmol, quantitative).
¹H NMR (CDCl₃) δ 8.44 (1H, d, J = 2.4 Hz), 7.66 (1H, dd, J = 8.2 Hz, 2.4 Hz), 7.57 (1H, d, J = 8.2 Hz), 5.24 (2H, s), 3.06 (3H, s).

### Step (ii): 9-(6-Bromopyridin-3-ylmethyl)-8-methoxy-2-(2-methoxyethoxy)-9H-purin-6-ylamine

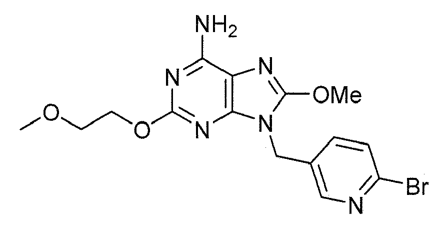

8-Methoxy-2-(2-methoxyethoxy)-9H-purin-6-ylamine trifluoroacetic acid salt (3.36g, 10.0 mmol) and methanesulphonic acid 6-bromo pyridin-3-ylmethyl ester (3.36 g, 13.7 mmol) obtained in Step (i) was dissolved in DMF (40 ml) and added with potassium carbonate (2.76 g, 20.0 mmol). The solution was stirred at room temperature for 15 hours. The carbonate within the reaction system was removed by filtration, and the filtrate was concentrated. To the residue was dissolved in DMF (15 ml), added with water (45 ml) and cooled to 0°C. The precipitated solid was collected by filtration, and purified by silica gel column chromatography (chloroform / methanol = 100/0 to 50/1) to give the sub-title compound (0.97 g, 2.38 mmol) as a pale yellow solid. Yield: 24 %.
¹H NMR (CDCl₃) δ 8.35 (1H, d, J = 2.4 Hz), 7.50 (1H, dd, J = 8.2 Hz, 2.4 Hz), 7.35 (1H, d, J = 8.2 Hz), 5.20 (2H, brs), 4.99 (2H, s), 4.39 (2H, t, J = 5.0 Hz), 4.03 (3H, s), 3.68 (2H, t, J = 5.0 Hz), 3.36 (3H, s).

### Step (iii) : 9-[6-(2-[1,3]Dioxan-2-ylethyl)pyridin-3-ylmethyl]-8-methoxy-2-(2-methoxyethoxy)-9H-purin-6-ylamine

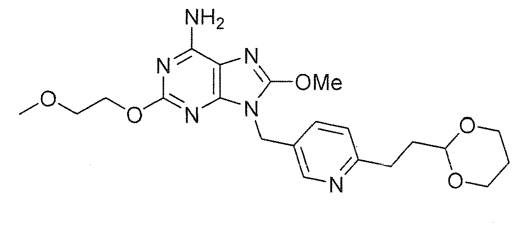

The compound obtained in Step (ii) (0.56 g, 1.37 mmol) was dissolved in THF (12 ml), and thereto added tetrakis(triphenylphosphine)palladium (40 mg, 0.034 mmol) and (1, 3-dioxane-2-yl ethyl) zinc bromide (0.5 M in THF, 19.2 ml, 9.6 mmol). The mixture was stirred at room temperature for 15 hours. 1M hydrochloric acid was added to neutralize, and the solvent was removed by an evaporator. To the residue was added with a saturated saline solution, extracted with chloroform, dried over magnesium sulphate and concentrated under vacuum. The residue was purified by silica gel column chromatography (chloroform / methanol = 100/1 to 25/ 1) to give the sub-title compound (0.57 g, 1.29 mmol) as a pale yellow solid to give. Yield: 94 %.
¹H NMR (DMSO-d₆)δ 8.44 (1H, d, J = 2.2 Hz), 7.55 (1H, dd, J = 8.0 Hz, 2.2 Hz), 7.21 (1H, d, J = 8.0 Hz), 6.90 (2H, brs), 5.03 (2H, s), 4.29 (2H, t, J = 4.8 Hz), 4.05 (3H, s), 4.00-3.94 (2H, m), 3.70-3.62 (2H, m), 3.60 (2H, t, J = 4.8 Hz), 3.28 (3H, s), 2.75-2.69 (2H, m), 1.86-1.80 (2H, m), 1.33-1.29 (1H, m).

### Step (iv) : 6-amino-2-(2-methoxyethoxy)-9-[6-(3-morpholin-4-yl-propyl)pyridin-3-ylmethyl]-7,9-dihydropurin-8-one

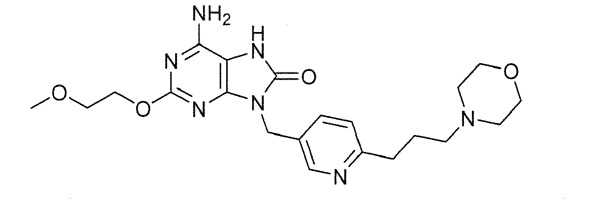

The compound obtained in Step (iii) (0.21 g, 0.46 mmol) was dissolved in THF (5 ml) and cooled to 0°C. 6 M hydrochloric acid (5 ml) was added, and the mixture was stirred at room temperature for 5 hours. The mixture was cooled to 0°C, neutralized with 28 % ammonia in water, and extracted with chloroform/ethanol =3/1. The organic layer was washed with saturated saline solution, dried over magnesium sulphate and concentrated under vacuum. The residue was added with chloroform (5 ml) and cooled to 0°C. The mixture was then added with sodium triacetate borohydride (98 mg, 0.46 mmol) and morpholine (0.040 mL, 0.46 mmol), and stirred at room temperature for 10 minutes. The residue mixture was added with saturated aqueous sodium hydrogen carbonate, extracted with chloroform / ethanol = 3/1, dried over magnesium sulphate and concentrated under vacuum. The precipitated solid was recrystallized from acetonitrile / methanol = 2/1 to give the title compound (63 mg, 0.14 mmol) as a pale yellow solid. Yield: 31 %.
¹H NMR (DMSO-d₆)δ 9.98 (1H, s), 8.45 (1H, d, J = 2.0 Hz), 7.60 (1H, dd, J = 8.0 Hz, 2.0 Hz), 7.21 (1H, d, J = 8.0 Hz), 6.49 (2H, brs), 4.84 (2H, s), 4.26 (2H, t, J = 4.6 Hz), 3.58 (2H, t, J = 4.6 Hz), 3.58-3.50 (4H, m), 3.27 (3H, s), 2.69 (2H, t, J = 7.6 Hz), 2.32-2.00 (6H, m), 1.82-1.74 (2H, m).

### Example 125: 6-amino-2-(2-methoxyethoxy)-9-[6-(3-dimethylaminopropyl)pyridin-3-ylmethyl]-7,9-dihydropurin-8-one

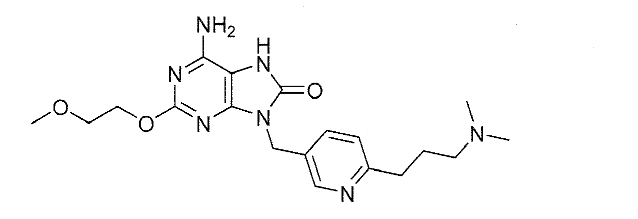

Using the compound obtained in Example 124, Step (iii) (0.17 g, 0.38 mmol), the title compound was obtained in a similar manner to Example 124, Step (iv). White solid (73mg, 48 %).
¹H NMR (DMSO-d₆)δ 10.01 (1H, s), 8.46 (1H, s), 7.60 (1H, d, J = 7.6 Hz), 7.20 (1H, d, J = 7.6 Hz), 6.50 (2H, brs), 4.85 (2H, s), 4.26 (2H, t, J = 4.6 Hz), 3.58 (2H, t, J = 4.6 Hz), 3.27 (3H, s), 2.67 (2H, t, J = 7.5 Hz), 2.18 (2H, t, J = 7.1 Hz), 2.08 (6H, s), 1.80-1.68 (2H, m).

### Example 126:

In a similar manner to Example 85, the following compounds were tested. The human TLR7 binding activity (EC50) of each compound is shown in Table 6.

**[Table 6]**

| Compound | EC50 (nM) |
|---|---|
| Example 94 | 2.5 |
| Example 104 | 5.3 |
| Example 105 | 3.3 |
| Example 110 | 16.5 |
| Example 116 | 19.6 |
| Example 118 | 14.0 |
| Example 120 | 3.7 |
| Example 123 | 6.9 |

### Example 127:

### IFN-a production inducing activity

A test compound was administered to B6C3F1 mice (Charles River Japan, Inc.) via tail vein at the dose of 1 mg/kg intravenously. After 3 hours, total blood was collected into a sample tube containing heparin. Plasma was prepared from total blood after centrifugation (3, 000 rpm, 10 min, at room temperature) and stored at -20°C.

Murine fibroblasts (L929/ 2-5AS-Luc), which constitutively expressed luciferase gene in which the promoter region for 2'-5'-oligoadenylate synthase was cloned, was seeded into 96-well plate at 4×10⁴ cells/well, and cultured for overnight with diluted plasma or mouse IFN-a. After Luclite (Perkin Elmer) was added to the plate, the luciferase activity was measured with TopCount (Perkin Elmer).

The concentration of IFN-α in plasma was caluculated by linear regression of logarithmic transformed concentration of mouse IFN-a and the luciferase activity. The results are shown in Table 7. The compounds tested showed IFN-α inducing activity.

**[Table 7]**

| IFN-α production inducing activity | | |
|---|---|---|
| Compound | Dose | Mouse IFN-α (IU/mL) |
| Example 54 | 1mg/kg | 53.6 ± 6.5^{a}) |
| Example 42 | 1mg/kg | 30.0 ± 7.3 |
| Example 10 | 1mg/kg | 7.3 ± 1.6 |
| Example 63 | 1mg/kg | 25.4 ± 13.9 |
| vehicle | 10mL/kg | 0.2 ± 0.0 |

| | | |
|---|---|---|
| a) mean± standard deviation (n=3) | | |

### Example 128:

### Antitumor activity

OV-HM ovarian carcinoma-bearing B6C3F1 mouse was prepared by intradermally inoculating 1×10⁶ viable OV-HM cells in back skin. Tumor was surgically removed ten days after the tumor incubation under isoflurane anesthesia. On Day 11, Day 14, Day 17, Day 20, Day 24, Day 28, and Day 32, a test compound was administered via tail vein at the dose of 1 mg/kg. On Day 35, mouse was euthanized, and lung was collected from the mouse. Metastasized tumor nodules in lung was counted, and the frequency of the metastasized mice was calculated in each group. The results are shown in Table 8. The compounds tested significantly inhibited the metastasis into lung, showing antitumor activity.

**Table 8: Antitumor activity**

| Compound | Dose | Frequency of metastasis | Number of tumor |
|---|---|---|---|
| Example 63 | 1mg/kg | 0/6 | 0.0** |
| vehicle | 10mL/kg | 5/5 | >64.5 |

| | | | |
|---|---|---|---|
| ** p<0.01 vs vehicle group (Wilcoxon) | | | |

### Example 129: 6-Amino-2-(2-methoxyethoxy)-9-[6-(1-methylpiperidin-2-ylmethoxy)pyridin-4-ylmethyl]-7,9-dihydropurin-8-one

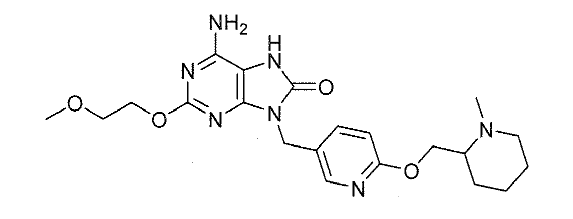

¹H NMR (DMSO-d₆) δ 9.97 (1H, brs), 8.14 (1H, d, J = 2.3 Hz), 7.65 (1H, dd, J = 8.5 Hz, 2.3 Hz), 6.77 (1H, d, J = 8.5 Hz), 6.49 (2H, brs), 4.80 (2H, s), 4.26-4.32 (3H, m), 4.17 (1H, m), 3.60 (2H, t, J = 4.8 Hz), 3.28 (3H, s), 2.73 (1H, d, J = 11.2 Hz), 2.19 (3H, s), 2.13-2.16 (1H, m), 1.99 (1H, m), 1.68 (2H, m), 1.19-1.50 (4H, m).

### Example 130: 6-Amino-2-(2-methoxyethoxy)-9-[6-(1-methylpyrrolidin-2-ylmethoxy)pyridin-3-ylmethyl]-7,9-dihydropurin-8-one

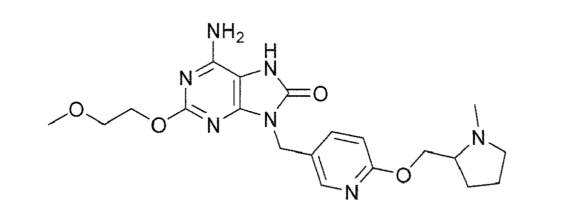

¹H NMR (DMSO-d₆) δ 9.96 (1H, brs), 8.14 (1H, d, J = 2.3 Hz), 7.64 (1H, dd, J = 8.5 Hz, 2.3 Hz), 6.77 (1H, d, J = 8.5 Hz), 6.49 (2H, brs), 4.80 (2H, s), 4.20-4.26 (3H, m), 4.08 (1H, m), 3.60 (2H, t, J = 4.6 Hz), 3.27 (3H, s), 2.93 (1H, m), 2.31 (3H, s), 2.15 (1H, m), 1.85 (1H, m), 1.54-1.66 (3H, m).

### Example 131: 6-Amino-9-[6-(1-ethylpiperidin-3-yloxy)pyridin-3-ylmethyl]-2-(2-methoxyethoxy)-7,9-dihydropurin-8-one

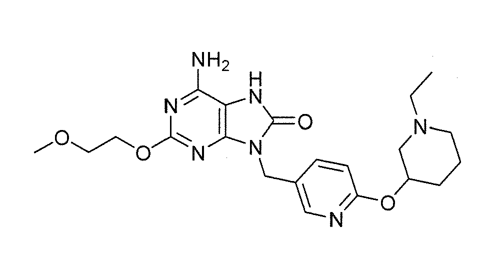

¹H NMR (DMSO-d₆) δ 9.97 (1H, brs), 8.14 (1H, d, J = 2.3 Hz), 7.65 (1H, dd, J = 8.5 Hz, 2.3 Hz), 6.75 (1H, d, J = 8.5 Hz), 6.49 (2H, brs), 4.98 (1H, m), 4.79 (2H, s), 4.27 (2H, t, J = 4.6 Hz), 3.59 (2H, t, J = 4.6 Hz), 3.27 (3H, s), 2.94 (1H, d, J = 8.4 Hz), 2.61 (1H, m), 2.33 (2H, m), 1.93-2.01 (3H, m), 1.68 (1H, m), 1.33 (1H, m), 1.49 (1H, m), 0.96 (3H, t, J = 7.2 Hz).

### Example 132: 6-Amino-9-[6-(1-isopropylpyrrolidin-3-yloxy)pyridin-3-ylmethyl]-2-(2-methoxyethoxy)-7,9-dihydropurin-8-one

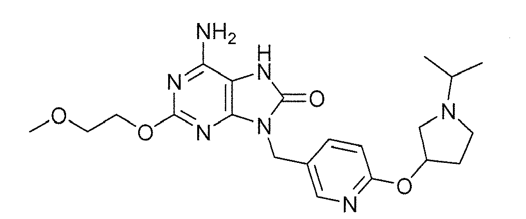

¹H NMR (DMSO-d₆) δ 9.97 (1H, brs), 8.13 (1H, d, J = 2.3 Hz), 7.64 (1H, dd, J = 8.5 Hz, 2.3 Hz), 6.75 (1H, d, J = 8.5 Hz), 6.50 (2H, brs), 5.29 (1H, m), 4.79 (2H, s), 4.28 (2H, t, J = 4.8 Hz), 3.60 (2H, t, J = 4.8 Hz), 3.28 (3H, s), 2.60-2.85 (3H, m), 2.10-2.40 (3H, ms), 1.73 (1H, m), 0.99 (6H, m).

### Example 133: 6-Amino-2-butoxy-9-{6-[2-(4-methylpiperazin-1-yl)ethoxy]pyridin-3-ylmethyl}-7,9-dihydropurin-8-one

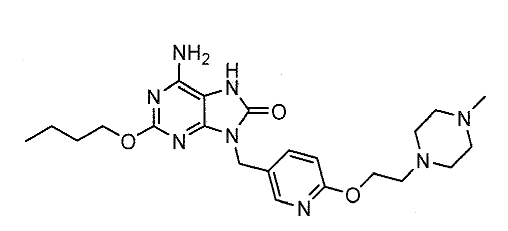

¹H NMR (DMSO-d₆) δ 9.95 (1H, brs), 8.14 (1H, d, J = 2.2 Hz), 7.64 (1H, dd, J = 2.4, 8.4 Hz), 6.77 (1H, d, J = 8.4 Hz), 6.46 (2H, s), 4.80 (2H, s), 4.31 (2H, t, J = 6.0 Hz), 4.15 (2H, t, J = 6.6 Hz), 2.63 (2H, t, J = 5.8 Hz), 2.43 (4H, m), 2.32 (4H, m), 2.11 (3H, s), 1.63 (2H, m), 1.38 (2H, m), 0.92 (3H, t, J = 7.0 Hz).

### Example 134 : 6-Amino-2-butoxy-9-{6-[3-(4-methylpiperazin-1-yl)propoxy]pyridin-3-ylmethyl}-7,9-dihydropurin-8-one

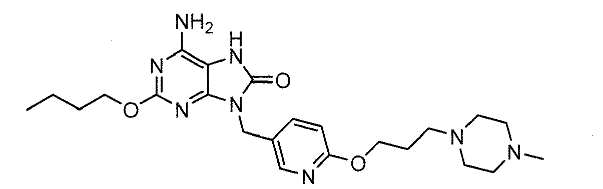

¹H NMR (DMSO-d₆) δ 9.95 (1H, s), 8.14 (1H, d, J = 2.2 Hz), 7.64 (1H, dd, J = 2.4, 8.4 Hz), 6.75 (1H, d, J = 8.4 Hz), 6.46 (2H, s), 4.80 (2H, s), 4.22 (2H, t, J = 6.6 Hz), 4.15 (2H, t, J = 6.6 Hz), 2.35 (10H, m), 2.13 (3H, s), 1.82 (2H, m), 1.63 (2H, m), 1.38 (2H, m), 0.92 (3H, t, J = 7.0 Hz).

### Example 135 : 6-Amino-2-(2-methoxyethoxy)-9-[6-(1-propylpiperidin-4-yloxy)pyridin-3-ylmethyl]-7,9-dihydropurin-8-one

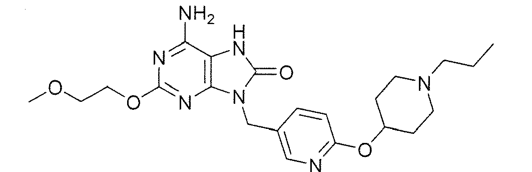

¹H NMR (DMSO-d₆) δ 9.96 (1H, brs), 8.12 (1H, d, J = 2.2 Hz), 7.63 (1H, dd, J = 8.5, 2.2 Hz), 6.73 (1H, d, J = 8.5 Hz), 6.48 (2H, s), 4.97-4.89 (1H, m), 4.78 (2H, s), 4.27 (2H, t, J = 4.7 Hz), 3.59 (2H, t, J = 4.7 Hz), 3.27 (3H, s), 2.70-2.60 (2H, m), 2.22 (2H, t, J = 7.2 Hz), 2.16-2.08 (2H, m), 1.95-1.89 (2H, m), 1.65-1.55 (2H, m), 1.46-1.36 (2H, m), 0.83 (3H, t, J = 7.4 Hz).

### Example 136 : 6-Amino-9-[6-(1-isopropylpiperidin-4-yloxy)pyridin-3-ylmethyl]-2-(2-methoxyethoxy)-7,9-dihydropurin-8-one

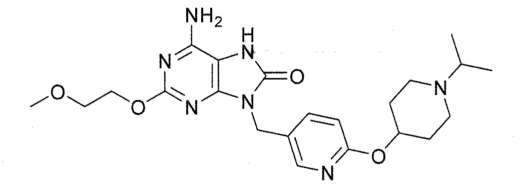

¹H NMR (DMSO-d₆) δ 9.96 (1H, brs), 8.12 (1H, d, J = 2.3 Hz), 7.63 (1H, dd, J = 8.5, 2.3 Hz), 6.73 (1H, d, J = 8.5 Hz), 6.48 (2H, s), 4.95-4.86 (1H, m), 4.78 (2H, s), 4.27 (2H, t, J = 4.7 Hz), 3.59 (2H, t, J = 4.7 Hz), 3.27 (3H, s), 2.70-2.60 (3H, m), 2.34-2.25 (2H, m), 1.96-1.89 (2H, m), 1.62-1.53 (2H, m), 0.95 (6H, d, J = 6.5 Hz).

## Claims

1. An adenine compound of the formula (1): 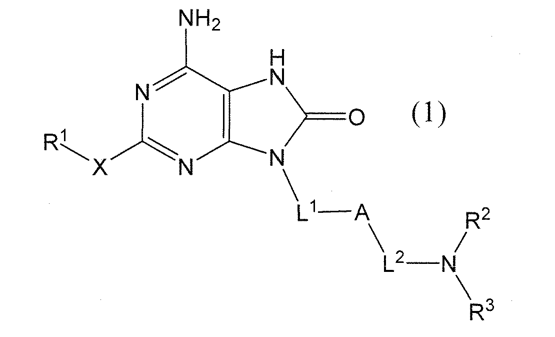 wherein
A is substituted or unsubstituted aromatic carbocycle, or substituted or unsubsituted aromatic heterocycle;
L¹ is a single bond, or straight chain or branched chain alkylene;
L² is a single bond, or straight chain or branched chain alkylene optionally substituted with hydroxy, amino, alkylamino or dialkylamino;
in the case that L² is a single bond, -NR²R³ is not unsubstituted amino, unsubstituted alkylamino, unsubstituted dialkylamino, unsubstituted pyrrolidinyl, unsubstituted piperidinno or unsubstituted morpholino;
any one to three of methylene group(s) in the alkylene in L² may be replaced by oxygen, sulfur, SO, SO₂, carbonyl, NR⁴CO, CONR⁴, NR⁴SO₂, SO₂NR⁴, NR⁴CO₂, OCONR⁴, NR⁵CONR⁴, NR⁶C(=NR⁴)NR⁵, C(=NR⁴)NR⁵ wherein R⁴, R⁵ and R⁶ are independently hydrogen or alkyl;
and one to three of methylene group(s) in the alkylene in L¹ may be replaced by oxygen;
R¹ is halogen, substituted or unsubstituted alkyl, substituted or unsubstituted alkenyl, substituted or unsubstituted alkynyl, substituted or unsubstituted cycloalkyl, substituted or unsubstituted aryl, or substituted or unsubstituted heteroaryl;
R² and R³ are independently hydrogen, substituted or unsubstituted alkyl, substituted or unsubstituted alkenyl, substituted or unsubstituted alkynyl, substituted or unsubstituted cycloalkyl, substituted or unsubstituted saturated heterocycle, substituted or unsubstituted aryl, or substituted or unsubstituted heteroaryl, or R² may be combined together with L² or R³ to form a substituted or unsubstituted 4- to 8-membered nitrogen-containing saturated heterocycle; and
X is oxygen, sulfur, SO, SO₂, NR⁷, NR⁷CO wherein R⁷ is hydrogen or alkyl, or a single bond; provided that when R¹ is halogen, then X is a single bond,
or a pharmaceutically acceptable salt thereof.

2. The adenine compound according to claim 1, or a pharmaceutically acceptable salt thereof, wherein L² in formula (1) is a single bond, or straight chain or branched chain alkylene, in which any one to three of methylene group(s) in said alkylene may be replaced by oxygen, sulfur, SO, SO₂, carbonyl, NR⁴CO, CONR⁴, NR⁴SO₂, SO₂NR⁴, NR⁴CO₂, OCONR⁴, NR⁵CONR⁴, NR⁶C(=NR⁴)NR⁵, C(=NR⁴)NR⁵ wherein R⁴, R⁵ and R⁶ are independently hydrogen or alkyl.

3. The adenine compound according to claim 1 or 2 wherein A in the formula (1) is substituted or unsubstituted benzene ring, or substituted or unsubstituted 5- to 6-membered monocyclic aromatic heterocycle;
in case that A is substituted, it is substituted with one or more group(s) independently selected from the group consisting of: halogen, hydroxy, nitro, alkyl with 1 to 6 carbon atom(s), haloalkyl with 1 to 6 carbon atom(s), alkoxy with 1 to 6 carbon atom(s), alkylthio with 1 to 6 carbon atom(s), haloalkoxy with 1 to 6 carbon atom(s), alkylcarbonyl with 2 to 6 arbon atom(s), alkylsulfonyl with 1 to 6 carbon atom(s), alkylsulfinyl with 1 to 6 carbon atom(s), and amino optionally substituted with the same or different one or two alkyl(s) with 1 to 6 carbon atom(s),
or a pharmaceutically acceptable salt thereof.

4. The adenine compound according to claim 3, wherein the 5- to 6-membered monocyclic aromatic heterocycle is pyridine, furan or thiophene, or a pharmaceutically acceptable salt thereof.

5. The adenine compound according to any one of claims 1 to 4 or a pharmaceutically acceptable salt thereof, wherein in case that alkyl, alkenyl or alkynyl in R¹ is substituted, each group may be substituted with one or more substituent(s) selected from the following (a) to (c):
(a) halogen, hydroxy, carboxy, mercapto, haloalkyl with 1 to 6 carbon atom(s) and haloalkoxy with 1 to 6 carbon atom(s);
(b) alkoxy with 1 to 6 carbon atom(s), alkylcarbonyl with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), alkylcarbonyloxy with 2 to 6 carbon atoms, and alkylthio with 1 to 6 carbon atom(s), wherein each group may further be substituted with one or more group(s) selected independently from halogen, hydroxy, carboxy, alkoxy with 1 to 6 carbon atom(s), alkoxycarbonyl with 2 to 6 carbon atoms, amino optionally substituted with the same or different one or two alkyl(s) with 1 to 6 carbon atom(s), carbamoyl optionally substituted with the same or different one or two alkyl(s) with 1 to 6 carbon atom(s), sulfamoyl optionally substituted with the same or different one or two alkyl(s) with 1 to 6 carbon atom(s), or alkylsulfonyl with 1 to 6 carbon atom(s);
(c) substituted or unsubstituted 3- to 8-membered cycloalkyl and substituted or unsubstituted 4- to 8-membered saturated heterocycle, wherein each group may further be substituted with one or more group(s) selected from the following (d), (e) and (f); substituted or unsubstituted 6- to 10-membered aryl, substituted or unsubstituted 5-to 10-membered heteroaryl, substituted or unsubstituted 6- to 10-membered aryloxy and substituted or unsubstituted 5- to 10-membered heteroaryloxy, wherein each group may further be substituted with one or more group(s) selected from the following (g), (h), (i) and (j); and substituted or unsubstituted amino, substituted or unsubstituted carbamoyl and substituted or unsubstituted sulfamoyl, wherein each group may further be substituted with one or two group(s) selected from the following (k), (l) and (m);
in case that cycloalkyl in R¹ is substituted, each group may be substituted with one or more group(s) selected from the following (d), (e) and (f):
(d) halogen, hydroxy, carboxy, mercapto, oxo, cyano, nitro, haloalkyl with 1 to 6 carbon atom(s), and haloalkoxy with 1 to 6 carbon atom(s);
(e) alkyl with 1 to 6 carbon atom(s), alkoxy with 1 to 6 carbon atom(s), alkenyl with 2 to 6 carbon atoms, alkynyl with 2 to 6 carbon atoms, alkoxycarbonyl with 2 to 6 carbon atoms, and alkylthio with 1 to 6 carbon atom(s), wherein each group may further be substituted with one or more group(s) selected independently from halogen, hydroxy, carboxy, alkoxy with 1 to 6 carbon atom(s), alkoxycarbonyl with 2 to 6 carbon atoms, amino optionally substituted with the same or different one or two alkyl(s) with 1 to 6 carbon atom(s), carbamoyl optionally substituted with the same or different one or two alkyl(s) with 1 to 6 carbon atom(s), sulfamoyl optionally substituted with the same or different one or two alkyl(s) with 1 to 6 carbon atom(s), and alkylsulfonyl with 1 to 6 carbon atom(s);
(f) substituted or unsubstituted 6- to 10-membered aryl and substituted or unsubstituted 5- to 10-membered heteroaryl, wherein each group may further be substituted with one or more group(s) selected from the following (g), (h), (i) and (j); substituted or unsubstituted amino, substituted or unsubstituted carbamoyl and substituted or unsubstituted sulfamoyl, wherein each group may further be substituted with one or two group(s) selected from the following (k), (1) and (m);
in case that aryl and heteroaryl in R¹ is substituted, each group may be substituted with one or more group(s) selected from the following (g), (h), (i) and (j):
(g) halogen, hydroxy, mercapto, cyano, nitro, haloalkyl with 1 to 6 carbon atom(s), and haloalkoxy with 1 to 6 carbon atom(s);
(h) alkyl with 1 to 6 carbon atom(s), alkoxy with 1 to 6 carbon atom(s), alkenyl with 2 to 6 carbon atoms, alkynyl with 2 to 6 carbon atoms and alkylthio with 1 to 6 carbon atom(s), wherein each group may further be substituted with one or more group(s) selected independently from halogen, hydroxy, carboxy, alkoxy with 1 to 6 carbon atom(s), alkoxycarbonyl with 2 to 6 carbon atoms, amino optionally substituted with the same or different one or two alkyl(s) with 1 to 6 carbon atom(s), carbamoyl optionally substituted with the same or different one or two alkyl(s) with 1 to 6 carbon atom(s), sulfamoyl optionally substituted with the same or different one or two alkyl(s) with 1 to 6 carbon atom(s), and alkylsulfonyl with 1 to 6 carbon atom(s);
(i) 3- to 8-membered cycloalkyl and 4- to 8-membered saturated heterocycle, wherein each group may further be substituted with one or more group(s) selected independently from halogen, hydroxy, carboxy, alkyl with 1 to 6 carbon atom(s) and alkoxy with 1 to 6 carbon atom(s);
(j) substituted or unsubstituted amino, substituted or unsubstituted carbamoyl and substituted or unsubstituted sulfamoyl, wherein each group may further be substituted with one or two group(s) selected from the following (k), (l) and (m);
in case that amino, carbamoyl and sulfamoyl in (c), (f) and (j) is substituted, each group may be substituted with one or two group(s) selected independently from the following (k), (l) and (m):
(k) alkyl with 1 to 6 carbon atom(s), alkenyl with 2 to 6 carbon atoms, alkynyl with 2 to 6 carbon atoms, alkylcarbonyl with 2 to 6 carbon atoms, alkoxycarbonyl with 2 to 6 carbon atoms, alkylsulfonyl with 1 to 6 carbon atom(s), alkylsulfinyl with 1 to 6 carbon atom(s), 3- to 8-membered cycloalkyl, 3- to 8-membered cycloalkylcarbonyl, and 3- to 8-membered cycloalkoxycarbonyl, 3- to 8-membered cycloalkylsulfonyl, and 3- to 8-membered cycloalkylsulfinyl, wherein each group may further be substituted with one or more group(s) selected independently from halogen, hydroxy, carboxy, alkyl with 1 to 6 carbon atom(s), alkoxy with 1 to 6 carbon atom(s) and alkoxycarbonyl with 2 to 6 carbon atoms;
(l) 6- to 10-membered aryl, 6- to 10-membered arylcarbonyl, 6- to 10-membered aryloxycarbonyl, 6- to 10-membered arylsulfonyl, 6- to 10-membered arylsulfinyl, 5- to 10-membered heteroaryl, 5- to 10-membered heteroarylcarbonyl, 5- to 10-membered heteroaryloxycarbonyl, 5- to 10-membered heteroarylsulfonyl, and 5- to 10-membered heteroarylsulfinyl, wherein each group may further be substituted with halogen, hydroxy, mercapto, carboxy, cyano, nitro, alkyl with 1 to 6 carbon atom(s), alkoxy with 1 to 6 carbon atom(s), alkoxycarbonyl with 2 to 6 carbon atoms or alkylthio with 1 to 6 carbon atom(s);
(m) two substituents are combined together with nitrogen atom to form 4- to 8-membered nitrogen-containing saturated heterocycle with 1 to 4 heteroatom(s) selected from 1 to 3 nitrogen(s), 0 to 1 oxygen and 0 to 1 sulfur, wherein the nitrogen-containing saturated heterocycle may be substituted on any carbon or nitrogen atom by halogen, hydroxy, carboxy, alkyl with 1 to 6 carbon atom(s), alkoxy with 1 to 6 carbon atom(s), alkoxycarbonyl with 2 to 6 carbon atoms or alkylcarbonyl with 2 to 6 carbon atoms, where the substituent may be kept in chemically stable state,
in case that alkyl, alkenyl and alkynyl in R² and R³ is substituted, each group may be substituted with one or more group(s) selected independently from the following (a') to (c'):
(a') halogen, hydroxy, mercapto, haloalkyl with 1 to 4 carbon atom(s) and haloalkoxy with 1 to 6 carbon atom(s), cyano;
(b') alkoxy with 1 to 6 carbon atom(s), alkylsulfonyl with 1 to 6 carbon atom(s), alkylsulfinyl with 1 to 6 carbon atom(s), alkylcarbonyloxy with 2 to 6 carbon atoms, alkylthio with 1 to 6 carbon atom(s), substituted or unsubstituted 3- to 8-membered cycloalkyl, and substituted or unsubstituted 3- to 8-membered cycloalkyloxy, wherein each group may further be substituted with the same or different one or more group(s) slected from halogen, hydroxy, alkyl with 1 to 6 carbon atom(s) and alkoxy with 1 to 6 carbon atom(s);
(c') substituted or unsubstituted 6- to 10-membered aryl, substituted or unsubstituted 6- to 10-membered aryloxy, substituted or unsubstituted 5- to 10-membered heteroaryl and substituted or unsubstituted 5- to 10-membered heteroaryloxy, wherein each group may further be substituted with the same or different one or more group(s) selected from the following (g') to (j'); substituted or unsubstituted amino, substituted or unsubstituted carbamoyl and substituted or unsubstituted sulfamoyl, wherein each group may further be substituted with the same or different one or more group(s) selected from the following (k') to (m');
in case that aryl, aryloxy, heteroaryl and heteroaryloxy in the above (c') is substituted, each group may be substituted with one or more group(s) selected from the following (g') to (j'):
(g') halogen, hydroxy, mercapto, cyano, nitro, haloalkyl with 1 to 6 carbon atom(s), and haloalkoxy with 1 to 6 carbon atom(s);
(h') alkyl with 1 to 6 carbon atom(s), alkoxy with 1 to 6 carbon atom(s), alkenyl with 2 to 6 carbon atoms, alkynyl with 2 to 6 carbon atoms and alkylthio with 1 to 6 carbon atom(s), wherein each group may further be substituted with one or more group(s) independently selected from halogen, hydroxy, alkoxy with 1 to 6 carbon atom(s), amino optionally substituted with the same or different one or two alkyl(s) with 1 to 6 carbon atom(s), carbamoyl optionally substituted with the same or different one or two alkyl(s) with 1 to 6 carbon atom(s), sulfamoyl optionally substituted with the same or different one or two alkyl(s) with 1 to 6 carbon atom(s), and alkylsulfonyl with 1 to 6 carbon atom(s);
(i') 3- to 8-membered cycloalkyl and 4- to 8-membered saturated heterocycle, wherein each group may further be substituted with one or more group(s) independently selected from halogen, hydroxy, oxo, alkyl with 1 to 6 carbon atom(s), alkoxy with 1 to 6 carbon atom(s), and alkylcarbonyl with 2 to 6 carbon atoms;
(j') amino, carbamoyl, sulfamoyl, wherein each group may further be substituted with one or two group(s) selected from the following (k') to (m');
in case that amino, carbamoyl and sulfamoyl in the above (c') and (j') is substituted, each group may be substituted with one or two group(s) selected from the following (k'), (l') and (m'):
(k') alkyl with 1 to 6 carbon atom(s), alkenyl with 2 to 6 carbon atoms, alkynyl with 2 to 6 carbon atoms, alkylcarbonyl with 2 to 6 carbon atoms, alkylsulfonyl with 1 to 6 carbon atom(s), alkylsulfinyl with 1 to 6 carbon atom(s), 3- to 8-membered cycloalkyl, 3- to 8-membered cycloalkylcarbonyl, 3- to 8-membered cycloalkoxycarbonyl, 3- to 8-membered cycloalkylsulfonyl, 3- to 8-membered cycloalkylsulfinyl, wherein each group may further be substituted with one or more group(s) selected independently from halogen, hydroxy, alkyl with 1 to 6 carbon atom(s), and alkoxy with 1 to 6 carbon atom(s);
(l') 6- to 10-membered aryl, 6- to 10-membered arylalkyl, 6- to 10-membered aryloxyalkyl, 6- to 10-membered arylcarbonyl, 6- to 10-membered arylsulfonyl, 6- to 10-membered arylsulfinyl, 5- to 10-membered heteroaryl, 5- to 10-membered heteroarylalkyl, 5- to 10-membered heteroaryloxyalkyl, 5- to 10-membered heteroarylcarbonyl, 5- to 10-membered heteroarylsulfonyl, and 5- to 10-membered heteroarylsulfinyl, wherein each group may further be substituted with one or more group(s) selected from halogen, hydroxy, mercapto, cyano, nitro, alkyl with 1 to 6 carbon atom(s), alkoxy with 1 to 6 carbon atom(s) and alkylthio with 1 to 6 carbon atom(s);
(m') two substituents are combined together with nitrogen atom to form 4- to 8-membered nitrogen-containing saturated heterocycle with 1 to 4 heteroatom(s) selected from 1 to 3 nitrogen(s), 0 to 1 oxygen and 0 to 1 sulfur, wherein the nitrogen-containing saturated heterocycle may be substituted on any carbon or nitrogen atom by halogen, hydroxy, alkyl with 1 to 6 carbon atom(s), alkoxy with 1 to 6 carbon atom(s) and alkylcarbonyl with 2 to 6 carbon atoms, where the substituent may be kept in chemically stable state,
in case that cycloalkyl, saturated heterocycle in R², the nitrogen-containing saturated heterocycle formed by combining R² with R³, and the nitrogen-containing saturated heterocycle formed by combining R² with L² is substituted, each group may be substituted with one or more group(s) selected independently from the group consisting of: halogen; hydroxy; oxo; substituted or unsubstituted alkyl with 1 to 6 carbon atom(s), substituted or unsubstituted alkoxy with 1 to 6 carbon atom(s) and substituted or unsubstituted alkylcarbonyl with 2 to 6 carbon atoms, wherein the alkyl, alkoxy or alkylcarbonyl may be substituted with one or more group(s) selected independently from the above (a') to (c'); substituted or unsubstituted aryl, substituted or unsubstituted aryloxy, substituted or unsubstituted a heteroaryl and substituted or unsubstituted heteroaryloxy, wherein the aryl, aryloxy, heteroaryl or heteroaryloxy may be substituted with one or more group(s) selected independently from the above (g') to (j'); substituted or unsubstituted amino, substituted or unsubstituted carbamoyl and substituted or unsubstituted sulfamoyl, wherein the amino, carbamoyl or sulfamoyl may be substituted with one or two group(s) selected independently from the above (k') to (m'); and
in case that aryl and heteroaryl in R² is substituted, each group may be substituted with one or more group(s) selected independently from the above (g') to (j').

6. The adenine compound any one of claim 5 or a pharmaceutically acceptable salt thereof,
wherein R² and R³ are independently hydrogen, substituted or unsubstituted alkyl with 1 to 6 carbon atom(s), substituted or unsubstituted 4- to 8-membered saturated heterocycle with 1 to 4 heteroatom(s) selected from 0 to 3 nitrogen(s), 0 to 1 oxygen and 0 to 1 sulfur, substituted or unsubstituted 3- to 8-membered cycloalkyl, substituted or unsubstituted 6- to 10-membered aryl, or substituted or unsubstituted 5- to 10-membered heteroaryl; or R² and R³ are combined together to form 4- to 8-membered nitrogen-containing saturated heterocycle with 1 to 4 heteroatom(s) selected from 1 to 3 nitrogen(s), 0 to 1 oxygen and 0 to 1 sulfur;
said alkyl is optionally substituted with one or more group(s) selected from halogen, hydroxy, alkoxy with 1 to 6 carbon atom(s), substituted or unsubstituted 6- to 10-membered aryl, substituted or unsubstituted 6- to 10-membered aryloxy, substituted or unsubstituted amino, and carbamoyl optionally substituted with the same or different one or two alkyl(s) with 1 to 6 carbon atom(s);
said saturated heterocycle, cycloalkyl and nitrogen-containing saturated heterocycle formed by combining R² with R³ are optionally substituted with one or more group(s) selected from halogen, hydroxy, oxo, alkyl with 1 to 6 carbon atom(s), alkoxy with 1 to 6 carbon atom(s), substituted or unsubstituted 6- to 10-membered aryl, substituted or unsubstituted 6- to 10-membered aryloxy, substituted or unsubstituted 6- to 10-membered arylalkyl, substituted or unsubstituted 6- to 10-membered aryloxyalkyl, substituted or unsubstituted 5- to 10-membered heteroaryl, substituted or unsubstituted 5- to 10-membered heteroaryloxy, substituted or unsubstituted 5- to 10-membered heteroarylalkyl, substituted or unsubstituted 5- to 10-membered heteroaryloxyalkyl, substituted or unsubstituted amino, and carbamoyl optionally substituted with the same or different one or two alkyl(s) with 1 to 6 carbon atom(s);
in case that said aryl, aryloxy, arylalkyl, aryloxyalkyl, heteroaryl, heteroaryloxy, heteroarylalkyl and heteroaryloxyalkyl are substituted, each group may be substituted with one or more group(s) selected from halogen, hydroxy, alkyl with 1 to 6 carbon atom(s), alkoxy with 1 to 6 carbon atom(s), substituted or unsubstituted amino; and in case that said amino is substituted, it may be substituted with the same or different one or two group(s) selected from alkyl with 1 to 6 carbon atom(s), alkylcarbonyl with 2 to 6 carbon atoms and alkylsulfonyl with 1 to 6 carbon atom(s), or two substituents on said substituted amino are combined together to form 4- to 8-membered saturated heterocycle with 1 to 4 heteroatom(s) selected from 1 to 3 nitrogen(s), 0 to 1 oxygen and 0 to 1 sulfur wherein said saturated heterocycle is optionally substituted with one or more group(s) selected from halogen, hydroxy, alkyl with 1 to 6 carbon atom(s), alkoxy with 1 to 6 carbon atom(s), alkylcarbonyl with 2 to 6 carbon atoms, and amino optionally substituted with the same or different one or two alkyl(s) with 1 to 6 carbon atom(s).

7. The adenine compound according to any one of claims 1 to 5 or a pharmaceutically acceptable salt thereof,
wherein R² and R³ are independently hydrogen; alkyl with 1 to 6 carbon atom(s); or alkyl with 1 to 6 carbon atom(s) substituted with 1 to 3 substituent(s) selected from halogen, cyano, hydroxy, alkoxy with 1 to 6 carbon atom(s), substituted or unsubstituted aryl, substituted or unsubstituted aryloxy and substituted or unsubstituted amino;
in case that aryl and aryloxy are substituted, each group may be substituted with one or more group(s) selected from halogen, hydroxy, alkyl with 1 to 6 carbon atom(s), alkoxy with 1 to 6 carbon atom(s) and substituted or unsubstituted amino;
in case that amino is substituted, it may be substituted with the same or different one or two group(s) selected from alkyl with 1 to 6 carbon atom(s), alkylcarbonyl with 2 to 6 carbon atoms and alkylsulfonyl with 1 to 6 carbon atom(s), or two substituents on said substituted amino are combined together to form 4- to 8-membered saturated heterocycle with 1 to 4 heteroatom(s) selected from 1 to 3 nitrogen(s), 0 to 1 oxygen and 0 to 1 sulfur wherein said saturated heterocycle is optionally substituted with one or more group(s) selected from halogen, hydroxy, alkyl with 1 to 6 carbon atom(s), alkoxy with 1 to 6 carbon atom(s), alkylcarbonyl with 2 to 6 carbon atoms, and amino optionally substituted with the same or different one or two alkyl(s) with 1 to 6 carbon atom(s).

8. The adenine compound any one of claim 7 or a pharmaceutically acceptable salt thereof wherein R² and R³ are independently hydrogen or alkyl with 1 to 6 carbon atom(s) optionally substituted with hydroxy, C₁₋₆ alkoxy, or an amino group optionally substituted with the same or different one or two C₁₋₆ alkyl(s).

9. The adenine compound according to any one of claims 1 to 5 or a pharmaceutically acceptable salt thereof,
wherein R² is substituted or unsubstituted 4- to 8-membered saturated heterocycle with 1 to 4 heteroatom(s) selected from 0 to 3 nitrogen(s), 0 to 1 oxygen and 0 to 1 sulfur, substituted or unsubstituted 3- to 8-membered cycloalkyl, substituted or unsubstituted 6- to 10-membered aryl, or substituted or unsubstituted 5- to 10-membered heteroaryl;
R³ is hydrogen or alkyl with 1 to 6 carbon atom(s);
in case that saturated heterocycle, cycloalkyl, aryl and heteroaryl are substituted, each group may be substituted with one or more group(s) selected from halogen, hydroxy, alkyl with 1 to 6 carbon atom(s), alkoxy with 1 to 6 carbon atom(s) and substituted or unsubstituted amino
in case that amino is substituted, it may be substituted with the same or different one or two group(s) selected from alkyl with 1 to 6 carbon atom(s), alkylcarbonyl with 2 to 6 carbon atoms and alkylsulfonyl with 1 to 6 carbon atom(s), or two substituents on said amino are combined together to form 4- to 8-membered saturated heterocycle with 1 to 4 heteroatom(s) selected from 1 to 3 nitrogen(s), 0 to 1 oxygen and 0 to 1 sulfur wherein said saturated heterocycle is optionally substituted with one or more group(s) selected from halogen, hydroxy, alkyl with 1 to 6 carbon atom(s), alkoxy with 1 to 6 carbon atom(s), alkylcarbonyl with 2 to 6 carbon atoms, and amino optionally substituted with the same or different one or two alkyl(s) with 1 to 6 carbon atom(s).

10. The adenine compound according to any one of claims 1 to 5 or a pharmaceutically acceptable salt thereof, wherein R² and R³ are combined together to form 4- to 8-membered nitrogen-containing saturated heterocycle with 1 to 4 heteroatom(s) selected from 1 to 3 nitrogen(s), 0 to 1 oxygen and 0 to 1 sulfur;
wherein said nitrogen-containing saturated heterocycle is optionally substituted with one or more group(s) selected from halogen; hydroxy; oxo; alkyl with 1 to 6 carbon atom(s), alkoxy with 1 to 6 carbon atom(s) and alkylcarbonyl with 2 to 6 carbon atoms wherein said alkyl, alkoxy and alkylcarbonyl is optionally substituted with 1 to 3 substituent(s) selected from halogen, cyano, hydroxy, alkoxy with 1 to 6 carbon atom(s), substituted or unsubstituted 6- to 10-membered aryl, substituted or unsubstituted 6- to 10-membered aryloxy, substituted or unsubstituted amino, and carbamoyl optionally substituted with the same or different one or two alkyl(s) with 1 to 6 carbon atom(s); substituted or unsubstituted 3- to 8-membered cycloalkyl; substituted or unsubstituted 6- to 10-membered aryl; substituted or unsubstituted 6- to 10-membered aryloxy; substituted or unsubstituted 5- to 10-membered heteroaryl; substituted or unsubstituted 5- to 10-membered heteroaryloxy; substituted or unsubstituted amino, and carbamoyl optionally substituted with the same or different one or two alkyl(s) with 1 to 6 carbon atom(s);
in case that aryl, aryloxy, heteroaryl and heteroaryloxy are substituted, each group may be substituted with one or more group(s) selected from halogen, hydroxy, alkyl with 1 to 6 carbon atom(s), alkoxy with 1 to 6 carbon atom(s) and substituted or unsubstituted amino;
in case that amino is substituted, it may be substituted with one or more group(s) selected from the same or different one or two group(s) selected from alkyl with 1 to 6 carbon atom(s), alkylcarbonyl with 2 to 6 carbon atoms and alkylsulfonyl with 1 to 6 carbon atom(s), or two substituents on said substituted amino are combined together to form 4- to 8-membered saturated heterocycle with 1 to 4 heteroatom(s) selected from 1 to 3 nitrogen(s), 0 to 1 oxygen and 0 to 1 sulfur wherein said saturated heterocycle is optionally substituted with one or more group(s) selected from halogen, hydroxy, alkyl with 1 to 6 carbon atom(s), alkoxy with 1 to 6 carbon atom(s), alkylcarbonyl with 2 to 6 carbon atoms, and amino optionally substituted with the same or different one or two alkyl(s) with 1 to 6 carbon atom(s).

11. The adenine compound according to claim 5 or 10, or a pharmaceutically acceptable salt thereof, wherein the nitrogen-containing saturated heterocycle formed by combining R² with R³ is substituted or unsubstituted azetidine, substituted or unsubstituted morpholine, substituted or unsubstituted piperidine, substituted or unsubstituted piperazine; substituted or unsubstituted pyrrolidine or substituted or unsubstituted 1,4-perhydrodiazepine.

12. The adenine compound according to any one of claims 1 to 5, or a pharmaceutically acceptable salt thereof, wherein R³ is hydrogen or alkyl with 1 to 6 carbon atom(s); any carbon atom on R² and L² are combined together to form optionally substituted 4- to 8-membered nitrogen-containing saturated heterocycle containing 1 to 4 heteroatom(s) selected from 1 to 3 nitrogen(s), 0 to 1 oxygen and 0 to 1 sulfur.

13. The adenine compound according to claim 12, or a pharmaceutically acceptable salt thereof, wherein -L²-NR²R³ in the formula (1) is represented by the formula: 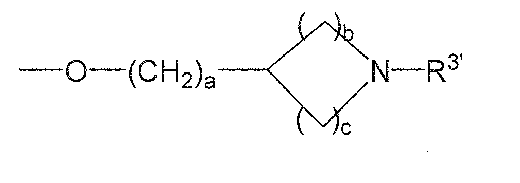 in which a is an integer of 0 to 2, b is an integer of 0 to 2, c is an integer of 1 to 4, with the proviso that the sum of b and c is 2 to 4, and R^{3'} is hydrogen or alkyl with 1 to 6 carbon atom(s).

14. The adenine compound according to any one of claims 1 to 11, or a pharmaceutically acceptable salt thereof, wherein -L²- in the formula (1) is a single bond or divalent group of the formula: -(O)ₚ-(CH₂)ₙ-wherein p is 0 or 1, n is an integer of 0 to 6 when p is 0 or an integer of 2 to 6 when p is 1.

15. The adenine compound according to any one of claims 1 to 14, or a pharmaceutically acceptable salt thereof, wherein L¹ in the formula (1) is alkylene with 1 to 6 carbon atom(s) or divalent group of the formula: - (CH₂)_{n'}-(O)_{p'}- wherein p' is 0 or 1; n' is an integer of 1 to 6 when p' is 0 or an integer of 2 to 6 when p' is 1.

16. The adenine compound according to claim 15, or a pharmaceutically acceptable salt thereof, wherein L¹ is alkylene with 1 to 3 carbons;
L² is methylene or divalent group of the formula: -O-(CH₂)ₙ- wherein n is an integer of 2 to 4.

17. The adenine compound according to any one of claims 1 to 16, or a pharmaceutically acceptable salt thereof, wherein X in the formula (1) is a single bond, NH, oxygen or sulphur; R¹ is alkyl with 1 to 6 carbon atom(s), or alkyl with 1 to 6 carbon atom(s) substituted with a substituent selected from haloalkyl with 1 to 4 carbon atom(s), alkoxy with 1 to 4 carbons, 3- to 6-membered cycloalkyl, 6- to 10-membered aryl and 5- to 10-membered heteroaryl wherein said cycloalkyl, aryl and heteroaryl is optionally substitued with one to four group(s) selected from halogen, hydroxy, alkyl with 1 to 6 carbon atom(s), and alkoxy with 1 to 6 carbon atom(s).

18. The adenine compound according to claim 17 or a pharmaceutically acceptable salt thereof, wherein X in the formula (1) is NH or oxygen.

19. The adenine compound according to any one of claims 1 to 5, 17 to 18, or a pharmaceutically acceptable salt thereof, wherein A is pyridine ring; L¹ is alkylene with 1 to 3 carbons; L² is single bond; R² is hydrogen, alkyl with 1 to 6 carbon atom(s), or alkyl with 1 to 6 carbon atom(s) substituted with amino, alkylamino or dialkylamino; R³ is alkyl with 1 to 6 carbon atom(s) substituted with amino, alkylamino or dialkylamino; or R² and R³ are combined together to form piperazine ring optionally substituted with alkyl with 1 to 6 carbon atom(s), 1,4-perhydrodiazepine ring optionally substituted with alkyl with 1 to 6 carbon atom(s), or saturated nitrogen-containing heterocycle selected from pyrrolidine ring, piperidine ring, morpholine ring, thiomorpholine ring and azetidine ring, wherein said saturated nitrogen-containing heterocycle is substituted with amino, alkylamino, dialkylamino, or alkyl with 1 to 6 carbon atom(s) substituted with amino, alkylamino or dialkylamino.

20. The adenine compound according to claim 1, which is selected from the following compounds:
6-amino-2-butoxy-9-(4-morpholin-4-ylmethylbenzyl)-7,9-dihydropurin-8-one;
6-amino-2-butoxy-9-(4-piperidin-1-ylmethylbenzyl)-7,9-dihydropurin-8-one;
6-amino-2-butoxy-9-[4-(4-methylpiperazin-1-ylmethyl)benzyl]-7,9-dihydropurin-8-one;
6-amino-2-butoxy-9-[4-(4-dimethylamminiopiperidin-1-ylmethyl)benzyl]-7,9-dihydropurin-8-one;
6-amino-2-butoxy-9-[4-(3-dimethylamminiopyrrolidin-1-ylmethyl)benzyl]-7,9-dihydropurin-8-one;
6-amino-2-butoxy-9-(4-{[methyl(1-methylpyrrolidin-3-yl)amino]methyl}benzyl)-7,9-dihydropurin-8-one;
N-{1-[4-(6-amino-2-butoxy-8-oxo-7,8-dihydropurin-9-ylmethyl)benzyl]piperidin-4-yl}acetamide;
1-[4-(6-amino-2-butoxy-8-oxo-7,8-dihydropurin-9-ylmethyl)benzyl]piperidin-4-carboxylic acid amide;
6-amino-2-butoxy-9-[3-(4-methylpiperazin-1-ylmethyl)benzyl]-7,9-dihydropurin-8-one;
6-amino-2-(2-methoxyethoxy)-9-(4-piperidin-1-ylmethylbenzyl)-7,9-dihydropurin-8-one;
6-amino-2-(2-methoxyethoxy)-9-[4-(4-methylpiperazin-1-ylmethyl)benzyl]-7,9-dihydropurin-8-one;
6-amino-2-(2-methoxyethoxy)-9-[4-(4-phenylpiperazin-1-ylmethyl)benzyl]-7,9-dihydropurin-8-one;
6-amino-2-(2-methoxyethoxy)-9-[4-(4-phenoxypiperidin-1-ylmethyl)benzyl]-7,9-dihydropurin-8-one;
6-amino-9-(4-dimethylamminiomethylbenzyl)-2-(2-methoxyethoxy)-7,9-dihydropurin-8-one;
6-amino-9-{4-[(diisopropylamino)methyl]benzyl}-2-(2-methoxyethoxy)-7,9-dihydropurin-8-one;
6-amino-2-(2-methoxyethoxy)-9-(4-{[(2-methoxyethyl)methylamino]methyl}benzyl)-7,9-dihydropurin-8-one;
6-amino-9-{4-[(cyclohexylmethylamino)methyl]benzyl}-2-(2-methoxyethoxy)-7,9-dihydropurin-8-one;
6-amino-9-(4-cyclohexylaminomethylbenzyl)-2-(2-methoxyethoxy)-7,9-dihydropurin-8-one;
6-amino-2-(2-methoxyethoxy)-9-{4-[(methylphenylamino)methyl]benzyl}-7,9-dihydropurin-8-one;
6-amino-9-{4-[(benzylmethylamino)methyl]benzyl}-2-(2-methoxyethoxy)-7,9-dihydropurin-8-one;
6-amino-9-(4-morpholin-4-ylmethylbenzyl)-2-propoxy-7,9-dihydropurin-8-one;
6-amino-2-cyclopropylmethoxy-9-(4-morpholin-4-ylmethylbenzyl)-7,9-dihydropurin-8-one;
6-amino-9-(4-morpholin-4-ylmethylbenzyl)-2-(4,4,4-trifluorobutoxy)-7,9-dihydropurin-8-one;
6-amino-9-[4-(4-methylpiperazin-1-ylmethyl)benzyl]-2-(4,4,4-trifluorobutoxy)-7,9-dihydropurin-8-one;
6-amino-9-(4-{[(2-methoxyethyl)methylamino]methyl}benzyl)-2-(4,4,4-trifluorobutoxy)-7,9-dihydropurin-8-one;
6-amino-9-[4-(4-methoxypiperidin-1-ylmethyl)benzyl]-2-(2,2,2-trifluoroethoxy)-7,9-dihydropurin-8-one;
6-amino-9-[4-(4-oxopiperidin-1-ylmethyl)benzyl]-2-(2,2,2-trifluoroethoxy)-7,9-dihydropurin-8-one;
6-amino-2-butylamino-9-(4-dimethylamminiomethylbenzyl)-7,9-dihydropurin-8-one;
6-amino-2-butylamino-9-(4-piperidin-1-ylmethylbenzyl)-7,9-dihydropurin-8-one;
6-amino-2-butylamino-9-(4-morpholin-4-ylmethylbenzyl)-7,9-dihydropurin-8-one;
6-amino-2-butylamino-9-[4-(4-dimethylamminiopiperidin-1-ylmethyl)benzyl]-7,9-dihydropurin-8-one;
6-amino-2-butylamino-9-[4-(4-methylpiperazin-1-ylmethyl)benzyl]-7,9-dihydropurin-8-one;
6-amino-2-butylamino-9-(3-piperidin-1-ylmethylbenzyl)-7,9-dihydropurin-8-one;
6-amino-2-butoxy-9-(3-morpholin-4-ylmethylbenzyl)-7,9-dihydropurin-8-one;
6-amino-9-[4-(4-aminopiperidin-1-ylmethyl)benzyl]-2-butoxy-7,9-dihydropurin-8-one;
6-amino-2-butoxy-9-[4-(2-dimethylaminoethoxy)benzyl]-7,9-dihydropurin-8-one;
6-amino-2-butoxy-9-[4-(3-dimethylamminiopropoxy)benzyl]-7,9-dihydropurin-8-one;
6-amino-2-(2-methoxyethoxy)-9-[4-(3-piperidin-1-ylpropoxy)benzyl]-7,9-dihydropurin-8-one;
6-amino-2-butylamino-9-[4-(3-morpholin-4-ylpropoxy)benzyl]-7,9-dihydropurin-8-one;
6-amino-2-butoxy-9-[6-(4-methylpiperazin-1-yl)pyridin-3-ylmethyl]-7,9-dihydropurin-8-one;
6-amino-2-butoxy-9-[6-(4-methyl-[1,4]diazepan-1-yl)pyridin-3-ylmethyl]-7,9-dihydropurin-8-one;
6-amino-2-(2-methoxyethoxy)-9-[6-(4-methylpiperazin-1-yl)pyridin-3-ylmethyl]-7,9-dihydropurin-8-one;
6-amino-9-[6-(4-methylpiperazin-1-yl)pyridin-3-ylmethyl]-2-(4,4,4-trifluorobutoxy)-7,9-dihydropurin-8-one;
6-amino-2-ethoxy-9-[6-(4-methylpiperazin-1-yl)pyridin-3-ylmethyl]-7,9-dihydropurin-8-one;
6-amino-9-[6-(4-methylpiperazin-1-yl)pyridin-3-ylmethyl]-2-(2,2,2-trifluoroethoxy)-7,9-dihydropurin-8-one;
6-amino-2-butylamino-9-[6-(4-methylpiperazin-1-yl)pyridin-3-ylmethyl]-7,9-dihydropurin-8-one;
6-amino-2-butylamino-9-[6-(4-methyl-[1,4]diazepan-1-yl)pyridin-3-ylmethyl]-7,9-dihydropurin-8-one;
6-amino-2-butylamino-9-(6-piperazin-1-ylpyridin-3-ylmethyl)-7,9-dihydropurin-8-one;
6-amino-2-butylamino-9-[6-(4-dimethylamminiopiperidin-1-yl)pyridin-3-ylmethyl]-7,9-dihydropurin-8-one;
6-amino-2-butylamino-9-{6-[(3-dimethylaminopropyl)methylamino]pyridin-3-ylmethyl}-7,9-dihydropurin-8-one;
6-amino-2-butylamino-9-[6-(3-dimethylamminiopyrrolidin-1-yl)pyridin-3-ylmethyl}-7,9-dihydropurin-8-one;
6-amino-2-butoxy-9-[6-(2-morpholin-4-ylethoxy)pyridin-3-ylmethyl}-7,9-dihydropurin-8-one;
6-amino-2-butylamino-9-[6-(2-morpholin-4-ylethoxy)pyridin-3-ylmethyl}-7,9-dihydropurin-8-one;
6-amino-2-butylamino-9-[6-(2-dimethylaminoethoxy)pyridin-3-ylmethyl}-7,9-dihydropurin-8-one;
6-amino-2-butylamino-9-[6-(4-dimethylamminiobutoxy)pyridin-3-ylmethyl}-7,9-dihydropurin-8-one;
6-amino-2-butylamino-9-[5-chloro-6-(4-methylpiperazin-1-yl)pyridin-3-ylmethyl]-7,9-dihydropurin-8-one;
6-amino-9-[5-chloro-6-(4-methylpiperazin-1-yl)pyridin-3-ylmethyl]-2-ethoxy-7,9-dihydropurin-8-one;
6-amino-2-butylamino-9-[5-chloro-6-(2-dimethylaminoethoxy)pyridin-3-ylmethyl]-7,9-dihydropurin-8-one;
6-amino-2-butylamino-9-[5-chloro-6-(2-morpholin-4-ylethoxy)pyridin-3-ylmethyl]-7,9-dihydropurin-8-one;
6-amino-2-butylamino-9-[4-(4-methylpiperazin-1-yl)-3-nitrobenzyl]-7,9-dihydropurin-8-one;
6-amino-9-[3-amino-4-(4-methylpiperazin-1-yl)benzyl]-2-butylamino-7,9-dihydropurin-8-one;
6-amino-2-ethoxy-9-(3-methoxy-4-morpholin-4-ylmethylbenzyl)-7,9-dihydropurin-8-one;
6-amino-9-(4-dimethylamminiomethylbenzyl)-2-ethoxy-7,9-dihydropurin-8-one;
6-amino-9-(4-diethylaminomethylbenzyl)-2-ethoxy-7,9-dihydropurin-8-one;
6-amino-9-(4-diisopropylaminomethylbenzyl)-2-ethoxy-7,9-dihydropurin-8-one;
6-amino-2-ethoxy-9-(4-piperidin-1-ylmethylbenzyl)-7,9-dihydropurin-8-one;
6-amino-2-ethoxy-9-[4-(4-methoxypiperidin-1-ylmethyl)benzyl]-7,9-dihydropurin-8-one;
6-amino-2-ethoxy-9-(4-morpholin-4-ylmethylbenzyl)-7,9-dihydropurin-8-one;
6-amino-2-ethoxy-9-(4-thiomorpholine-4-ylmethylbenzyl)-7,9-dihydropurin-8-one;
6-amino-2-ethoxy-9-[4-(4-methylpiperazin-1-ylmethylbenzyl)]-7,9-dihydropurin-8-one;
6-amino-2-butyl-9-(4-dimethylamminiomethylbenzyl)-7,9-dihydropurin-8-one;
6-amino-2-butyl-9-(4-morpholin-4-ylmethylbenzyl)-7,9-dihydropurin-8-one;
6-amino-2-butyl-9-[4-(4-methoxypiperidin-1-ylmethyl)benzyl]-7,9-dihydropurin-8-one;
6-amino-2-butoxy-9-[3-(4-dimethylamminiomethylphenoxy)propyl]-7,9-dihydropurin-8-one;
6-amino-2-butoxy-9-(5-dimethylamminiomethylfuran-2-ylmethyl)-7,9-dihydropurin-8-one;
6-amino-9-(4-dimethylamminiomethylbenzyl)-2-[(pyridin-4-ylmethyl)amino]-7,9-dihydropurin-8-one;
6-amino-2-(2-methoxyethoxy)-9-[4-(4-pyridin-4-ylpiperazin-1-ylmethyl)benzyl]-7,9-dihydropurin-8-one;
6-amino-9-(4-{[bis(2-methoxyethyl)amino]methyl}benzyl)-2-(2-methoxyethoxy)-7,9-dihydropurin-8-one;
6-amino-9-(4-{[bis(2-hydroxyethyl)amino]methyl}benzyl)-2-butoxy-7,9-dihydropurin-8-one;
6-amino-2-butoxy-9-(4-{[(2,3-dihydroxypropyl)methylamino]methyl}benzyl)-7,9-dihydropurin-8-one;
6-amino-2-butoxy-9-(4-{[(2-dimethylamminioethyl)methylamino]methyl}benzyl)-7,9-dihydropurin-8-one;
6-amino-9-[6-(2-dimethylaminoethoxy)pyridin-3-ylmethyl]-2-(2-methoxyethoxy)-7,9-dihydropurin-8-one;
6-amino-2-butoxy-9-(4-dimethylamminiomethylbenzyl)-7,9-dihydropurin-8-one;
6-amino-2-butoxy-9-[4-(3-hydroxyazetidine-1-ylmethyl)benzyl]-7,9-dihydropurin-8-one;
6-amino-9-(4-{[bis(2-diethylamminioethyl)amino]methyl}benzyl)-2-butoxy-7,9-dihydropurin-8-one;
6-amino-2-butoxy-9-{4-[4-(2-dimethylaminoacetyl)piperazin-1-ylmethyl]benzyl}-7,9-dihydropurin-8-one;
2-{4-[4-(6-amino-2-butoxy-8-oxo-7,8-dihydropurin-9-ylmethyl)benzyl]piperazin-1-yl}-N,N-dimethylacetamide; 6-amino-2-(2-methoxyethoxy)-9-[4-(4-methoxypiperidin-1-ylmethyl)benzyl]-7,9-dihydropurin-8-one;
6-amino-9-{4-[(butylmethylamino)methyl]benzyl}-2-(2-methoxyethoxy)-7,9-dihydropurin-8-one;
4-({4-[6-amino-2-(2-methoxyethoxy)-8-oxo-7,8-dihydropurin-9-ylmethyl]benzyl}methylamino)butyronitrile;
N-(1-{4-[6-amino-2-(2-methoxyethoxy)-8-oxo-7,8-dihydropurin-9-ylmethyl]benzyl}pyrrolidin-3-yl)-N-methylacetamide;
6-amino-9-(4-{[ethyl(tetrahydropyran-4-yl)amino]methyl}benzyl)-2-(2-methoxyethoxy)-7,9-dihydropurin-8-one;
6-amino-9-[4-(4,4-difluoropiperidin-1-ylmethyl)benzyl]-2-(2-methoxyethoxy)-7,9-dihydropurin-8-one;
6-amino-9-[4-(4-cyclopentylpiperazin-1-ylmethyl)benzyl]-2-(2-methoxyethoxy)-7,9-dihydropurin-8-one;
6-amino-9-(4-{[isopropyl(2-methoxyethyl)amino]methyl}benzyl)-2-(2-methoxyethoxy)-7,9-dihydropurin-8-one;
6-amino-2-butoxy-9-{6-[(2-dimethylamminioethyl)methylamino]pyridin-3-ylmethyl}-7,9-dihydropurin-8-one;
6-amino-9-[5-chloro-6-(4-methylpiperazin-1-yl)pyridin-3-ylmethyl]-2-(2-methoxyethoxy)-7,9-dihydropurin-8-one;
6-amino-9-[5-chloro-6-(4-methyl-[1,4]diazepan-1-yl)pyridin-3-ylmethyl]-2-(2-methoxyethoxy)-7,9-dihydropurin-8-one;
6-amino-2-butoxy-9-(6-{2-[(2-hydroxyethyl)methylamino]ethoxy}pyridin-3-ylmethyl)-7,9-dihydropurin-8-one;
6-amino-2-butoxy-9-[6-(2-dimethylamminio-1-dimethylamminiomethylethoxy)pyridin-3-ylmethyl]-7,9-dihydropurin-8-one;
6-amino-2-(2-methoxyethoxy)-9-[6-(2-piperidin-1-ylethoxy)pyridin-3-ylmethyl]-7,9-dihydropurin-8-one;
6-amino-9-[6-(3-dimethylamminio-2,2-dimethylpropoxy)pyridin-3-ylmethyl]-2-(2-methoxyethoxy)-7,9-dihydropurin-8-one;
6-amino-2-(2-methoxyethoxy)-9-[6-(1-methylpiperidine-3-ylmethoxy)pyridin-3-ylmethyl]-7,9-dihydropurin-8-one;
6-amino-2-(2-methoxyethoxy)-9-[6-(1-methylpiperidine-4-yloxy)pyridin-3-ylmethyl]-7,9-dihydropurin-8-one;
6-amino-9-[6-(2-dimethylaminoethoxy)pyridin-3-ylmethyl}-2-ethoxy-7,9-dihydropurin-8-one;
6-amino-2-ethoxy-9-{6-[2-(4-methylpiperazin-1-yl)ethoxy]pyridin-3-ylmethyl}-7,9-dihydropurin-8-one;
6-amino-2-ethoxy-9-{6-[3-(4-methylpiperazin-1-yl)propoxy]pyridin-3-ylmethyl}-7,9-dihydropurin-8-one;
6-amino-2-butylamino-9-[6-(3-dimethylamminiopropoxy)pyridin-3-ylmethyl}-7,9-dihydropurin-8-one;
6-amino-2-(2-methoxyethoxy)-9-[6-(1-methylpiperidine-4-ylmethoxy)pyridin-3-ylmethyl]-7,9-dihydropurin-8-one;
6-amino-9-[5-chloro-6-(2-dimethylaminoethoxy)pyridin-3-ylmethyl]-2-(2-methoxyethoxy)-7,9-dihydropurin-8-one;
6-amino-9-[5-chloro-6-(3-dimethylamminiopropoxy)pyridin-3-ylmethyl]-2-(2-methoxyethoxy)-7,9-dihydropurin-8-one;
6-amino-9-[5-chloro-6-(3-dimethylamminio-2,2-dimethylpropoxy)pyridin-3-ylmethyl]-2-(2-methoxyethoxy)-7,9-dihydropurin-8-one;
6-amino-9-[5-chloro-6-(2-pyrrolidin-1-ylethoxy)pyridin-3-ylmethyl]-2-(2-methoxyethoxy)-7,9-dihydropurin-8-one;
6-amino-9-{5-chloro-6-[3-(4-methylpiperazin-1-yl)propoxy]pyridin-3-ylmethyl}-2-(2-methoxyethoxy)-7,9-dihydropurin-8-one;
6-amino-9-[5-chloro-6-(1-methylpiperidine-4-yloxy)pyridin-3-ylmethyl]-2-(2-methoxyethoxy)-7,9-dihydropurin-8-one;
6-amino-2-(2-methoxyethoxy)-9-[6-(3-morpholin-4-yl-propyl)pyridin-3-ylmethyl]-7,9-dihydropurin-8-one;
6-amino-2-(2-methoxyethoxy)-9-[6-(3-dimethylaminopropyl)pyridin-3-ylmethyl]-7,9-dihydropurin-8-one;
6-amino-2-(2-methoxyethoxy)-9-[6-(1-methylpiperidine-2-ylmethoxy)pyridin-3-ylmethyl]-7,9-dihydropurin-8-one;
6-amino-2-(2-methoxyethoxy)-9-[6-(1-methylpyrrolidin-2-ylmethoxy)pyridin-3-ylmethyl]-7,9-dihydropurin-8-one;
6-amino-9-[6-(1-ethylpiperidine-3-yloxy)pyridin-3-ylmethyl]-2-(2-methoxyethoxy)-7,9-dihydropurin-8-one;
6-amino-9-[6-(1-isopropylpyrrolidin-3-yloxy)pyridin-3-ylmethyl]-2-(2-methoxyethoxy)-7,9-dihydropurin-8-one;
6-amino-2-butoxy-9-{6-[2-(4-methylpiperazin-1-yl)ethoxy]pyridin-3-ylmethyl}-7,9-dihydropurin-8-one;
6-amino-2-butoxy-9-{6-[3-(4-methylpiperazin-1-yl)propoxy]pyridin-3-ylmethyl}-7,9-dihydropurin-8-one;
6-amino-2-(2-methoxyethoxy)-9-[6-(1-propylpiperidin-4-yloxy)pyridin-3-ylmethyl]-7,9-dihydropurin-8-one;
6-amino-9-[6-(1-isopropylpiperidin-4-yloxy)pyridin-3-ylmethyl]-2-(2-methoxyethoxy)-7,9-dihydropurin-8-one,
or a pharmaceutically acceptable salt thereof.

21. A pharmaceutical composition comprising as an active ingredient the adenine compound according to any one of claims 1 to 20, or a pharmaceutically acceptable salt thereof.

22. TLR7 activating agent comprising as an active ingredient the adenine compound according to any one of claims 1 to 20, or a pharmaceutically acceptable salt thereof.

23. An immune-response modifier comprising as an active ingredient the adenine compound according to any one of claims 1 to 20, or a pharmaceutically acceptable salt thereof.

24. A therapeutic or prophylactic agent for allergic diseases, viral diseases or cancer, which comprises as an active ingredient the adenine compound according to any one of claims 1 to 20, or a pharmaceutically acceptable salt thereof.

25. A therapeutic or prophylactic agent for asthma, COPD, allergic rhinitis, allergic conjunctivitis, atopic dermatitis, cancer, hepatitis B, hepatitis C, HIV, HPV, bacterial infectious disease or dermatitis, which comprises as an active ingredient the adenine compound according to any one of claims 1 to 20, or a pharmaceutically acceptable salt thereof.
